# FASCICULE DE BREVET EUROPEEN

(11) **EP 1 768 643 B1**
(45) Date de publication et mention de la délivrance du brevet: **01.07.2009**
(21) Numéro de dépôt: 05786330.0
(22) Date de dépôt: 01.07.2005
(51) Int. Cl.: A61K 8/81, A61Q 5/02

(54) **COMPOSITIONS COSMETIQUES CONTENANT AU MOINS UN TENSIOACTIF ET AU MOINS UN COPOLYMERE ETHYLENIQUE A GREFFONS POLYVETHYLENEGLYCOL**
KOSMETISCHE ZUSAMMENSETZUNGEN MIT MINDESTENS EINEM TENSID UND MINDESTENS EINEM ETHYLENCOPOLYMER MIT POLYETHYLENGLYCOLPFROPFEN
COSMETIC COMPOSITIONS CONTAINING AT LEAST ONE SURFACTANT AND AT LEAST ONE ETHYLENE COPOLYMER WITH POLYETHYLENE GLYCOL GRAFTS

(30) Priorité: 02.07.2004 FR 0451409; 02.07.2004 FR 0451411; 12.07.2004 US 586726 P; 14.07.2004 US 587553 P
(43) Date de publication de la demande: 04.04.2007
(73) Titulaire: L'ORÉAL, 75008 Paris (FR)
(72) Inventeur: SAMAIN, Henri, F-91570 Bievres (FR); PAUL, Laurence, F-95320 Saint Leu La Foret (FR); JEGOU, Gwenaëlle, F-93190 Livry Gargan (FR)
(74) Mandataire: Le Blainvaux Bellegarde, Françoise
(86) Numéro de dépôt international: PCT/FR2005/001701
(87) Numéro de publication internationale: WO 2006/013270

(56) Documents cités:
- EP-A- 1 323 753
- JP-A- 2002 256 030
- US-A1- 2004 052 746

## Description

La présente invention a trait à des compositions comprenant au moins un tensioactif et au moins un copolymère éthylénique à greffons polyéthylèneglycol.

II est connu d'employer des polymères dans le domaine cosmétique, et notamment en capillaire dans les produits non rinçés, par exemple pour apporter de la tenue ou du coiffant à la chevelure.

Dans le domaine des compositions capillaires dites "rincées", telles que les shampooings ou après-shampooings, on utilise aussi des polymères cationiques synthétiques, solubles dans l'eau, qui sont connus pour apporter une bonne cosmétique aux cheveux; toutefois, ces polymères n'apportent aucun effet de mise en forme des cheveux. II en est de même avec les polymères dérivés naturels cationiques tels les guars modifiées qui apportent également un caractère cosmétique sans permettre une mise en forme. Dans le domaine des compositions rincées, les polymères n'apportent pas suffisamment de coiffant associée à une cosmétique acceptable.

La présente invention a pour but de proposer des compositions cosmétiques comprenant des polymères capables d'apporter un réel effet coiffant tout en conservant une cosmétique acceptable aux compositions, et notamment des polymères peu visqueux qui ne modifient que faiblement la viscosité des compositions les comprenant.

Après de nombreuses recherches, la demanderesse a mis en évidence que l'utilisation de polymères comprenant entre autre des monomères du type (méth)acrylate de polyéthylèneglycol tels que définis ci-après, pouvait permettre la réalisation de compositions coiffantes rinçées ou non rinçées ayant des propriétés cosmétiques adéquates.

Des polymères contenant des motifs (méth)acrylate de polyéthylène glycol (MPEG) sont décrits dans l'art antérieur.

Ainsi, il est connu par EP372546, des copolymères à base de MPEG et de monomères de type (méth)acrylamides d'alkyles en C1-C8, pouvant comprendre des monomères cationiques. Toutefois, ces polymères ne comprennent qu'une faible proportion de monomères cationiques, ce qui ne leur permet pas de générer des effets cosmétiques adéquats, notamment un dépôt sur le cheveu qui soit suffisant pour apporter les propriétés recherchées.

Le document JP2002-322219 décrit des polymères contenant des motifs MPEG en association avec des monomères hydrophobes à base de polypropylène glycol (PPO) ou de polyoxyde de tétraméthylène, et des monomères cationiques. Or, on a constaté que ces polymères qui comprennent des monomères hydrophobes, ne permettent pas d'obtenir des propriétés cosmétiques satisfaisantes.

L'exemple 6 de EP1323753 contient un tensioactif anionique, un tensioactif amphotère et u copolymère cationique.

JP2002-256 030 décrit des compositions de nettoyage comprenant des copolymère de méthacrylate dedimethylaminopropylammonium et méthacrylate de PEG et des tensioactifs non ioniques.

US2004/052746 décrit une composition de shampooing comprenant un polymère associatif cationique et des tensioactifs anioniques, non ioniques et amphotères.

II est également connu par le brevet JP2002-284627 une composition comprenant des polymères cationiques dans lequel les monomères de type PEG sont associés à des monomères comprenant des motifs amines quaternaires. Or, la présence de motifs quaternaires peut induire, au fur et à mesure des applications, un surcroît de dépôt qui peut nuire, dans certains cas, à la qualité cosmétique de la composition. Par ailleurs, ces polymères contiennent un taux de charge cationique faible, de l'ordre de 0,5 à 6%, qui ne permet pas une affinité optimale pour le cheveu.

Le document JP2000-302649 décrit une composition capillaire comprenant un polymère qui comprend des monomères cationiques ou amphotères, des monomères à groupement polyéther, notamment de type PEG ou PPO, ainsi que des monomères optionnels qui peuvent être principalement hydrophobes (par exemple méthacrylate de stéaryle).

II est également connu par le brevet JP07-285831, des compositions capillaires comprenant un polymère qui comprend des monomères de type MPEG en association avec des monomères ioniques, cationiques ou amphotères, et des monomères additionnels de type (méth)acrylates d'alkyle en C1-C24, principalement hydrophobes. Toutefois, la présence de comonomères hydrophobes, par exemple de type acrylate de butyle ou de stéaryle, ne permet pas d'obtenir des propriétés cosmétiques adéquates, notamment ne permettent pas d'obtenir un bon démêlage des cheveux mouillés, juste après le shampoing.

On connaît encore la demande WO03/075867 qui décrit des copolymères blocs linéaires comportant une séquence poly(alkylène glycol) encadrée par deux séquences éthyléniques. Ces polymères présentent le défaut d'avoir une séquence centrale de type poly(alkylène glycol) de masse élevée qui confère au polymère une forte cristallinité, ce qui peut conduire à des produits opaques et/ou présentant un caractère gras.

La demanderesse a mis en évidence de nouveaux polymères permettant d'apporter un effet coiffant et conditionneur aux produits cosmétiques capillaires.

Sans être tenu par la présente explication, on peut penser que ceci peut notamment être dû à la présence de motifs (méth)acrylate de PEG (MPEG) au sein de la chaîne de polymère, motifs qui contribuent en grande part à l'effet obtenu. On a en effet constaté que cet effet n'était pas obtenu avec un simple mélange de polymère cationique et de polymère de type PEG.

De manière surprenante, les polymères selon l'invention possèdent des propriétés cosmétiques intéressantes, par exemple lors de l'application dans une composition de shampooing comprenant une association de tensioactifs particuliers; on a en effet constaté que les cheveux se démêlent facilement lors du shampooing, et qu'ils présentent de la douceur; après séchage, les compositions selon l'invention permettent également, une fois la chevelure séchée, une mise en forme des cheveux particulièrement intéressante.

Les compositions cosmétiques selon l'invention sont caractérisées par le fait qu'elles comprennent :
I) au moins un tensioactif anionique et au moins un tensioactif non ionique et
II) au moins un copolymère éthylénique comprenant, en % en poids par rapport au poids total du polymère, :
   - a) 10-60% en poids d'un ou plusieurs monomères de formule (1) telle que définie ci-après ;
   - b) 40-90% en poids d'au moins un monomère "essentiellement cationique" choisi parmi :
      - (i) un ou plusieurs monomères cationiques de formule (IIa),
      - (ii) un ou plusieurs monomères amphotères de formules (IIc) et (IId), et
      - (iii) un mélange d'un ou plusieurs monomères cationiques de formule (IIa) avec un ou plusieurs monomères anioniques choisis parmi l'anhydride maléique et/ou ceux de formule (IIb); et/ou avec un ou plusieurs monomères amphotères choisis parmi ceux de formules (IIc) et (IId).
   - c) et éventuellement 0-50% en poids de monomères hydrophiles non ioniques, à l'exclusion de acrylate de méthyle, du méthacrylate de méthyle et de l'acrylate d'isopropyle s'ils sont présents en une quantité supérieure ou égale à 10% en poids.

Dans la suite de la présente description, on entendra par 'radical cyclique' un radical monocyclique ou polycyclique, qui peut se présenter lui-même sous forme d'un ou plusieurs cycles, saturés et/ou insaturés, éventuellement substitués (par exemple cyclohexyle, cyclodécyle, benzyle ou fluorényle), mais également un radical qui comprend un ou plusieurs desdits cycles (par exemple p-tertbutylcyclohexyle ou 4-hydroxybenzyle).

On entendra par'radical saturé et/ou insaturé', les radicaux totalement saturés, les radicaux totalement insaturés, y compris aromatiques, ainsi que les radicaux comportant une ou plusieurs doubles et/ou triples liaisons, le reste des liaisons étant des liaisons simples.

Les tensioactifs anioniques parmi lesquels on peut citer, seuls ou mélanges, les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffinesulfonates; les alkylsulfosuccinates, les alkyléthersulfosuccinates, les alkylamidesulfosuccinates; les alkylsulfosuccinamates; les alkylsulfoacétates ; les alkylétherphosphates; les acylsarcosinates; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle. On peut également citer les sels d'acides gras tels que les sels des acides oléique, ricinoléique, palmitique, stéarique, les acides d'huile de coprah ou d'huile de coprah hydrogénée; les acyl-lactylates dont le radical acyle comporte 8 à 20 atomes de carbone; les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'éthylène, et leurs mélanges. Les acides gras ne sont pas préférés.

Parmi les tensioactifs anioniques, on préfère utiliser les sels des alkylsufates, les alkyléthersulfates, les alkyléthercarboxylates, et leur mélanges, en particulier sous forme de sels de métaux alcalins ( N, K) ou alcalinaux terreux (par ex Mg), d'ammonium, d'amine ou d'aminoalcool et leurs mélanges

La quantité de tensioactifs anioniques va de préférence de 3% à 40% en poids, en particulier entre 5% et 25% en poids, rapportée au poids total de la composition cosmétique.
- Les tensioactifs non ioniques parmi lesquels on peut citer, seuls ou mélanges, les alcools, les alpha-diols, les alkylphénols ou les acides gras polyéthoxylés, polypropoxylés ou polyglycérolés, ayant une chaîne grasse comportant par exemple 8 à 22 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.

De préfémece, le tensioactif non ionique choisi parmi :
- les alcools gras glycérolés ;
- les alkylpolyglycosides.

Par chaîne grasse, on entend une chaîne hydrocarbonée comprenant 6 à 30 atomes de carbone, de préférence de 8 à 24 atomes de carbone, linéaire ou ramifiée, saturée ou non.

En ce qui concerne les alkypolyglycosides, ces composés sont bien connus et peuvent être plus particulièrement représenté par la formule générale suivante :

R₁O-(R₂O)ₜ (G)ᵥ (II)

dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un motif sucre comportant de 5 à 6 atomes de carbone, t désigne une valeur allant de 0 à 10, de préférence 0 à 4, de préférence 0 à 4 et v désigne une valeur allant de 1 à 15.

Des alkylpolyglycosides préférés selon la présente invention sont des composés de formule (II) dans laquelle R₁ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (II), peut aller de 1 à 15, de préférence de 1 à 4. Le degré moyen de polymérisation est plus particulièrement compris entre 1 et 2 et encore plus préférenciellement de 1,1 à 1,5. Les liaisons glycosidiques entre les motifs sucre sont de type 1-6 ou 1-4 et de préférence 1-4.

Des composés de formule (11) sont notamment représentés par les produits vendus par la société COGNIS sous les dénominations PLANTAREN^{®} (600 CS/U, 1200 et 2000) ou PLANTACARE^{®} (818, 1200 et 2000). On peut également utiliser les produits vendus par la société SEPPIC sous les dénominations TRITON CG 110 (ou ORAMIX CG 110) et TRITON CG 312 (ou ORAMIX^{®} NS 10), les produits vendus par la société B.A.S.F. sous la dénomination LUTENSOL GD 70 ou encore ceux vendus par la société CHEM Y sous la dénomination AG10 LK. On peut également utiliser par exemple, l'Alkyl en C8/C16 polyglucoside 1,4 en solution aqueuse à 53% commercialisé par COGNIS sous la référence PLANTACARE^{®} 818 UP.

En ce qui concerne les tensioactifs mono ou polyglycérolés, ils comportent de préférence en moyenne de 1 à 30 groupements glycérol, plus particulièrement de 1 à 10 groupements glycérol et en particulier de 1,5 à 5.

Les tensioactifs monoglycérolés ou polyglycérolés sont de préférence choisis parmi les composés suivants :
A) les composés de formule suivantes : RO[CH₂CH(CH₂OH)O]ₘH RO[CH₂CH(OH)CH₂O]ₘH ou RO[CH(CH₂OH)CH₂O]ₘH ; dans laquelle R représente un radical hydrocarboné saturé ou insaturé, linéaire ou ramifié, comportant de 8 à 40 atomes de carbone et de préférence de 10 à 30 atomes de carbone ; m est un nombre compris entre 1 et 30, de préférence entre 1 et 10, plus particulièrement de 1,5 à 6.
   R peut éventuellement comprendre des hétéroatomes tels que par exemple oxygène et azote. En particulier, R peut éventuellement comprendre un ou plusieurs groupements hydroxy et/ou éther et/ou amide.
   R désigne de préférence des radicaux alkyle et/ou alkényle en C10-C20, éventuellement mono ou polyhydroxylé.

On peut utiliser par exemple, l'hydroxylauryléther polyglycérolé (3.5 moles) commercialisé sous la dénomination Chimexane® NF de Chimex.

Parmi les tensioactifs non-ioniques on utilise de préférence les alkyl C6-C24 polyglucosides et plus particulièrement les alkyl C8-C16 polyglucoside.

La quantité totale de tensioactifs non ioniques va de préférence de 0.5% à 25% en poids, en particulier de 1% à 20% en poids, rapportée au poids total de la composition cosmétique et plus particulièrement de 2 à 10% en poids.

De préférence la composition peut également comprendre au moins un tensioactif amphotère.
- les tensioactifs amphotères parmi lesquels on peut citer, seuls ou mélanges, les dérivés d'amines secondaires ou tertiaires aliphatiques, dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate); on peut citer encore les alkyl (C₈-C₂₀) bétaïnes, les sulfobétaïnes, les alkyl (C₈-C₂₀) amidoalkyl (C₁-C₆) bétaïnes telles que la cocoamidopropylbétaïne ou les alkyl (C₈-C₂₀) amidoalkyl (C₁-C₆) sulfobétaïnes.

Parmi les tensioactifs amphotères on utilise de préférence les alkyle C8-C20 bétaïnes, les alkyle C8-C20 amido alkyle C6-C8 bétaïnes, les alkylamphodiacétates et leur mélanges.

La quantité totale de tensioactifs amphotères va de préférence de 0.5% à 20% en poids, en particulier de 1% à 10% en poids, rapportée au poids total de la composition cosmétique et plus particulièrement de 1 à 5% en poids.

Parmi les mélanges de tensioactifs utilisables, on préfère les associations de tensioactifs anioniques , de tensioactif non ioniques et de tensioactifs amphotères.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec un tensioactif non ionique et :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société COGNIS.

La quantité totale de tensioactifs va notamment de 3,5% à 50% en poids, de préférence de 5% à 30% en poids, et encore plus préférentiellement de 8% à 25% en poids, par rapport au poids total de la composition.

### Copolymère éthylénique :

Le copolymère éthylénique selon invention comprend donc au moins un monomère de formule (I), qui peut être présent seul ou en mélange, : 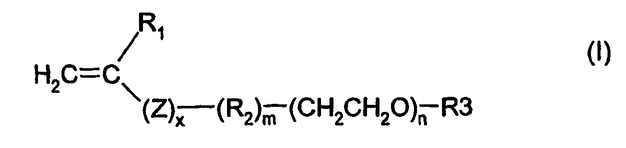 dans laquelle:
- R1 est un atome d'hydrogène ou un radical hydrocarboné, linéaire ou ramifié, de type CₚH₂ₚ₊₁, avec p étant un entier compris entre 1 et 12 inclus;
- Z est un groupement divalent choisi parmi -COO-, -CONH-, -CONCH₃-, -OCO- , - O-, -SO₂- -CO-O-CO- ou -CO-CH₂-CO-;
- x est 0 ou 1;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P; et leurs sels.

Notamment, R1 peut représenter un radical méthyle, éthyle, propyle, butyle. De préférence, R1 représente l'hydrogène ou un radical méthyle.

De préférence, Z représente COO ou CONH.

De préférence, x est égal à 1.

Dans le radical R2, le ou les hétéroatomes, quand ils sont présents, peuvent être intercalés dans la chaîne dudit radical R2, ou bien ledit radical R2 peut être substitué par un ou plusieurs groupes les comprenant tels que hydroxy ou amino (NH2, NHR' ou NR'R" avec R' et R" identiques ou différents représentant un alkyle linéaire ou ramifié en C1-C22, notamment méthyle ou éthyle).

Notamment R2 peut être :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi O, N, S, F, Si et P; ou bien un radical benzylène -C₆H₄-CH₂- éventuellement substitué, par un radical alkyle en C1-C12 comprenant éventuellement 1 à 8 hétéroatomes choisis parmi O, N, S, F, Si et P;
- un radical pyridinium de formule : 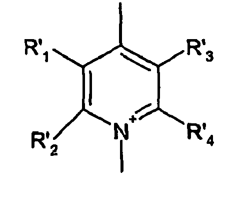 avec R'1 à R'4, identiques ou différents, choisis parmi H et un radical alkyle en C1-C12 comprenant éventuellement 1 à 8 hétéroatomes choisis parmi O, N, S, F, Si et P; notamment R'1 à R'4 peuvent être méthyle et/ou éthyle;
- un radical de formule -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH-, -CH₂-CH₂-NH-CO-NH- ;-CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-. -CH₂-CH(NR'R")-, -CH₂-CH₂-CH₂-NR'-, -CH₂-CH₂-CH₂-O-; -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux.

De préférence, n est compris entre 5 et 200 inclus, et encore mieux entre 7 et 100 inclus, voire entre 9 et 50 inclus.

De préférence, R3 est un atome d'hydrogène; un radical benzyle ou phényle éventuellement substitué par un radical alkyle en C1-C12 comprenant éventuellement 1 à 8 hétéroatomes choisis parmi O, N, S, F, Si et P; un radical alkyle en C1-C30, notamment C1-C22, voire C2-C16, comprenant éventuellement 1 à 18 hétéroatomes choisis parmi O, N, S, F, Si et P.

Ces radicaux benzyle, phényle ou alkyle peuvent comprendre notamment une fonction choisie parmi les fonctions suivantes:

| | | | | |
|---|---|---|---|---|
| Succinimido 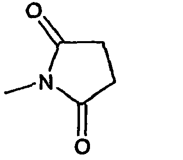 | Glutarate-succinimido 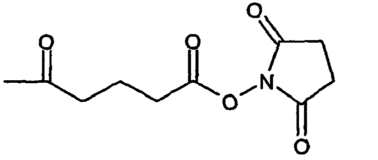 | | Glutarate 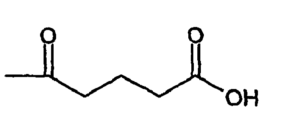 | |
| maléimido 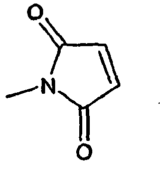 | | Mésityle 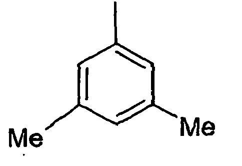 | Benzoate 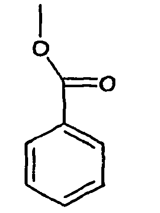 | |
| Tosyle 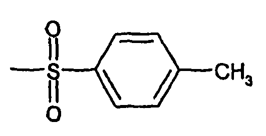 | | Triéthoxysilane 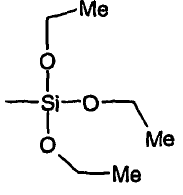 | Phtalimide 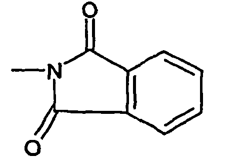 | |
| Thioester 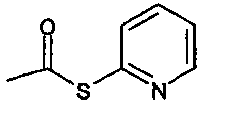 | Benzotriazole carbonate 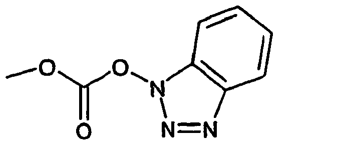 | | | Butyraldéhyde 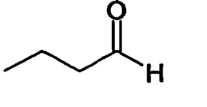 |
| Acétaldéhyde diéthylacétal 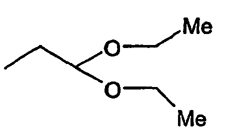 | | Biotine 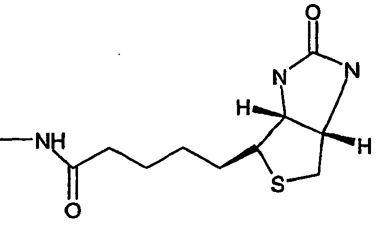 | | |
| Phospholipide 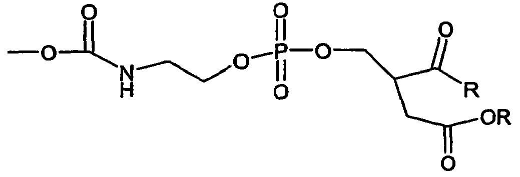 avec R = alkyle en C12-C18, et notamment lauryle, myristyle, palmityle, stéaryle, oléyle ou linoléyle | | | Succinate N-hydroxysuccinimide 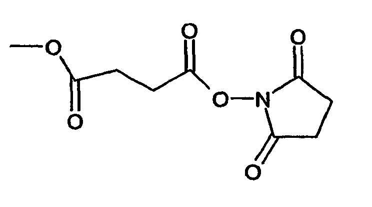 | |

ou encore choisie parmi -SO₃H, -COOH, -PO₄, -NR5R6 ou -N⁺R5R6R7, avec R5, R6 et R7, indépendamment l'un de l'autre, choisis parmi H ou alkyles en C1-C18, linéaire, ramifié ou cyclique, notamment méthyle, comprenant éventuellement 1 ou plusieurs hétéroatomes ou encore portant des groupements protecteurs tels que le t-butyloxycarbonyle (aussi appelé BOC) ou le 9-fluorenylmethoxycarbonyle (aussi appelé FmoC).

Parmi les radicaux R3, on peut citer les chaînes méthyle, éthyle, propyle, benzyle, éthylhexyle, lauryle, stéaryle, béhényle (-(CH₂)₂₁-CH₃), et également les chaînes alkyles fluorées telles que par exemple heptadecafluorooctyl sulfonyl amino éthyle CF₃-(CF₂)₇-SO₂-N(C₂H₅)-CH₂-CH₂; ou encore les chaînes -CH₂-CH₂-CN, succinimido, maléimido, mésityle, tosyle, triéthoxysilane ou phtalimide.

Les motifs amines et/ou les groupes anioniques du monomère de formule (I) peuvent éventuellement être neutralisés.

Les motifs amines du monomère peuvent éventuellement être neutralisés. Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. II peut s'agir d'acides aliphatiques linéaires, ramifiés ou cycliques
ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

La neutralisation des groupes anioniques peut être effectuée par une base minérale, telle que LiOH, NaOH, KOH, Ca(OH)₂, NH₄OH, Mg(OH)₂ ou n(OH)₂; ou par une base organique telle qu'une alkylamine primaire, secondaire ou tertiaire, notamment la triéthylamine ou la butylamine. Cette alkylamine primaire, secondaire
ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la dimethylamino2-propanol. On peut encore citer la lysine ou la 3-(diméthylamino)propylamine.

Parmi les monomères de formule (I) particulièrement préférés, on peut citer :
- le (méth)acrylate de poly(éthylène glycol) dans lequel R1 est H ou méthyle; Z est COO, x = 1, m=0 et R3 = H;
- le (méth)acrylate de méthyl-poly(éthylène glycol), aussi appelé (méth)acrylate de méthoxy-poly(éthylèneglycol), dans lequel R1 est H ou méthyle, Z est COO, x = 1, m=0 et R3 = méthyle;
- les (méth)acrylate d'alkyl-poly(éthylène glycol) dans lequel R1 est H ou méthyle,
   Z est COO, x = 1, m=0 et R3 = alkyl.
- les (méth)acrylates de phényl-poly(éthylène glycol), aussi appelé (méth)acrylate de poly(éthylène glycol) phényl éther, dans lequel R1 est H ou méthyl, Z est COO, x = 1, m=0 et R3 = phényle;
- le monomère suivant : 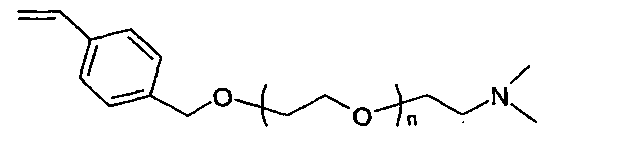
dans lequel n est de préférence compris entre 3 et 100 inclus, notamment 5 à 50 inclus, voire 7 à 30 inclus.

Les monomères de formule (I) tout particulièrement préférés sont choisis parmi les (méth)acrylates de poly(éthylène glycol) et les (méth)acrylates de méthylpoly(éthylène glycol), de préférence ceux ayant un poids moléculaire compris entre 350 et 15 000 g/mol., notamment entre 500 et 8000 g/mol.

Tout particulièrement, on préfère les (méth)acrylates de poly(éthylène glycol) et en particulier ceux ayant un poids moléculaire entre 350 et 15 000 g/mol., notamment entre 500 et 8000 g/mol.

Des exemples de monomères commerciaux sont :
- le CD 350 (méthacrylate de méthoxy-poly(éthylène glycol 350) et le CD 550 (méthacrylate de méthoxy-poly(éthylène glycol 550), fourni par SARTOMER Chemicals; .
- le M90G (méthacrylate de méthoxy-poly(éthylène glycol (9 unités de répétition)) et le M230G (méthacrylate de méthoxy-polyéthylène glycol (23 unités de répétitions)) disponibles chez Shin-Nakamura Chemicals;
- les méthacrylates de méthoxy-poly(éthylène glycol) de poids moléculaires moyens 300, 475 ou 1100, disponibles chez Sigma-Aldrich;
- l'acrylate de méthoxy-poly(éthylène glycol) de poids moléculaire moyen 426 disponible chez Sigma-Aldrich;
- les méthacrylates de méthoxy-poly(éthylène glycol) disponibles chez LAPORTE sous les dénominations commerciales : MPEG 350, MPEG 550, S10W, S20W.
- les poly(éthylène glycol) monomethyl éther, mono(succinimidyl succinate) ester de poids moléculaire moyen 1900 ou 5000, de chez Polysciences;
- le méthacrylate de behenyl poly(éthylène-glycol PEG-25), disponible chez Rhodia, sous la dénomination SIPOMER BEM;
- les acrylates de poly(éthylène glycol) phényl éther de poids moléculaires moyens 236, 280 ou 324 disponibles chez Aldrich;
- le méthoxy polyéthylène glycol 5000 2-(vinyl sulfonyl) éthyl éther disponible commercialement chez Fluka;
- le méthacrylate de polyéthylène glycol éthyl éther disponible chez Aldrich ;
- les méthacrylates de polyéthylène glycol 8000, 4000, 2000 de Monomer & Polymer Dajac laboratories ;
- le polyéthylène glycol N-hydroxy succinimide vinyl sulfone disponible commercialement chez Nektar Molecule enginnering ( Shearwater).

De préférence, le monomère de formule (I) a un poids moléculaire compris entre 350 et 15 000 g/mol., notamment entre 500 et 8000 g/mol.

Le monomère de formule (I), seul ou en mélange, est présent à raison de 10% inclus à 60% exclus en poids, par rapport au poids du polymère final, notamment de 20% inclus à 55% inclus en poids, de préférence de 30% inclus à 50% inclus en poids.

### Motifs ioniques

Le copolymère éthylénique selon l'invention comprend également au moins un monomère "essentiellement cationique", ou l'un de ses sels, choisi parmi :
- (i) un ou plusieurs monomères cationiques de formule (IIa),
- (ii) un ou plusieurs monomères amphotères de formules (IIc) et (IId), et
- (iii) un mélange d'un ou plusieurs monomères cationiques de formule (IIa) avec un ou plusieurs monomères anioniques choisis parmi l'anhydride maléique et/ou ceux de formule (IIb); et/ou avec un ou plusieurs monomères amphotères choisis parmi ceux de formules (IIc) et (IId).

De préférence, le monomère "essentiellement cationique" est choisi parmi les monomères cationiques de formule (IIa) et les monomères amphotères de formule (IIc) ou (IId), préférentiellement parmi les monomères cationiques de formule (IIa).

Par monomère cationique, on entend un monomère comprenant des motifs capables de posséder une charge cationique dans le domaine de pH compris entre 3 et 12. Ces motifs ne possèdent pas obligatoirement une charge permanente quelque soit le pH. L'unité cationique n'a pas besoin d'être protonée à chacun de ces pH. 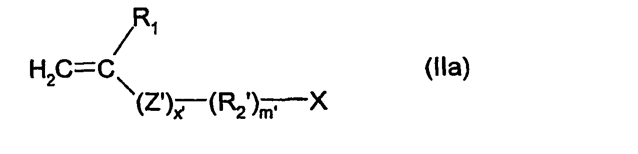 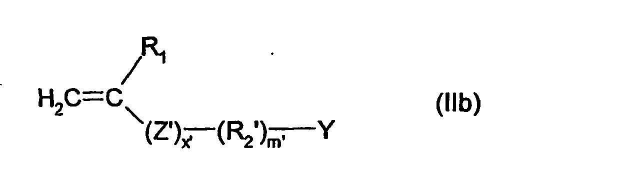 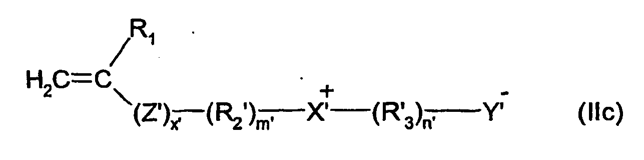 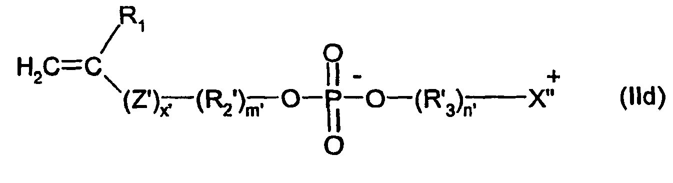 dans lesquelles :
- R1 est un atome d'hydrogène ou un radical hydrocarboné, linéaire ou ramifié, de type CₚH₂ₚ₊₁, avec p étant un entier compris entre 1 et 12, inclus; Notamment, R1 peut représenter un radical méthyle, éthyle, propyle, isopropyle, n-butyle, isobutyle, tertio-butyle. De préférence, R1 représente l'hydrogène ou un radical méthyle.
- Z' est un groupement divalent choisi parmi -COO-, -CONH-, -CONCH₃-, -OCO-
ou -O-, -SO₂- -CO-O-CO- ou -CO-CH₂-CO-;
De préférence, Z' est choisi parmi COO et CONH.
- x' est 0 ou 1, de préférence 1.
- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P; Dans le radical R'2, le ou les hétéroatomes, quand ils sont présents, peuvent être intercalés dans la chaîne dudit radical R'2, ou bien ledit radical R'2 peut être substitué par un ou plusieurs groupes les comprenant tels que hydroxy ou amino (NH2, NHR' ou NR'R" avec R' et R" identiques ou différents représentant un alkyle linéaire ou ramifié en C1-C22, notamment méthyle ou éthyle). Notamment R'2 peut être :

- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi N, O, S, F, Si et/ou P; ou bien un radical benzylène -C₆H₄-CH₂- éventuellement substitué, par un radical alkyle en C1-C12 comprenant éventuellement 1 à 25 hétéroatomes choisis parmi O, N, S, F, Si et P;
- un radical de formule -CH₂-O-CO-O-, CH₂-CH₂-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH2-CH(NHR')-, -CH₂-CH₂-CH(NR'R")-, -CH₂-CH(NR'R")-, -CH₂-CH₂-CH₂-NR'-, - CH₂-CH₂-CH₂-O-; -CH₂-CH₂-CHR'-O- avec R' et R" représentant un alkyle linéaire
ou ramifié en C1-C22 comprenant éventuellement 1 à 12 hétéroatomes choisis parmi O, N, S, F, Si et P;
- ou un mélange de ces radicaux;
- m' est 0 ou 1;
- X (dans la formule IIa) est un groupe de formule -N(R₆)(R₇) ou -P(R₆)(R₇) ou - P⁺R₆R₇R₈, avec R6, R7 et R8 représentant, indépendamment l'un de l'autre, soit (i) un atome d'hydrogène, soit (ii) un groupement alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, Si et P; soit (iii) R6 et R7 peuvent former avec l'atome d'azote ou de phosphore, un premier cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N.
   Par exemple, R6 et R7 peuvent être choisis parmi l'hydrogène, un groupement méthyle, éthyle, propyle, isopropyle, n-butyle, t-butyle, isobutyle, octyle, lauryle, stéaryle.
   De préférence R6 et R7 sont choisis, indépendamment l'un de l'autre, parmi H, CH3 et C2H5.

De manière alternative, X peut représenter un groupement -R'6-N-R'7- dans lequel R'6 et R'7 forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N.

Par exemple, X peut constituer un cycle aromatique ou non comportant un groupement amine tertiaire ou peut représenter un hétérocycle aromatique ou non, contenant un azote tertiaire.
Parmi ces radicaux X préférés, on peut citer les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, phosphonium, et leurs mélanges.
Les groupements guanidino et amidino sont respectivement de formule :

| | |
|---|---|
| Guanidino 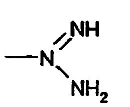 | Amidino 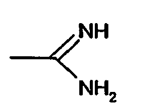 |

Les monomères de formule (IIa) peuvent être neutralisés par des agents neutralisants de nature chimique différentes.

L'agent neutralisant peut être choisi parmi les acides minéraux ou organiques au sens de Bronsted et de préférence parmi les acides organiques. De manière avantageuse, il peut être choisi parmi les agents neutralisants ayant un log P inférieur
ou égal à 2, par exemple compris entre -8 et 2, de préférence compris entre -6 et 1, notamment compris entre -6 et 0.
II peut également être choisi parmi les agents ayant un log P supérieur à 2, de préférence supérieur ou égal à 2,5, notamment supérieur à 3, et en particulier compris entre 3 et 15, voire entre 3,5 et 10.

Ainsi que précisé plus loin, les valeurs de log P sont connues et sont déterminées selon un test standard qui détermine la concentration de l'agent neutralisant dans l'octanol-1 et l'eau.

Les acides minéraux pouvant être utilisés sont notamment l'acide sulfurique, l'acide chlorhydrique, l'acide bromhydrique, l'acide iodhydrique, l'acide phosphorique, l'acide borique.

Les agents neutralisants de type acides organiques peuvent être choisis parmi les acides aliphatiques linéaires, ramifiés ou cycliques et/ou les acides insaturés ou aromatiques, et peuvent notamment comprendre 1 à 1000 atomes de carbone, notamment 2 à 500 atomes de carbone. Ils possèdent au moins une fonction acide au sens de Bronsted, et notamment un ou plusieurs groupes acide carboxylique, sulfonique et/ou phosphonique. IIs peuvent comporter, en outre, un ou plusieurs hétéroatomes choisis parmi O, N, Si, F et P, par exemple sous la forme de groupes hydroxyle.

On peut en particulier employer comme neutralisant des acides gras ayant 6 à 32 atomes de carbone, notamment 8 à 28, linéaires, ramifiés ou cycliques, saturés ou insaturés, éventuellement aromatiques, et comportant au moins une fonction

COOH ou acide sulfonique (-SO₃H).

On peut également employer des hydroxyacides, notamment des alphahydroxy-acides, ayant 6 à 32 atomes de carbone, notamment 8 à 28, linéaires, ramifiés ou cycliques, saturés ou insaturés, éventuellement aromatiques, et comportant au moins une fonction COOH ou acide sulfonique (-SO₃H).

On peut encore employer des acides alkylbenzène-sulfoniques, dans lesquels le groupement alkyle peut comprendre de 4 à 30, notamment 6 à 24, atomes de carbone.

On peut encore employer des agents neutralisants amphotères, notamment du type alkyl-bétaïnes ou alkylamidopropylbétaïnes, dans lesquels le groupement alkyle peut comprendre 4 à 30, notamment 6 à 24, atomes de carbone; on peut en particulier citer la cocoamidopropylbétaïne.

On peut notamment citer l'acide alpha-hydroxyéthanoïque, l'acide alpha-hydroxyoctanoïque, l'acide alpha-hydroxycaprylique, l'acide ascorbique, l'acide acétique, l'acide benzoïque, l'acide béhénique, l'acide caprique, l'acide citrique, l'acide caproïque, l'acide caprylique, l'acide dodécylbenzène sulfonique, l'acide 2-éthylcaproïque, l'acide folique, l'acide fumarique, l'acide galactarique, l'acide gluconique, l'acide glycolique, l'acide 2-hexadécyl eicosanoïque, l'acide hydroxycaproique, l'acide 12-hydroxystéarique, l'acide isolaurique (ou 2-butyl octanoïque), l'acide isomyristique (ou 2-hexyl octanoïque), l'acide isoarachidique (ou 2-octyl dodécanoïque), l'acide isolignocérique (ou 2-décyl tétradécanoïque), l'acide lactique, l'acide laurique, l'acide malique, l'acide myristique, l'acide oléïque, l'acide palmitique, l'acide propionique, l'acide sébacique, l'acide stéarique, l'acide tartrique, l'acide téréphtalique, l'acide trimésique, l'acide undécylénique, la propylebétaïne, la cocoamidopropylbétaïne, et le chlorhydrate de bétaïne de formule [(CH₃)₃N+CH₂CO₂H.Cl-], ainsi que leurs mélanges.

De préférence, on peut utiliser comme agent neutralisant, l'acide caproïque, l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement le chlorhydrate de bétaïne et/ou l'acide béhénique.

A titre d'information, on donne ci-après la valeur de log P de certains acides usuels :

| | |
|---|---|
| Acide sulfurique | -1.031 +/-0.613 |
| Acide acétique | -0.285+/-0.184 |
| Acide propionique | 0.246+/-0.184 |
| Acide citrique | - 1.721+1-0.396 |
| Acide gluconique | -3.175 +/- 0.852 |
| Acide borique | -0.292+/-0.753 |
| Acide phosphorique | -2.148+/-0.587 |
| Acide benzoïque | 1.895+/-0.206 |
| Acide stéarique | 8.216 +/-0.186 |
| Acide béhénique | 10.342+/-0.186 |
| Acide oléique | 7.698 +/- 0.199 |

Par neutralisation, on entend l'action d'un acide, organique selon l'invention, et comprenant au moins une fonction acide au sens de Bronsted, sur tout ou partie des monomères et/ou polymère ci-dessus mentionné, comprenant au moins une fonction basique au sens de Bronsted.

L'agent neutralisant, seul ou en mélange, peut être ajouté en une quantité de 0,01 à 3 équivalent molaire, notamment 0,05 à 2,5, voire 0,1 à 2 équivalent molaire, par rapport aux fonctions amines totales du polymère ou des monomères.

II est ainsi possible de sous-neutraliser le polymère, c'est-à-dire que l'agent neutralisant peut être présent en une quantité nécessaire pour neutraliser 1 à 99%, notamment 5 à 90%, voire 10 à 80%, des fonctions amines totales du polymère ou des monomères; ce qui signifie qu'il est présent en une quantité de 0,01 à 0,99 équivalent molaire, notamment 0,05 à 0,9, voire 0,1 à 0,8 équivalent molaire.
II est également possible de sur-neutraliser le polymère, c'est-à-dire que l'agent neutralisant peut être présent en une quantité nécessaire pour neutraliser 101 à 300%, notamment de 120 à 250%, voire de 150 à 200%, des fonctions amines totales du polymère ou des monomères; ceci peut être le cas lorsque l'on souhaite assurer au polymère une gamme de pH et/ou une force ionique adéquate vis-à-vis des formulations envisagées. II peut donc être présent en une quantité de 1,01 à 3 équivalent molaire, notamment 1,2 à 2,5, voire 1,5 à 2 équivalent molaire, par rapport aux fonctions amines totales du polymère ou des monomères.
De préférence, l'agent neutralisant, seul ou en mélange, est présent en une quantité stoechiométrique par rapport aux fonctions amines totales du polymère ou des monomères; il est donc présent en une quantité nécessaire pour neutraliser 100% des motifs amines du polymère ou des monomères, soit 1 équivalent molaire.

Préférentiellement, la nature et la quantité d'agent neutralisant peut être déterminée par l'homme du métier de manière à obtenir au final un polymère soluble ou dispersible dans l'eau.

Parmi les monomères de formule (IIa) préférés, on peut citer :

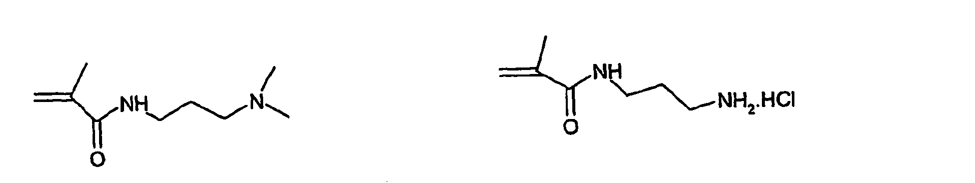

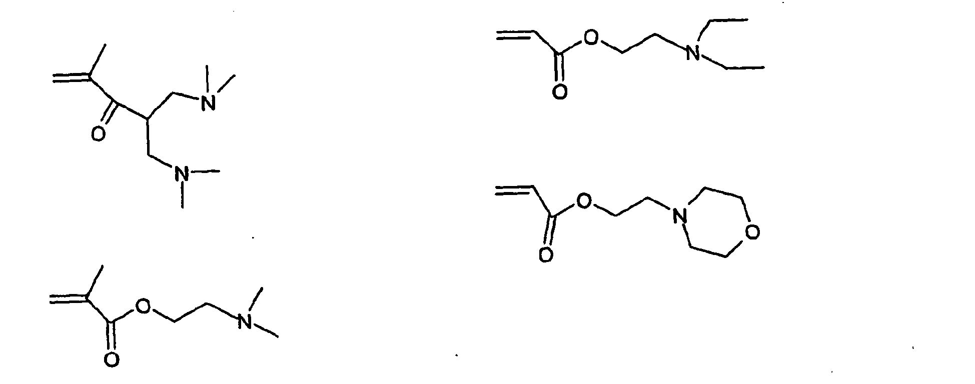

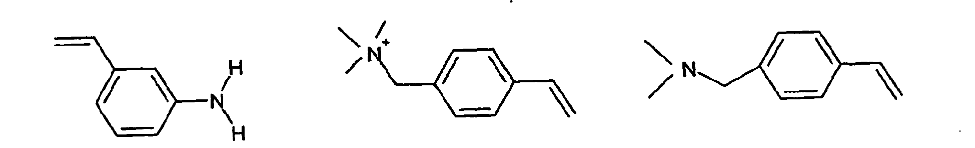

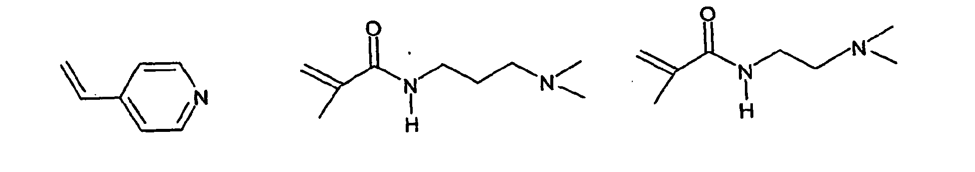

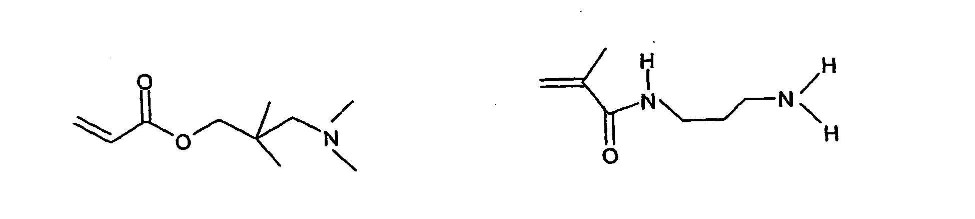

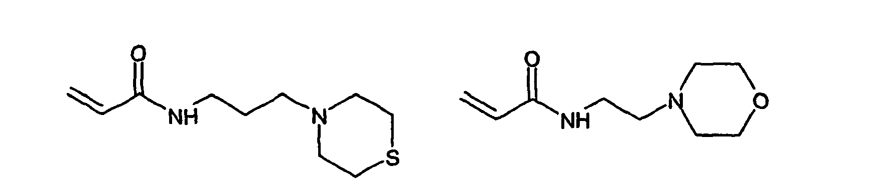

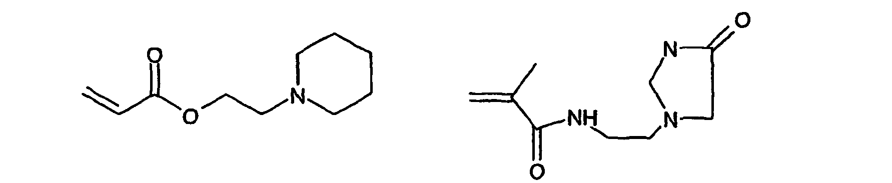

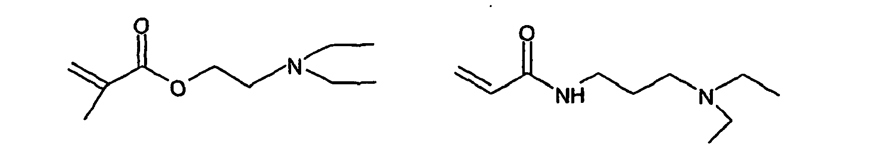

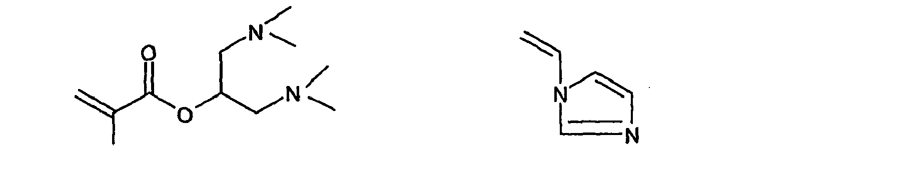

Parmi les monomères de formule (IIa) particulièrement préférés, on peut citer le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle et plus particulièrement le (méth)acrylamide de diméthylaminopropyle.

Dans la formule (IIb), la signification des radicaux R1, Z', x', R'2 et m' est la même que celle donnée ci-dessus pour la formule (IIa).
Dans la formule (IIb), Y est un groupement choisi parmi -COOH, -SO₃H, -OSO₃H, -PO₃H₂ et -OPO₃H₂.

La neutralisation des groupes anioniques peut être effectuée par une base minérale, telle que LiOH, NaOH, OH, Ca(OH)₂, NH₄OH, Mg(OH)₂ ou Zn(OH)₂; ou par une base organique telle qu'une alkylamine primaire, secondaire ou tertiaire, notamment la triéthylamine ou la butylamine. Cette alkylamine primaire, secondaire
ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la dimethylamino2-propanol. On peut encore citer la lysine ou la 3-(diméthylamino)propylamine.

II est entendu que selon l'état de l'art, les groupes SO₄H₂ et PO₄H₂ sont liés à R'2 par l'atome d'oxygène tandis que les groupes SO₃H et PO₃H sont liés à R'2 respectivement via les atomes de S et P.

Parmi les monomères anioniques préférés, on peut citer l'anhydride maléique et les monomères de formule (IIb) préférés suivants : l'acide acrylique, l'acide méthacrylique, l'acide crotonique, l'acide itaconique, l'acide fumarique, l'acide maléique, l'acrylate de 2-carboxyéthyle (CH2=CH-C(O)-O-(CH₂)₂-COOH); l'acide styrènesulfonique, l'acide 2-acrylamido 2-methylpropanesulfonique, l'acide vinylbenzoïque, l'acide vinylphosphorique, le (méth)acrylate de sulfopropyle, et les sels de ceux-ci.

Dans la formule (IIc), la signification des radicaux R1, Z', x', R'2 et m' est la même que celle donnée ci-dessus pour la formule (IIa).
Les autres radicaux ont la signification suivante :
- X'⁺ est un groupe divalent de formule -N⁺(R₆)(R₇)- avec R6 et R7 représentant, indépendamment l'un de l'autre, soit (i) un atome d'hydrogène, soit (ii) un groupement alkyle linéaire, ramifié ou cyclique, éventuellement aromatique, comprenant de 1 à 25 atomes de carbone, pouvant comprendre 1 à 20 hétéroatomes choisis parmi O, N, S et P; soit (iii) R6 et R7 peuvent former avec l'atome d'azote un premier cycle, saturé ou insaturé, éventuellement aromatique, comprenant au total 5, 6, 7 ou 8 atomes, et notamment 4, 5, 6 ou 7 atomes de carbone et/ou 2 à 3 hétéroatomes choisi parmi O, S et N; ledit premier cycle pouvant être fusionné avec un
ou plusieurs autres cycles, saturés ou insaturés, éventuellement aromatiques, comprenant chacun 5, 6, 7 ou 8 atomes, et notamment 4, 5, 6 ou 7 atomes de carbone et/ou 2 à 3 hétéroatomes choisi parmi O, S et N.

Par exemple, R6 et R7 peuvent être choisis parmi l'hydrogène, un groupement méthyle, éthyle, propyle, isopropyle, n-butyle, t-butyle ou isobutyle.

Parmi les radicaux X'⁺ préférés, on peut citer les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, et leurs mélanges.
- Y'⁻ est un groupement choisi parmi -COO⁻, -SO₃⁻' -OSO₃⁻, -PO₃²⁻ et -OPO₃²⁻.
- R'3 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P.
   Dans le radical R'3, le ou les hétéroatomes, quand ils sont présents, peuvent être intercalés dans la chaîne dudit radical R'3, ou bien ledit radical R'3 peut être substitué par un ou plusieurs groupes les comprenant tels que hydroxy ou amino (NH₂, NHR' ou NR'R" avec R' et R" identiques ou différents représentant un alkyle linéaire ou ramifié en C1-C18, notamment méthyle ou éthyle). Notamment R'3 peut être :

- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 5 hétéroatomes choisis parmi O, N, S, F, Si et P; ou bien un radical benzylène -C₆H₄-CH₂- éventuellement substitué, par un radical alkyle en C1-C12 comprenant éventuellement 1 à 5 hétéroatomes choisis parmi O, N, S, F, Si et P;
- un radical de formule -CH₂-O-CO-O-, CH₂-CH₂O-CO-O-, -CH₂-CO-O-, -CH₂-CH₂-CO-O-, -[(CH₂)₅CO-O]ₙ-, -CH₂-CH(CH₃)-O-, -(CH₂)₂-O-, -CH₂-O-CO-NH-, - CH₂CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR)-, -CH₂-CH₂-CH(NR'R")-, -CH₂-CH(NR'R")-. -CH₂-CH₂-CH₂-NR'-, - CH₂-CH₂-CH₂O-; -[CH₂-CH₂-O]ₙ- et -[CH₂-CH(CH₃)-O]ₙ-, -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;
- n' est compris entre 1 et 100, de préférence 1 et 5 inclus.

Dans la formule (IId), la signification des radicaux R1, Z', x', R'2 et m' est la même que celle donnée ci-dessus pour la formule (IIa), et celle des radicaux R'3 et n' la même que celle donnée pour la formule (IIc).

Dans la formule (IId), X"⁺ est un groupement de formule -N⁺R₆R₇R₈ avec R6, R7 et R8 représentant, indépendamment l'un de l'autre, soit (i) un atome d'hydrogène, soit (ii) un groupement alkyle linéaire, ramifié ou cyclique, éventuellement aromatique, comprenant de 1 à 18 atomes de carbone, pouvant comprendre 1 à 5 hétéroatomes choisis parmi O, N, S et P; soit (iii) R6 et R7 peuvent former avec l'atome d'azote un premier cycle, saturé ou insaturé, éventuellement aromatique, comprenant au total 5, 6 ou 7 atomes, et notamment 4, 5 ou 6 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 3 hétéroatomes choisi parmi O, S et N.
Par exemple, R6, R7 et R8 peuvent être choisis parmi l'hydrogène, un groupement méthyle, éthyle, propyle, isopropyle, n-butyle, t-butyle, isobutyle, octyle, lauryle ou stéaryle.

Parmi les radicaux X"⁺ préférés, on peut citer les radicaux triméthylammonium; triéthylammonium; N,N-diméthyl,N-octylammonium; N,N-diméthyl,N-laurylammonium.

Parmi les monomères de formule (IIc) ou (IId) préférés, on peut citer la N,N-dimethyl-N-(2-methacryloyloxyethyl)-N-(3-sulfopropyl) ammonium bétaïne (notamment la SPE de la société Raschig); la N,N-dimethyl-N-(3-methacrylamidopropyl)-N-(3-sulfopropyl) ammonium bétaïne (SPP de Raschig), et la 1-(3-sulfopropyl)-2-vinylpyridinium bétaïne (SPV de Raschig), ainsi que la 2-méthacryloyloxyéthylphosphorylcholine.

Lorsque le monomère 'essentiellement cationique' est choisi parmi les mélanges de monomères cationiques et/ou amphotères avec des monomères anioniques, lesdits monomères anioniques sont de préférence présents à raison de 5 à 40% en poids, par rapport au poids du mélange "cationiques et/ou amphotères + anioniques", notamment de 10 à 30% en poids, de préférence de 15 à 25% en poids.

Le monomère 'essentiellement cationique' est présent à raison de 40 à 90% en poids par rapport au poids du polymère final, notamment de 45 à 80% en poids, de préférence de 50 à 70% en poids.

De préférence, le polymère selon l'invention comprend les monomères de formule (I) et les monomères ioniques (les monomères cationiques + les éventuels monomères amphotères et anioniques) en un rapport pondéral pouvant aller de 60/40 à 40/60, avec une préférence pour un rapport 50/50.

Le copolymère éthylénique selon l'invention peut comprendre, de manière optionnelle, d'autres monomères que ceux mentionnés ci-dessus. Ces monomères additionnels sont donc non ioniques.
Lorsqu'il comprend de tels monomères dits additionnels, ceux-ci sont obligatoirement choisis parmi les monomères dits hydrophiles au sens de la présente invention.

Par monomère hydrophile, on entend les monomères ayant une valeur du logarithme du coefficient de partage apparent octanol-1/eau, aussi appelé log P, inférieure ou égale à 2, par exemple comprise entre -8 et 2, de préférence inférieure ou égale à 1,5, notamment inférieure ou égale à 1, et en particulier comprise entre -7 et 1, voire entre -6 et 0.

Les valeurs de log P sont connues et sont déterminées selon un test standard qui détermine la concentration du monomère dans l'octanol-1 et l'eau.
Les valeurs peuvent notamment être calculées à l'aide du logiciel ACD (Advanced Chemistry Development) Software solaris V4.67; elles peuvent également être obtenues à partir de Exploring QSAR : hydrophobic, electronic and steric constants (ACS professional reference book, 1995).
II existe encore un site Internet qui fournit des valeurs estimées (adresse : http://esc.syrres.com/interkow/kowdemo.htm).

Nous indiquons ci-après la valeur du log P de certains monomères usuels, déterminée à l'aide du logiciel ACD :

| | méthacrylate (* ou méthacrylamide) | Acrylate (* ou acrylamide) |
|---|---|---|
| Méthyl (méth)acrylate | 1.346 +/- 0.250 | 0.793 +/- 0.223 |
| Ethyl (méth)acrylate | 1.877 +/- 0.250 | 1.325 +/- 0.223 |
| Propyl (méth)acrylate | 2.408 +/- 0.250 | 1.856 +/- 0.223 |
| Isopropyl (méth)acrylate | 2.224 +/- 0.254 | 1.672 +/- 0.228 |
| Butyl (méth)acrylate | 2.940 +/- 0.250 | 2.387 +/- 0.223 |
| Isobutyl (méth)acrylate | 2.756 +/- 0.254 | 2.208+/-0.228 |
| terbutyl(méth)acrylate | 2.574 +/- 0.261 | 2.022 +/- 0.238 |
| cyclohexyle (méth)acrylate | 3.405 +/-0.252 | 2.853+/- 0.226 |
| octyl(méth)acrylate | 5.065 +/- 0.521 | 4.513 +/- 0.224 |
| lauryl(méth)acrylate | 7.190 +/- 0.251 | 6.638 +/- 0.224 |
| tridecyl(méth)acrylate | 7.712+/-0.251 | 7.170+/- 0.224 |
| cétyl (méth)acrylate | 9.316+/-0.251 | 8.764+/- 0.224 |
| palmityl(méth)acrylate | >9 | >9 |
| stéaryl (méth)acrylate | 10.379+/- 0.251 | 9.826+/-0.224 |
| behenyl (méth)acrylate | 11.952 +/- 0.225 | 12.504 ± 0,251 |
| oléyl (méth)acrylate | >9 | 9.308 ± 0.232 |
| Tetrahydrofurfuryl (méth)acrylate | 1,352 ± 0,283 | 0,800 ± 0,263 |
| 2-ethyl hexyl (méth)acrylate | 4,881 ± 0,254 | 4,329 ± 0,229 |
| 2-hydroxyethyl (méth)acrylate | 0,718 ± 0,277 | 0,166 ± 0,258 |
| éthoxyéthyle (méth)acrylate | 1,887 ± 0,293 | 1,335 ± 0,268 |
| Hydroxypropyl (méth)acrylate | | 0,383 ± 0,241 |
| N-isopropyle (méth)acrylamide * | 0,748 ± 0,276 | 0,195 ± 0,256 |
| N-octyl (méth)acrylamide * | 3,558 ± 0,273 | 3,036 ± 0,253 |
| N,N-diméthyl (méth)acrylamide* | 0,906 ± 0,553 | -0,168 ± 0,556 |
| N,N-dibutyl (méth)acrylamide* | 3,573 ± 0,570 | 3,021 ± 0,557 |
| | | |
| Acétate de vinyle | 0,730 ± 0,286 | |
| méthyl vinyl éther | 0,509 ± 0,286 | |
| éthyl vinyl éther | 1,040 ± 0,286 | |
| vinylcaprolactame | 1,499 ± 0,207 | |
| Vinylpyrrolidone | 0,370 ± 0,206 | |
| N-vinyl acétamide | 0 ± 0,231 | |

Les monomères hydrophiles additionnels peuvent notamment être choisis parmi ceux de formule (III), seuls ou en mélange, : 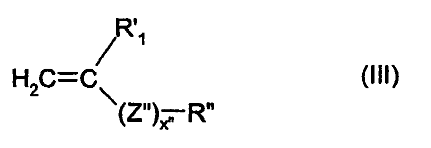 dans lequel :
- R'₁ est l'hydrogène ou -CH₃;
- Z" est un groupement divalent choisi parmi -COO-, -CONH-, -CONCH₃-, -OCO-, - SO₂, -CO-O-CO-, -CO-CH₂-CO- ou -O- ; de préférence COO et CONH;
- x" est 0 ou 1;
- R" est un radical carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
   Dans le radical R", le ou les hétéroatomes, quand ils sont présents, peuvent être intercalés dans la chaîne dudit radical ou bien ledit radical peut être substitué par un ou plusieurs groupes les comprenant tels que hydroxy, ester, amide, uréthane
ou urée.

Notamment R" peut être un radical méthyle, éthyle, propyle, isopropyle, n-butyle, isobutyle, tertiobutyle, phényle, benzyle, ou un radical de formule -CH₂CH₂-CH₂OH, -CH₂-CH₂-OH, -CH₂-CH₂-CH₂OH ou furfuryle.

Les monomères non ioniques hydrophiles additionnels sont notamment choisis parmi les monomères ci-après : le méthacrylate de méthyle, l'acrylate de méthyle, le méthacrylate d'éthyle, l'acrylate d'éthyle, l'acrylate de propyle, l'acrylate d'isopropyle, le méthacrylate de tetrahydrofurturyle, acrylate de tetrahydrofurfuryle, le méthacrylate de 2-hydroxyéthyle, l'acrylate de 2-hydroxyéthyle, le méthacrylate d'éthoxyéthyle, acrylate d'éthoxyéthyle, le N-isopropyl acrylamide, le N-isopropyl méthacrylamide, le N,N-diméthyl acrylamide, le N,N-diméthyl méthacrylamide, l'acétate de vinyle, le méthyl vinyl éther, l'éthyl vinyl éther, la vinylpyrrolidone, la vinylcaprolactame, la N-vinyl acétamide, l'acrylate d'hydroxypropyle, la N-vinyllactame, l'acrylamide, la N-méthyl acrylamide, la N,N-diméthyl acrylamide, la N-méthyl-N-vinylacétamide, le N-vinylformamide, le N-méthyl-N-vinylformamide, l'alcool vinylique (copolymérisé sous forme d'acétate de vinyle puis hydrolysé).

Le monomère additionnel, seul ou en mélange, peut ne pas être présent dans le polymère selon l'invention (0%), ou bien peut être présent en une quantité pouvant aller jusqu'à 50% en poids, par rapport au poids du polymère final; notamment il peut être présent en une quantité de 0,1 à 35% en poids, de préférence de 1 à 25% en poids, par exemple de 3 à 15% en poids, voire de 5 à 9,5% en poids, par rapport au poids total du polymère.
Toutefois, on a constaté que lorsque ce monomère additionnel était choisi parmi acrylate de méthyle, le méthacrylate de méthyle et de l'acrylate d'isopropyle, ces monomères ne pouvaient être présents en une quantité supérieure ou égale à 10% en poids. Ces monomères peuvent donc être présents à raison de 0-9,5% en poids dans le polymère final, notamment de 0,1 à 8% en poids, de préférence de 1 à 5% en poids.

De préférence, le copolymère selon invention ne comprend pas de monomères autres que ceux de formules (I), (IIa), (IIb), (IIc) et (IId). De préférence, le copolymère selon invention comprend uniquement des monomères de formules (I) et (IIa).

De préférence, le polymère utilisable selon l'invention comprend les monomères de formule (I) et les monomères "essentiellement cationiques" en un rapport pondéral pouvant aller de 60/40 à 40/60, avec une préférence pour un rapport 50/50.

Dans un mode de réalisation particulier de l'invention, le polymère est constitué essentiellement de monomère de formule (I), seul ou en mélange, et de monomères de formule (IIa), seul ou en mélange.

Tout particulièrement, on préfère les polymères dans lesquels:
- le monomère de formule (I), seul ou en mélange, est présent à raison de 10 à 60% en poids, par rapport au poids du polymère final, notamment de 20 à 60% en poids, de préférence de 30 à 50% en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 40 à 90% en poids par rapport au poids du polymère final, notamment de 40 à 80% en poids, de préférence de 50 à 70% en poids et est choisi parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle; et
- le polymère étant de préférence neutralisé par un agent neutralisant acide organique notamment choisi parmi l'acide 2-éthylcaproïque, l'acide oléique, l'acide béhénique, l'acide stéarique, l'acide acétique, l'acide citrique, l'acide tartrique, le chlorhydrate de bétaïne et/ou l'acide gluconique, et préférentiellement l'acide béhénique et/ou le chlorhydrate de bétaïne.

Encore plus particulièrement, on préfère les polymères dans lesquels:
- le monomère de formule (I), seul ou en mélange, est présent à raison de 10 à 60% en poids, par rapport au poids du polymère final, notamment de 20 à 60% en poids, de préférence de 30 à 50% en poids, et est choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- le monomère 'essentiellement cationique' est présent à raison de 40 à 90% en poids par rapport au poids du polymère final, notamment de 40 à 80% en poids, de préférence de 50 à 70% en poids et est choisi parmi, seul ou en mélange, le (méth)acrylamide de diméthylaminopropyle, et
- le polymère étant neutralisé par un agent neutralisant choisi parmi l'acide béhénique et/ou le chlorhydrate de bétaïne.

Les polymères selon l'invention peuvent être préparés selon les méthodes usuelles de polymérisation radicalaire classique, bien connues de l'homme du métier, et telles que décrites par exemple dans l'ouvrage "Chimie et physicochimie des polymères" de Gnanou et al. (édition Dunod). Notamment ces polymères peuvent être préparés par :
- polymérisation directe en solution dans l'eau avec pré-neutralisation ou non du motif cationique et/ou du motif anionique;
- polymérisation en émulsion dans l'eau avec pré-neutralisation ou non du motif cationique et/ou du motif anionique, avec utilisation d'un tensioactif;
- polymérisation dans un solvant organique, tel que l'éthanol ou la méthyléthylcétone, avec pré-neutralisation ou non du motif cationique et/ou du motif anionique, suivie d'une étape de mise en solution ou dispersion dans l'eau avec évaporation du solvant.
Ces polymérisations peuvent être effectuées en présence d'amorceur radicalaire notamment de type peroxyde (Trigonox 21 S : tert-Butyl peroxy-2-ethylhexanoate)
ou azoïque (AIBN V50 : 2,2'-azo-bis(2-amidinopropane) dihydrochlorure), qui peut être présent à raison de 0,3 à 5% en poids par rapport au poids total des monomères.

Les polymères selon l'invention sont non réticulés. IIs se présentent sous forme de copolymères éthyléniques statistiques, de préférence filmogènes, d'un ou plusieurs monomères éthyléniques contenant des groupes PEG (les groupes PEG sont pendants le long du squelette) et d'un ou plusieurs monomères éthyléniques comportant des fonctions cationiques (amines neutralisées et non quaternaires) et/ou bétaïnes et, éventuellement d'un ou plusieurs autres comonomères éthyléniques hydrophiles non ioniques, monovalents.

Par polymère "éthylénique" , on entend un polymère obtenu par polymérisation de monomères comprenant une insaturation éthylénique.
Par polymère "filmogène", on entend un polymère apte à former à lui seul ou en présence d'un agent auxiliaire de filmification, un film continu et adhérent sur un support, notamment sur les matières kératiniques.

Ils présentent une masse moléculaire moyenne en poids (Mw) qui est de préférence comprise entre 500 et 5 000 000, notamment comprise entre 1 000 et 3 000 000 et plus préférentiellement comprise entre 2 000 et 2 000 000, voire 4 000 et 500 000, entre autre 7 000 et 250 000, encore mieux 8 000 et 100 000.
On détermine les masses molaires moyennes en poids (Mw) par chromatographie par perméation sur gel ou par diffusion de la lumière, selon l'accessibilité de la méthode (solubilité des polymères considérés).

Les polymères utilisables selon l'invention sont de préférence véhiculables en milieu aqueux, c'est-à-dire qu'ils sont de préférence hydrosolubles ou hydrodispersibles.
Par hydrosoluble, on entend qu'il est soluble dans l'eau, à raison d'au moins 5% en poids, à 25°C, et forme une solution limpide.
Par hydrodispersible, on entend qu'il forme dans l'eau, à une concentration de 5 % en poids, à 25°C, une dispersion stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

La mise en solution ou en dispersion dans l'eau peut être effectuée par solubilisation directe du polymère s'il est soluble, ou bien par neutralisation des motifs amines et/ou des motifs acides de façon à rendre le polymère soluble ou dispersible dans l'eau. La mise en solution ou dispersion aqueuse peut également s'effectuer via une étape intermédiaire de solubilisation dans un solvant organique suivie de l'addition d'eau avant évaporation du solvant organique.

Par ailleurs, on a constaté que les polymères utilisables selon l'invention présentent avantageusement une viscosité dans l'eau adéquate avec les applications envisagées, qui peut être, par exemple comprise entre 1 et 1000 mPa.s, de préférence entre 1,5 et 750 mPa.s, et encore mieux entre 2 et 500 mPa.s.

La viscosité est mesurée à l'aide d'un viscosimètre BROOKFIELD, pour une solution à 15% en poids de polymère dans l'eau ou la méthyléthylcétone (solvant choisi en fonction de la solubilité du polymère et/ou de la méthode de polymérisation), à 25°C, avec un mobile de type aiguille (spindle) choisies parmi les modèles numéros 00 à 07 de Brookfield, de préférence le mobile no 1 ; pour un temps de mesure de 5 minutes, à une vitesse comprise entre 0,1 et 6 tours/minute. La viscosité est mesurée après complète dissolution du polymère dans l'eau ou la méthyléthylcétone.

En outre, les polymères utilisables selon l'invention peuvent présenter de préférence une température de transition vitreuse (Tg) comprise entre -150°C et 20°C, notamment -120°C et 10°C, mieux entre -100°C et 0°C; la Tg est mesurée selon la méthode donnée avant les exemples.

Les polymères utilisables selon l'invention peuvent présenter de préférence une température de fusion (Tf) comprise entre -100°C et 80°C, notamment entre -80°C et 50°C, mieux entre -70°C et 45°C, voire -10°C et 25°C.

En outre, les polymères utilisables selon l'invention présentent de préférence une reprise en eau comprise entre 3% et 150% en poids, de préférence entre 4% et 100% en poids, notamment entre 5% et 50% en poids, à 75% d'humidité relative (75%HR); la reprise en eau est mesurée selon la méthode donnée avant les exemples.

Ils peuvent également présenter une reprise en eau comprise entre 3% et 200% en poids, de préférence entre 2,5% et 150% en poids, notamment entre 3% et 100% en poids, à 85% d'humidité relative (85%HR).

Les polymères peuvent être présents dans la composition sous forme solubilisée, par exemple dans l'eau ou un solvant organique, ou bien sous forme de dispersion aqueuse ou organique.

Ils peuvent être utilisés dans les compositions cosmétiques ou pharmaceutiques à raison de 0,01 à 30% en poids de matière sèche, notamment de 0,1 à 20% en poids, voire de 0,1 à 10% en poids, encore mieux de 0,5 à 3% en poids, par rapport au poids total de la composition.

La composition peut ainsi comprendre, un milieu hydrophile comprenant de l'eau
ou un mélange d'eau et de solvant(s) organique(s) hydrophile(s) comme les alcools et notamment les monoalcools, linéaires ou ramifiés en C1-C6, comme l'éthanol, le tertiobutanol, le n-butanol, l'isopropanol ou le n-propanol, et les polyols comme la glycérine, la diglycérine, le propylène glycol, le sorbitol, le pentylène glycol, et les polyéthylène glycols, ou bien encore les éthers de glycols notamment en C₂ et des cétones en C₂-C₄ hydrophiles.

L'eau ou le mélange d'eau et de solvants organiques hydrophiles peut être présent dans la composition selon l'invention en une teneur allant de 30% à 99% en poids, par rapport au poids total de la composition, et de préférence de 40% à 80% en poids.

La composition peut également comprendre une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.

Comme corps gras liquides à température ambiante, appelés souvent huiles, utilisables dans l'invention, on peut citer : les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore l'huile d'amande douce, l'huile d'olive, l'huile de germes de blé, l'huile d'arachide, l'huile de colza, l'huile de carthame, l'huile de coprah, l'huile de noisette, l'huile de palme, l'huile de noyau d'abricot, l'huile de calophyllum, les huiles de tournesol, de maïs, de soja, de pépins de raisin, de sésame, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol; des alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées,; leurs mélanges.
Ces huiles peuvent être présentes en une teneur allant de 0,01 à 90%, et mieux de 0,1 à 85% en poids, par rapport au poids total de la composition.

Par cire au sens de la présente invention, on entend un composé lipophile, solide à température ambiante (25°C), à changement d'état solide/liquide réversible, ayant un point de fusion supérieur ou égal à 25°C pouvant aller jusqu'à 120°C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.
Les cires peuvent être hydrocarbonées, fluorées et/ou siliconées et être d'origine végétale, minérale, animale et/ou synthétique. En particulier, les cires présentent un point de fusion supérieur à 30°C et mieux supérieur à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone.
Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0,1 à 50% en poids de cires, par rapport au poids total de la composition et mieux de 1 à 30% en poids.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition peut comprendre en outre un polymère conditionneur différent des copolymères à greffons PEG, généralement constitué par un polymère cationique Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863 et ayant une densité de charge cationique convenable.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 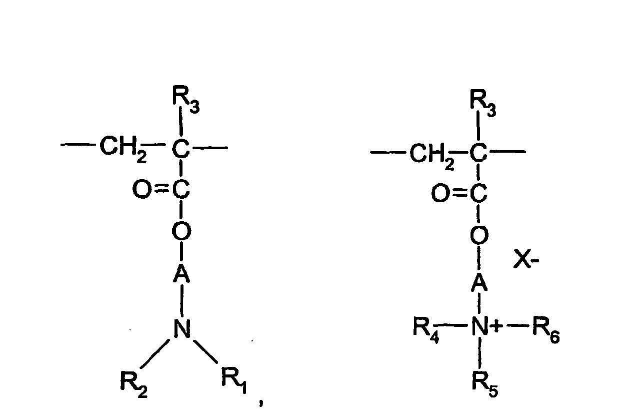 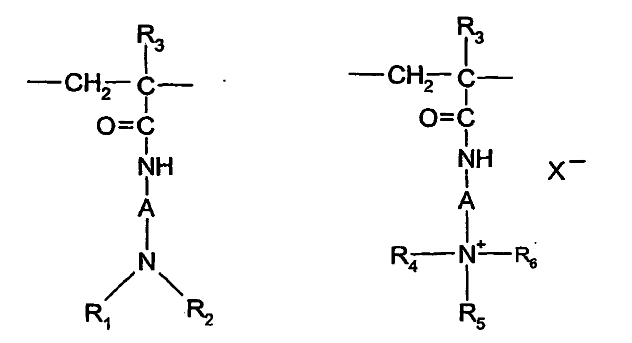 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quatemisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymères d'acrylamide et de méthosulfate de méthacryloyloxyéthyltriméthylammonium vendus sous la dénomination RETEN par la société HERCULES,
   - les copolymères vinylpyrrolidone / acrylate ou méthacrylate de dialkylaminoalkyle quaternisés ou non. Ces polymères sont décrits en détail dans les brevets français 2.077.143 et 2.393.573,
   - les terpolymères méthacrylate de diméthyl amino éthyle/ vinylcaprolactame/ vinylpyrrolidone,
   - les copolymères vinylpyrrolidone / méthacrylamidopropyl dimethylamine.
   - et les copolymères vinylpyrrolidone / méthacrylamide de diméthylaminopropyle quatemisé.
(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques
   ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quatemisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "CARTARETINE F, F4 ou F8" par la société SANDOZ.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "HERCOSETT 57" par la société Hercules Inc. par la société HERCULES dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (I) ou (I') : 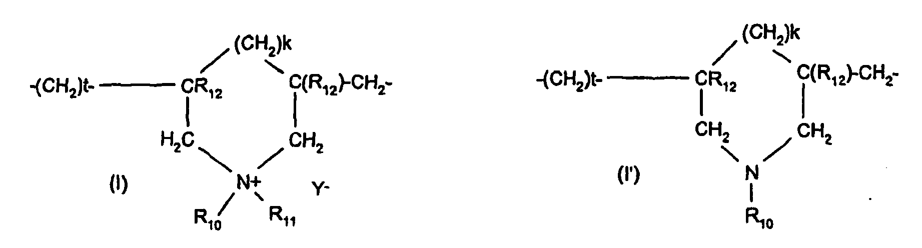 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide.
(8) les polymères de diammonium quaternaire contenant des motifs récurrents répondant à la formule : 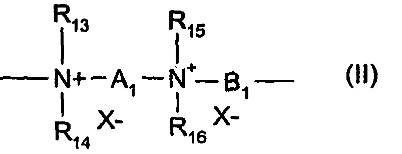 formule (II) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène
   ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;

   De préférence, X⁻ est un anion tel que le chlorure ou le bromure.
   Ces polymères ont une masse molaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule : 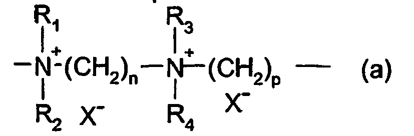 dans laquelle R₁, R₂, R₃ et R₄ identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
   Un composé de formule (a) particulièrement préféré est celui pour lequel R₁, R₂, R₃ et R₄, représentent un radical méthyle et n = 3, p = 6 et X = CI, dénommé Hexadimethrine chloride selon la nomenclature INCI (CTFA).
(9) les polymères de polyammonium quaternaires comprenant des motifs de formule (III): 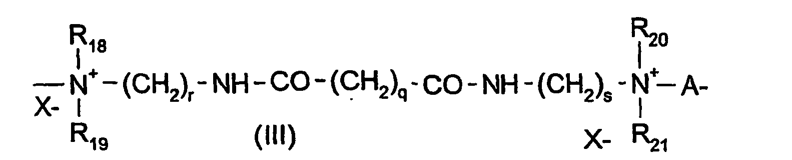 formule dans laquelle :
   R₁₈, R₁₉, R₂₀ et R₂₁, identiques ou différents, représentent un atome d'hydrogène ou un radical méthyle, éthyle, propyle, β-hydroxyéthyle, β-hydroxypropyle
   ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polymères réticulés de sels de méthacryloyloxyalkyl(C1-C4) trialkyl(C1-C4)ammonium tels que les polymères obtenus par homopolymérisation du diméthylaminoéthylméthacrylate quatemisé par le chlorure de méthyle, ou par copolymérisation de l'acrylamide avec le diméthylaminoéthylméthacrylate quatemisé par le chlorure de méthyle, l'homo ou la copolymérisation étant suivie d'une réticulation par un composé à insaturation oléfinique, en particulier le méthylène bis acrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine, notamment les chitosanes ou leurs sels ;
les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90 % en poids, le pyrrolidone-carboxylate de chitosane commercialisé sous la dénomination KYTAMER^{®} PC par la société AMERCHOL.

Parmi tous les polymères cationiques susceptibles d'être utilisés dans le cadre de la présente invention, on préfère mettre en oeuvre les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium, le pyrrolidone-carboxylate de chitosane commercialisé sous la dénomination KYTAMER^{®} PC par la société AMERCHOL et leurs mélanges.

Selon l'invention, le ou les polymères cationiques ou amphotères peuvent représenter de 0,001 % à 20 % en poids, de préférence de 0,01 % à 10% en poids et plus particulièrement de 0,02 à 5% en poids par rapport au poids total de la composition finale.

Selon un mode préféré de l'invention, les compositions peuvent comprendre en outre au moins une silicone.

A titre de silicones utilisables dans les compositions de la présente invention, on peut notamment citer les silicones volatiles ou non, cycliques ou acycliques, ramifiées ou non, organomodifiées ou non, telles que décrites ci-dessous.
Les silicones utilisables conformément à l'invention peuvent être solubles ou insolubles dans la composition et en particulier être des polyorganosiloxanes insolubles dans la composition de l'invention ; elles peuvent se présenter sous forme d'huiles, de cires, de résines ou de gommes.
Selon l'invention, toutes les silicones peuvent être utilisées telle quelle ou sous formede solutions, de dispersions, d'émulsions, de nanoémulsions ou de microémulsions.
Les organopolysiloxanes sont définis plus en détail dans l'ouvrage de Walter NOLL "Chemistry and Technology of Silicones" (1968) Academie Press. Elles peuvent être volatiles ou non volatiles.
Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.

On peut également citer les cyclocopolymères du type diméthylsiloxane/méthylalkylsiloxane, tel que la "SILICONE VOLATILE FZ 3109" commercialisée par la société UNION CARBIDE, de structure chimique :

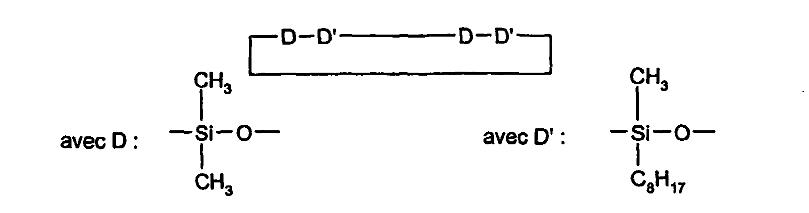

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bisnéopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est, par exemple, mesurée à 25°C selon la norme ASTM 445 Appendice C.

Parmi ces polyalkylsiloxanes, on peut citer à titre non limitatif les produits commerciaux suivants :
- les huiles SILBIONE^{®} des séries 47 et 70 047 ou les huiles MIRASIL^{®} commercialisées par RHODIA telles que, par exemple, l'huile 70 047 V 500 000 ;
- les huiles de la série MIRASIL^{®} commercialisées par la société RHODIA ;
- les huiles de la série 200 de la société DOW CORNING telles que plus particulièrement la DC200 de viscosité 60 000 mm²/s ;
- les huiles VISCASIL^{®} de GENERAL ELECTRIC et certaines huiles des séries SF (SF 96, SF 18) de GENERAL ELECTRIC.

On peut également citer les polydiméthylsiloxanes à groupements terminaux diméthylsilanol, connus sous le nom de dimethiconol (CTFA), tels que les huiles de la série 48 de la société RHODIA .

Dans cette classe de polyalkylsiloxanes, on peut également citer les produits commercialisés sous les dénominations "ABIL WAX^{®} 9800 et 9801" par la société GOLDSCHMIDT qui sont des polyalkyl(C₁-C₂₀)-siloxanes.

Les polyalkylarylsiloxanes sont particulièrement choisis parmi les polydiméthyl/méthylphénylsiloxanes, les polydiméthyldiphényl-siloxanes linéaires et/ou ramifiés de viscosité de 1.10⁻⁵ à 5.10⁻² m²/s à 25°C.

Parmi ces polyalkylarylsiloxanes on peut citer, à titre d'exemple, les produits commercialisés sous les dénominations suivantes :
- les huiles SILBIONE^{®} de la série 70 641 de RHODIA ;
- les huiles des séries RHODORSIL^{®} 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

On peut plus particulièrement citer les produits suivants :
- polydiméthylsiloxane
- les gommes polydiméthylsiloxane/méthylvinylsiloxane,
- polydiméthylsiloxane/diphénylsiloxane,
- polydiméthylsiloxane/phénylméthylsiloxane,
- polydiméthylsiloxane/diphényisiloxane/méthylvinyisiloxane.

Des produits plus particulièrement utilisables conformément à l'invention sont des mélanges tels que :
- les mélanges formés à partir d'un polydiméthylsiloxane hydroxylé en bout de chaîne ou diméthiconol (CTFA) et d'un poly-diméthylsiloxane cyclique également appelé cyclométhicone (CTFA) tel que le produit Q2 1401 commercialisé par la société DOW CORNING ;
- les mélanges formés à partir d'une gomme polydiméthyl-siloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶ m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SiO_{4/2} dans lesquels R représente un groupement hydrocarboné possédant 1 à 16 atomes de carbone ou un groupement phényle. Parmi ces produits, ceux particulièrement préférés sont ceux dans lesquels R désigne un groupe alkyle inférieur en C₁-C₄, plus particulièrement méthyle, ou un groupe phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthylsiloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 .
- des groupements acyloxyalkyle tels que, par exemple, les polyorganosiloxanes décrits dans le brevet US-A-4957732.
- des groupements anioniques du type carboxylique comme, par exemple, dans les produits décrits dans le brevet EP 186 507 de la société CHISSO CORPORATION, ou de type alkyl-carboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

Les silicones telles que décrites ci-dessus peuvent être utilisées seules ou en mélange, en une quantité comprise entre 0,01 et 20 % en poids, de préférence entre 0,1 et 5 % en poids par rapport au poids total de la composition.

La composition selon l'invention peut également comprendre des ingrédients couramment utilisés en cosmétique, tels que les vitamines, les parfums, les agents nacrants, les épaississants, les polymères différents des polymères à groupement PEG les gélifiants, les oligo-éléments, les adoucissants, les séquestrants, les parfums, les agents alcalinisants ou acidifiants, les conservateurs, les filtres solaires, les anti-oxydants, les agents anti-chutes des cheveux, les agents anti-pelliculaires, les anti-radicaux libres, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Avantageusement, le pH de la composition de la présente invention est choisi dans la gamme allant de 2 à 11 et préférentiellement de 3 à 10 par exemple de 5 à 8.

La composition selon l'invention peut comprendre un propulseur. Le propulseur est choisi notamment parmi les gaz comprimés ou liquéfiés usuellement employés pour la préparation de compositions aérosols et leurs mélanges. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Elle trouve notamment une application particulièrement intéressante dans le domaine capillaire, notamment pour le maintien de la coiffure ou la mise en forme des cheveux. Les compositions capillaires sont de préférence des shampooings,
Les compositions peuvent être conditionnées sous diverses formes, notamment dans des flacons, des vaporisateurs, des flacons-pompe ou dans des récipients aérosol afin d'assurer une application de la composition sous forme vaporisée ou sous forme de mousse.

Dans un mode de réalisation préféré, les compositions conformes à l'invention peuvent être utilisées pour le lavage des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants. Dans ce mode de réalisation de l'invention, les compositions comprennent au moins 4% en poids par rapport au poids total de la composition d'au moins un tensioactif détergent anionique et/ou non ionique.

L'invention a donc encore pour objet un procédé de traitement des matières kératiniques telles que la peau ou les cheveux, **caractérisé en ce qu**'il consiste à appliquer sur les matières kératiniques une composition cosmétique telle que définie précédemment, puis à effectuer éventuellement un rinçage à l'eau.
Ainsi, ce procédé selon l'invention permet le maintien de la coiffure, le soin et le lavage ou le démaquillage de la peau, des cheveux ou de toute autre matière kératinique.

Dans un autre mode de réalisation préféré, les compositions de l'invention peuvent se présenter sous forme d'après-shampooing à rincer ou non, ou encore sous forme de compositions à rincer, à appliquer avant ou après tout traitement capillaire, notamment une coloration, une décoloration, une permanente ou un défrisage ou encore entre les deux étapes d'une permanente ou d'un défrisage.
Lorsque la composition se présente sous la forme d'un après-shampooing éventuellement à rincer, elle contient avantageusement au moins un tensioactif cationique, par exemple en une concentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.
Les compositions selon l'invention peuvent également se présenter sous forme de lotions aqueuses ou hydroalcooliques pour le soin de la peau et/ou des cheveux.

La composition suivant l'invention, après application sur les cheveux et cuir chevelu humains peut être rincée ou non rincée après tout traitement. Elle peut se présenter sous toute forme classiquement utilisée dans le domaine concerné et par exemple sous forme de lotion plus ou moins épaissie, de gel, de crème, de spray ou de mousse. Cette composition peut être monophasique ou multiphasique.

Selon un mode de réalisation préféré de l'invention, la composition peut être utilisée comme shampoing.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Les compositions de l'invention sont illustrés plus en détail dans les exemples suivants.

### Mesure de la Tg

Un film est réalisé à partir d'une solution aqueuse à 6% en poids de polymère et séché 48 heures dans une atmosphère contrôlée à 50% d'humidité relative et 25°C. Les films ainsi obtenus ont une épaisseur comprise entre 10 et 20 µm.
L'appareil de mesure est une DSC (TA instruments).
L'échantillon issu du film est déposé dans un creuset hermétique et est chauffé selon le protocole suivant :
- équilibre à température initiale Ti;
- chauffe 1 : augmentation de la température, à une vitesse de +10°C/min jusqu'à température finale :Tf (°C);
- isotherme durant 1 minute;
- diminution de la température à une vitesse de -10°C/min jusqu'à Ti (°C);
- chauffe2 : augmentation de la température à une vitesse de +10°C/min jusqu'à Tf (°C);
- isotherme durant 1 minute.
   avec Ti : Température initiale -120°C
   avec Tf : Température finale + 120°C
   Les Tg sont mesurées lors des étapes de chauffe 1 et 2.

### Mesure de la reprise en eau

On dépose environ 1 g de polymère sec dans une coupelle en aluminium de 4,5 cm de diamètre (0,01 m²). On laisse sécher 48 heures à l'étuve à 60°C, sous pression réduite. On retire les coupelles et on les pèse immédiatement (moins d'une minute après leur sortie de l'étuve). On obtient P1.
Les coupelles sont ensuite placées dans une boite à gant ayant l'humidité relative considérée (75% HR ou 85% HR) et y sont laissées 6 heures. Elles sont ensuite pesées à nouveau immédiatement après leur sortie de la boite à gant. On obtient P2.
La reprise en eau est calculée de la manière suivante : [(P2-P1) x 100] / P1

### Exemple de préparation 1

Dans un réacteur (quadricol) surmonté de deux ampoules d'addition, d'un réfrigérant et muni d'une agitation mécanique, on introduit 75 ml de méthyléthylcétone (MEC), on porte à 80°C.
Parallèlement, on prépare une solution 1 comprenant les monomères : 50 g de méthacrylate de polyéthylèneglycol (MPEG 550), 50 g de diméthylaminopropylméthacrylamide (DMAPMA) et l'amorceur : 0,5 g de (Trigonox 21S).
On prépare également une solution 2 comprenant 75 ml de méthyléthylcétone et 0,5 g d'amorceur (Trigonox 21 S).
La solution 1 est coulée goutte à goutte en 1 heure et la solution 2 en deux heures, dans le réacteur quadricol. Le mélange résultant est maintenu à 80°C ensuite pendant 5 heures. La solution jaune-orangée obtenue est refroidie. On obtient 95 g de polymère. Le polymère présente une viscosité Brookfield à 15% dans la MEC, à 25°C, mesurée avec un mobile du type aiguille (spindle) numéro 1, à une vitesse de 0,1 tour/minute, de : 7,5 mPa.s.

On peut ensuite procéder à la neutralisation du polymère de la manière suivante :
on ajoute 290 ml d'HCl 1 N sous agitation au 95 g de polymère, et 200 ml d'eau distillée. Puis on procède à l'évaporation du solvant (MEC).
   Le polymère neutralisé est soluble dans l'eau (au moins jusqu'à 50% en poids).
   Sa Tg est de -60°C.
   Le polymère neutralisé présente une reprise en eau à 85% HR de 51 %.

### Exemple de préparation 2

Dans un réacteur (quadricol) surmonté de deux ampoules d'addition, d'un réfrigérant et muni d'une agitation mécanique, on introduit 100 ml d'eau que l'on porte à 80°C.
Parallèlement, on prépare une solution 1 comprenant 50 g de monomère MPEG 550, 1 g d'amorceur (persulfate de potassium KPS) et 50 ml d'eau.
On prépare également une solution 2 comprenant 50 g de monomère DMAPMA neutralisé à 100% par le chlorhydrate de bétaïne et 50 g d'eau.
Les solutions 1 et 2 sont coulées en 1 heure dans le quadricol. Après une heure à 80°C, on y coule goutte à goutte en 15 minutes, un mélange de 1 g de KPS dans 50 ml d'eau. Le mélange résultant est ensuite maintenu à 80°C pendant 3 heures. On obtient 90 g de polymère neutralisé par le chlorhydrate de bétaïne.

Le polymère présente une viscosité Brookfield à 15% dans l'eau, à 25°C, mesurée avec un mobile du type aiguille (spindle) numéro 1, à une vitesse de 6 tours/minute, de : 164 mPa.s.
Le polymère est soluble dans l'eau (au moins jusqu'à 50% en poids).
Sa Tg est de -60°C.
Le polymère neutralisé présente une reprise en eau à 85% HR de 90%.

### Exemples de préparation 3 à 17

On prépare, selon le procédé de l'exemple 1 (procédé solvant) ou de l'exemple 2 (procédé dans l'eau), les polymères suivants, qui sont selon l'invention ou comparatifs :

| Exemple | Monomères | | Procédé et neutralisation | solubilité |
|---|---|---|---|---|
| Exemple 3 | MPEG 550 | 10% | Procédé 1 | eau |
| | DMAPMA | 90% | HCl | |
| Exemple 4 | MPEG 1100 | 25% | Procédé 1 | eau |
| | DMAPMA | 75% | HCl | |
| Exemple 5 | MPEG 1100 | 50% | Procédé 1 | Eau |
| | DMAPMA | 50% | HCl | |
| Exemple 6 | MPEG 550 | 50% | Procédé 1 | Eau |
| | DMAPMA | 50% | HCl | |
| Exemple 7 | MPEG 550 | 50% | Procédé 2 | Eau |
| | SPE | 50% | Pas de neutr. | |
| Exemple 8 | MPEG 550 | 50% | Procédé 1 | Eau |
| | MADAME | 50% | HCl | |
| Exemple 9 | MPEG 550 | 50% | Procédé 1 | Eau |
| | Méthacrylate de morpholinoéthyle | 50% | HCl | |
| Exemple 10 | MPEG 2000 | 50% | Procédé 2 | Eau |
| | DMAPMA | 50% | Pas de neutr. | |
| Exemple 11 | MPEG 550 | 50% | Procédé 1 | Eau |
| | DMAPMA | 50% | Chlorhydrate de bétaïne | |
| Exemple 12 | MPEG 550 | 40% | Procédé 1 | Eau |
| | DMAPMA | 50% | HCl | |
| | MAEE | 10% | | |
| Exemple 13 | MPEG 550 | 40% | Procédé 2 | Eau - |
| | DMAPMA | 50% | HCl | |
| | Acrylate d'hydroxyéthyle | 10% | | |
| Exemple 14 | MPEG 550 | 40% | Procédé 1 | Eau |
| | DMAPMA | 50% | HCl | |
| | vinylpyrrolidone | 10% | | |
| Exemple 15 | MPEG 550 | 40% | Procédé 1 | Eau |
| | DMAPMA | 35% | HCl | |
| | Acide acrylique | 15% | | |
| Exemple 16 | MPEG 550 | 40% | Procédé 1 | Eau |
| | Méthacrylate de tetrahydrofurfuryle | 10% | HCl | |
| | DMAPMA | 50% | | |
| | | | | |
| Exemple 17 | MPEG 550 | 40 % | Procédé 1 | eau |
| | Vinylcaprolactame | 10% | HCl | |
| | DMAPMA | 50% | | |
| Exemple 18 | MPEG 550 | 50% | Procédé 1 | Dispersible |
| | DMAPMA | 50% | Acide béhénique à 20% | dans l'eau |
| Exemple 19 | MPEG 550 | 50% | Procédé 1 Acide oléïque | Dispersible dans l'eau |
| | DMAPMA | 50% | | |
| Exemple 20 | MPEG 8000 | 50% | Procédé 1 | Eau |
| | DMAPMA | 50% | Post neutralisation Chlorhydrate de bétaïne | |
| Exemple 21 | MPEG 5000 | 25% | Procédé 1 | Eau |
| | DMAPMA | 75% | Pré neutralisation Chlorhydrate de bétaïne | |
| Exemple 22 | MPEG 4000 | 50% | Procédé 1 | Eau |
| | DMAPMA | 50% | Chlorhydrate de bétaïne | |
| Exemple 23 | MPEG 8000 | 20% | Procédé 1 | Eau |
| | DMAPMA | 80% | Chlorhydrate de bétaïne | |

| | | | | |
|---|---|---|---|---|
| MPEG : méthacrylate de polyéthylèneglycol (avec PM = 550, 1100 ou 2000) DMAPMA : méthacrylamide de diméthylaminopropyle SPE : N,N-dimethyl-N-(2-methacryloyloxyethyl)-N-(3-sulfopropyl) ammonium bétaïne MADAME : méthacrylate de diméthylaminoéthyle MAEE : méthacrylate d'éthoxyéthyle | | | | |

### Exemples de polymères comparatifs

on prépare selon l'exemple 1, les polymères hors invention suivants :

| | | | |
|---|---|---|---|
| Comparatif 1 | MPEG 550 | 35% | Procédé 1 |
| (monomère additionnel hydrophobe) | DMAPMA | 50% | HCl |
| | acrylate d'éthylhexyle | 15% | |
| Comparatif 2 | MPEG 550 | 50% | Procédé 1 |
| (polymère réticulé) | DMAPMA | 50% | HCl |
| | Diméthacrylate de butanediol | 1% | |
| Comparatif 3 | MPEG 550 | 50% | |
| (polymère quatemisé) | TMEACL* | 50% | |

| | | | |
|---|---|---|---|
| *TMEACL : acrylate de 2-(diméthylamino)éthyle, quaternisé par du chlorure de méthyle | | | |

### Exemples de compositions

### Exemples de shampooings selon l'invention

L'invention peut être exemplifiée par les compositions non exhaustives suivantes. Les pourcentages sont exprimés en pourcentage en poids de matière active.

| | ***Ex 20*** | ***Ex 21*** | ***Ex 22*** | ***Ex 23*** |
|---|---|---|---|---|
| SODIUM LAURETH SULFATE [1] | 8%ma | 8%ma | 8%ma | 8%ma |
| COCO-GLUCOSIDE [2] | 5%ma | 5%ma | 5%ma | 5%ma |
| COCO-BETAINE [3] | 2%ma | 2%ma | 2%ma | 2%ma |
| POLYQUATERNIUM 10 [6] | 0.5%ma | - | - | 0.5%ma |
| DMAPMA/MPEG 550 (50/50)[7] | 0.5%ma | 0.5%ma | 0.5%ma | 0.5%ma |
| GUAR HYDROXYPROPYLTRIMONIU M CHLORIDE [8] | - | 0.1%ma | - | - |
| DIMETHICONE [9] | - | 2%ma | - | - |
| DIMETHICONE [10] | - | - | 2%ma | 1.0%ma |
| COCAMIDE MIPA [13] | - | - | 1.5%ma | 1.5%ma |
| CONSERVATEUR | Qs | Qs | Qs | Qs |
| PARFUM | Qs | Qs | Qs | Qs |
| SOUDE/ ACIDE CITRIQUE | Qs pH 6 | QspH7 | Qs pH 7 | Qs pH 7 |
| EAU | QsP 100% | QsP 100% | QsP 100% | QsP 100% |

| | ***Ex 24*** | ***Ex 25*** | ***Ex 26*** |
|---|---|---|---|
| SODIUM LAURETH SULFATE [1] | 8%ma | 8%ma | 8%ma |
| COCO-GLUCOSIDE [2] | 5%ma | 5%ma | 5%ma |
| COCO-BETAINE [3] | 2%ma | 2%ma | 2%ma |
| POLYQUATERNIUM 10 [6] | - | - | - |
| DMAPMA/MPEG 550 (50/50)[7] | 0.5%ma | 0.5%ma | 0.5%ma |
| GUAR HYDROXYPROPYLTRIMONI UM CHLORIDE [8] | 0.2%ma | 0.2%ma | - |
| DIMETHICONE [11] | 2%ma | - | - |
| AMODIMETHICONE [12] | - | 2%ma | 3%ma |
| COCAMIDE MIPA [13] | 1.5%ma | 1.5%ma | 1.5%ma |
| CONSERVATEUR | qs | qs | qs |
| PARFUM | qs | qs | qs |
| SOUDE/ ACIDE CITRIQUE | qs pH 7 | qs pH 7 | qs pH 7 |
| EAU | qsp 100 % | qsp 100% | qsp 100% |

| | | | |
|---|---|---|---|
| *[1] Texapn N 702 (Cognis)* *[2] Plantacare 818 UP (Cognis)* *[3] Dehyton AB* 30 *(Cognis)* *[6] Ucar polymer JR400 L T (Amerchol)* *[7] Polymère de l'exemple 11* *[8] Jaguar C13S (Rhodia)* *[9] Belsil DM 300 000 (Wacker)* *[10] Mirasil DM 500 000 (Rhodia)* *[11] Dow Corning 200 fluid 60 000 (Dow corning)* *[12] Dow Corning* 939 *emulsion (Dow corning)* *[13] Empilan CIS (Huntsman)* | | | |

| *Constituants* | ***Ex 27*** | ***Ex 28*** | ***Ex 29*** | ***Ex 30*** |
|---|---|---|---|---|
| SODIUM LAURETH SULFATE [1] | 10%ma | 10%ma | 10%ma | 10%ma |
| LAURETH-12 [16] | 4%ma | 4%ma | 4%ma | 4%ma |
| POLYQUATERNIUM 10 [6] | 0.5%ma | - | - | 0.5%ma |
| DMAPMA/MPEG 550 (50/50)[7] | 0.5%ma | 0.5%ma | 0.5%ma | 0.5%ma |
| GUAR HYDROXYPROPYLTRIMONIUM CHLORIDE [8] | - | 0.1%ma | - | - |
| DIMETHICONE [9] | - | 2%ma | - | - |
| DIMETHICONE [10] | - | - | 2%ma | 1%ma |
| COCAMIDE MIPA [13] | - | - | 1.5%ma | 1.5%ma |
| CONSERVATEUR | qs | qs | qs | qs |
| PARFUM | qs | qs | qs | qs |
| SOUDE/ ACIDE CITRIQUE | qs pH 6 | qs pH7 | qs pH 7 | qs pH 7 |
| EAU | qsP 100% | qsP 100% | qsP 100% | qsP 100% |

| ***Constituants*** | ***Ex 31*** | ***Ex 32*** | ***Ex 33*** |
|---|---|---|---|
| SODIUM LAURETH SULFATE [1] | 10%ma | 10%ma | 10%ma |
| LAURETH-12 [16] | 4%ma | 4%ma | 4%ma |
| DMAPMAIMPEG 550 (50/50)[7] | 0.5%ma | 0.6%ma | 0.5%ma |
| GUAR HYDROXYPROPYLTRIMONIUM CHLORIDE [8] | 0.2%ma | 0.2%ma | - |
| DIMETHICONE [11] | 2%ma | - | - |
| AMODIMETHICONE [12] | - | 2%ma | 3%ma |
| COCAMIDE MIPA [13] | 1.5%ma | 1.5%ma | 1.5%ma |
| CONSERVATEUR | Qs | Qs | Qs |
| PARFUM | Qs | Qs | Qs |
| SOUDE/ ACIDE CITRIQUE | QspH7 | Qs pH 7 | Qs pH 7 |
| EAU | QsP 100% | QsP 100% | QsP 100% |

| ***Constituants*** | ***Ex 34*** | ***Ex 35*** | ***Ex 36*** | ***Ex 37*** |
|---|---|---|---|---|
| LAURETH-5 CARBOXYLIC ACID [14] | 6%ma | 6%ma | 6%ma | 6%ma |
| COCO-GLUCOSIDE [2] | 11 %ma | 11%ma | 11 %ma | 11%ma |
| POLYQUATERNIUM 10 [6] | 0.5%ma | - | - | 0.5%ma |
| DMAPMAIMPEG 550 (50/50)[7] | 0.5%ma | 0.5%ma | 0.5%ma | 0.5%ma |
| GUAR HYDROXYPROPYLTRIMONIUM CHLORIDE [8] | - | 0.1%ma | - | - |
| DIMETHICONE [9] | - | 2%ma | - | - |
| DIMETHICONE [10] | - | - | 2%ma | 1%ma |
| COCAMIDE MIPA [13] | - | - | 1.5%ma | **1**.5%ma |
| CONSERVATEUR | Qs | Qs | Qs | Qs |
| PARFUM | Qs | Qs | Qs | Qs |
| SOUDE/ ACIDE CITRIQUE | Qs pH 6 | Qs pH 7 | QspH7 | Os pH 7 |
| EAU | QsP 100% | QsP 100% | QsP 100% | QsP 100% |

| | ***Ex 38*** | ***Ex 39*** | ***Ex 40*** |
|---|---|---|---|
| LAURETH-5 CARBOXYLIC ACID [14] | 6%ma | 6%ma | 6%ma |
| COCO-GLUCOSIDE [2] | 11%ma | 11%ma | 11 %ma |
| DMAPMA/MPEG 550 (50/50)[7] | 0.5%ma | 0.5%ma | 0.5%ma |
| GUAR HYDROXYPROPYLTRIMONIUM CHLORIDE [8] | 0.2%ma | 0.2%ma | - |
| DIMETHICONE [11] | 2%ma | - | - |
| AMODIMETHICONE [12] | - | 2%ma | 3%ma |
| COCAMIDE MIPA [13] | 1.5%ma | 1.5%ma | 1.5%ma |
| CONSERVATEUR | Qs | Qs | Qs |
| PARFUM | Qs | Qs | Qs |
| SOUDE/ ACIDE CITRIQUE | Qs pH 7 | QspH7 | Qs pH 7 |
| EAU | QsP 100% | QsP 100% | QsP 100% |

| Constituants | Ex 41 | Ex 42 | Ex 43 |
|---|---|---|---|
| AMMONIUM LAURYL SULFATE [15] | 15%ma | 15%ma | 15%ma |
| COCO-GLUCOSIDE [2] | 5%ma | 5%ma | 5%ma |
| POLYQUATERNIUM 10 [6] | - | - | - |
| DMAPMA/MPEG 550 (50/50)[7] | 0.5%ma | 0.5%ma | 0.5%ma |
| GUAR HYDROXYPROPYLTRIMONIUM CHLORIDE [8] | 0.2%ma | 0.2%ma | - |
| DIMETHICONE [11] | 2%ma | - | - |
| AMODIMETHICONE [12] | - | 2%ma | 3%ma |
| COCAMIDE MIPA [13] | 1.5%ma | 1.5%ma | 1.5%ma |
| CONSERVATEUR | Qs | Qs | Qs |
| PARFUM | Qs | Qs | Qs |
| SOUDE/ ACIDE CITRIQUE | Qs pH 7 | Qs pH 7 | Qs pH 7 |
| EAU | QsP 100% | QsP 100% | QsP 100% |

| | | | |
|---|---|---|---|
| *[1] Texapon N 702 (Cognis)* *[2] Plantacare 818 UP (Cognis)* *[3] Dehyton AB* 30 *(Cognis)* *[4] Tego bétaine F50 (Goldshmidt)* *[5] Miranol C 2M- conc. NP (Rhodia)* *[6] Ucar polymer JR400 L T (Amerchol)* *[7] Polymère ex preparation 11* *[8] Jaguar C13S (Rhodia)* *[9] Belsil DM 300 000 (Wacker)* *[10] Mirasil DM 500 000 (Rhodia)* *[11] Dow Corning 200 fluid 60 000 (Dow corning)* *[12] Dow Corning 939 emulsion (Dow corning)* *[13] Empilan CIS (Huntsman)* *[14] AKYPO RLM 45 CA (Kao)* *[15] EMPICOL AL 30*/*FL (Huntsman)* *[16] REINOPAL 12 (Goldshmidt)* | | | |

On peut remplacer le polymère DMAPMA/MPEG 550 (50/50)[7] par les polymères des exemples de préparation 1 à 23.

### Exemple 44

On a préparé une composition comprenant les constituants suivants (% en poids):
- 7,5% de lauryl éther sulfate de sodium
- 2,5% de tensioactif amphotère coco-bétaïne (Dehyton AB30 de Cognis)
- 5% de tensioactif coco-polyglucoside (Plantacare 818 UP de Cognis)
- 1,5% de polymère de l'exemple 11
- qsp 100% eau

La composition de shampooing obtenue apporte un bon effet coiffant et les propriétés cosmétiques sur cheveux secs particulièrement bonnes.

### Exemple 45

On a préparé une composition comprenant les constituants suivants (% en poids):
- 7,5% de lauryl éther sulfate de sodium
- 2,5% de tensioactif amphotère coco-bétaïne
- 5% de tensioactif coco-polyglucoside
- 1,5% de polymère neutralisé de l'exemple 18
- qsp 100% eau

Les résultats sont rassemblés dans le tableau ci-après (test effectué pour la composition de l'exemple 45, comprenant le polymère de l'exemple 18, et pour la composition de l'exemple 44, comprenant le polymère de l'exemple 11)

| | Démêlage cheveu humide | Lissage cheveu humide | Démêlage cheveu sec | brillance | ressort |
|---|---|---|---|---|---|
| Composition selon l'exemple 44 | +++ | +++ | +++ | +++ | ++ |
| Composition selon l'exem-ple 45 | ++++ | ++++ | | ++++ | |
| Témoin (shampooing DOP camomille) | ++ | ++ | ++ | ++ | 0 |

## Revendications

1. Composition cosmétique **caractérisée par le fait qu'**elle comprend dans un milieu aqueux cosmétiquement acceptable:
I) au moins un tensioactif anionique et au moins un tensioactif non ionique,
II) au mois un copolymère éthylénique comprenant, en % en poids par rapport au poids total du polymère, :
- a) 10-60% en poids d'un ou plusieurs monomères de formule (I) : 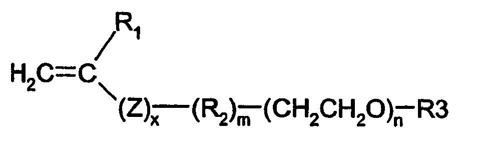 dans laquelle :
- R1 est un atome d'hydrogène ou un radical hydrocarboné, linéaire ou ramifié, de type CₚH₂ₚ₊₁, avec p étant un entier compris entre 1 et 12 inclus;
- Z est un groupement divalent choisi parmi -COO-, -CONH-, -CONCH₃-, -OCO- , - O-, -SO₂- -CO-O-CO- ou -CO-CH₂-CO-.,
- x est 0 ou 1;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P; et leurs sels.
- b) 40-90% en poids d'au moins un monomère "essentiellement cationique", ou l'un de ses sels, choisi parmi :
- (i) un ou plusieurs monomères cationiques de formule (IIa),
- (ii) un ou plusieurs monomères amphotères de formules (IIc) et (IId), et
- (iii) un mélange d'un ou plusieurs monomères cationiques de formule (IIa) avec un ou plusieurs monomères anioniques choisis parmi l'anhydride maléique et/ou ceux de formule (IIb); et/ou avec un ou plusieurs monomères amphotères choisis parmi ceux de formules (IIc) et (IId). 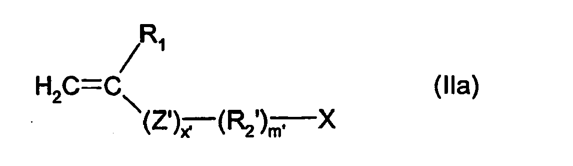 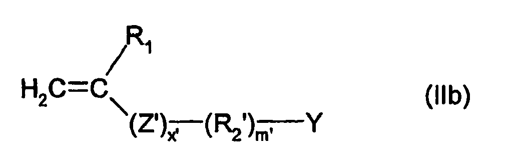 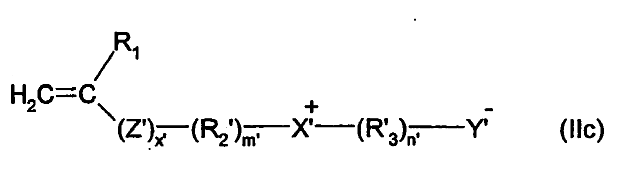 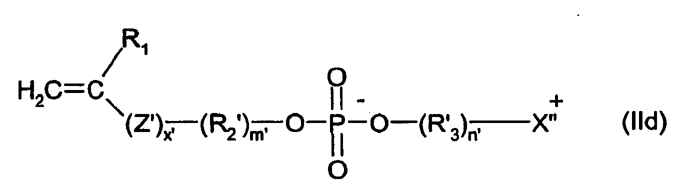
dans lesquelles :
- R1 est un atome d'hydrogène ou un radical hydrocarboné, linéaire ou ramifié, de type CₚH₂ₚ₊₁, avec p étant un entier compris entre 1 et 12, inclus; de préférence l'hydrogène ou un radical méthyle, éthyle, propyle, butyle.
- Z' est un groupement divalent choisi parmi -COO-, -CONH-, -CONCH₃-, -OCO-
ou -O-, -SO₂- -CO-O-CO- ou -CO-CH₂-CO-; de préférence, COO et CONH.
- x' est 0 ou 1, de préférence 1.
- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m' est 0 ou 1;
- X (dans la formule IIa) est (a) un groupe de formule -N(R₆)(R₇) ou -P(R₆)(R₇) ou
- P⁺R₆R₇R₈, avec R6, R7 et R8 représentant, indépendamment l'un de l'autre, soit
(i) un atome d'hydrogène, soit (ii) un groupement alkyle linéaire, ramifié ou cyclique, saturé ou insaturé, éventuellement aromatique, comprenant de 1 à 18 atomes de carbone, pouvant comprendre 1 à 10 hétéroatomes choisis parmi O, N, S, F, SI et P; soit (iii) R6 et R7 peuvent former avec l'atome d'azote ou de phosphore un premier cycle saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 4 hétéroatomes choisi parmi O, S et N ;
ou (b) X représente un groupement -R'6-N-R'7- dans lequel R'6 et R'7 forment avec l'atome d'azote un cycle, saturé ou insaturé, éventuellement aromatique, comprenant au total 5, 6, 7 ou 8 atomes, et notamment 4, 5 ou 6 atomes de carbone et/ou 2 à 4 hétéroatomes choisi parmi O, S et N; ledit cycle pouvant être fusionné avec un ou plusieurs autres cycles, saturés ou insaturés, éventuellement aromatiques, comprenant chacun 5, 6 ou 7 atomes, et notamment 4, 5, 6, 7 ou 8 atomes de carbone et/ou 2 à 4 hétéroatomes choisi parmi O, S et N.
- Y est un groupement choisi parmi -COOH, -SO₃H, -OSO₃H, -PO₃H₂ et -OPO₃H₂.
- X'⁺ est un groupe divalent de formule -N⁺(R₆)(R₇)- avec R6 et R7 représentant, indépendamment l'un de l'autre, soit (i) un atome d'hydrogène, soit (ii) un groupement alkyle linéaire, ramifié ou cyclique, éventuellement aromatique, comprenant de 1 à 25 atomes de carbone, pouvant comprendre 1 à 20 hétéroatomes choisis parmi O, N, S et P; soit (iii) R6 et R7 peuvent former avec l'atome d'azote un premier cycle, saturé ou insaturé, éventuellement aromatique, comprenant au total 5, 6, 7 ou 8 atomes, et notamment 4, 5, 6 ou 7 atomes de carbone et/ou 2 à 3 hétéroatomes choisi parmi O, S et N; ledit premier cycle pouvant être fusionné avec un
ou plusieurs autres cycles, saturés ou insaturés, éventuellement aromatiques, comprenant chacun 5, 6, 7 ou 8 atomes, et notamment 4, 5, 6 ou 7 atomes de carbone et/ou 2 à 3 hétéroatomes choisi parmi O, S et N.
- Y'⁻ est un groupement choisi parmi -COO⁻, -SO₃⁻, -OSO₃⁻, -PO₃²⁻ et -OPO₃²⁻.
- R'3 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P.
- n' est compris entre 1 et 100, de préférence 1 et 5 inclus;
- X"⁺ est un groupement de formule -N⁺R₆R₇R₈ avec R6, R7 et R8 représentant, indépendamment l'un de l'autre, soit (i) un atome d'hydrogène, soit (ii) un groupement alkyle linéaire, ramifié ou cyclique, éventuellement aromatique, comprenant de 1 à 18 atomes de carbone, pouvant comprendre 1 à 5 hétéroatomes choisis parmi O, N, S et P; soit (iii) R6 et R7 peuvent former avec l'atome d'azote un premier cycle, saturé ou insaturé, éventuellement aromatique, comprenant au total 5, 6 ou 7 atomes, et notamment 4, 5 ou 6 atomes de carbone et/ou 2 à 3 hétéroatomes choisi parmi O, S et N; ledit premier cycle pouvant être fusionné avec un ou plusieurs autres cycles, saturés ou insaturés, éventuellement aromatiques, comprenant chacun 5, 6 ou 7 atomes, et notamment 4, 5, 6 ou 7 atomes de carbone et/ou 2 à 3 hétéroatomes choisi parmi O, S et N.
- c) et éventuellement 0-50% en poids de monomères hydrophiles non ioniques, à l'exclusion de l'acrylate de méthyle, du méthacrylate de méthyle et de l'acrylate d'isopropyle s'ils sont présents en une quantité supérieure ou égale à 10% en poids.

2. Composition selon la revendication 1, **caractérisée en ce que**, dans la formule (I), R1 représente l'hydrogène ou un radical méthyle, éthyle, propyle, butyle.

3. Composition selon l'une des revendications précédentes, **caractérisée en ce que**, dans la formule (I), Z représente COO ou CONH.

4. Composition selon l'une des revendications précédentes, **caractérisée en ce que**, dans la formule (I), le radical R2 est choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 25 hétéroatomes choisis parmi O, N, S, F, Si et P; ou bien un radical benzylène -C₆H₄-CH₂- éventuellement substitué, par un radical alkyle en C1-C12 comprenant éventuellement 1 à 8 hétéroatomes choisis parmi O, N, S, F, Si et P;
- un radical pyridinium de formule: 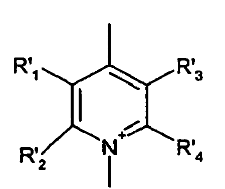 avec R'1 à R'4, identiques ou différents, choisis parmi H et un radical alkyle en C1-C12 comprenant éventuellement 1 à 8 hétéroatomes choisis parmi O, N, S, F, Si et P; notamment R'1 à R'4 peuvent être méthyle et/ou éthyle;
- un radical de formule -CH₂-CHOH-, -CH₂-CH₂-CHOH-, -CH₂-CH₂-CH(NH₂)-, - CH₂-CH(NH₂)-, -CH₂-CH₂-CH(NHR')-, -CH₂-CH(NHR')-, -CH₂-CH₂-CH(NR'R")-, - CH₂-CH(NR'R")-, -CH₂-CH₂-CH₂-NR'-, -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH- ou -CH₂-CH₂-NH-CO-NH- ; -CH₂-CH₂-CH₂-O-; -CH₂-CH₂-CHR'-O- avec R' et R" représentant un alkyle linéaire ou ramifié en C1-C22 comprenant éventuellement 1 à 12 hétéroatomes choisis parmi O N, S, F, Si et P;
- ou un mélange de ces radicaux.

5. Composition selon l'une des revendications précédentes, **caractérisée en ce que**, dans la formule (I), n est compris entre 5 et 200 inclus, et encore mieux entre 7 et 100 inclus, voire entre 9 et 50 inclus.

6. Composition selon l'une des revendications précédentes, **caractérisée en ce que,** dans la formule (I), R3 est un atome d'hydrogène; un radical succinimido, maléimido, mésityle, tosyle, triéthoxysilane, phtalimide ou -CH₂-CH₂CN; ou encore un radical benzyle ou phényle éventuellement substitué par un radical alkyle en C1-C12 comprenant éventuellement 1 à 8 hétéroatomes choisis parmi O, N, S, F, Si et P; un radical alkyle en C1-C30, notamment C1-C22, voire C2-C16, comprenant éventuellement 1 à 18 hétéroatomes choisis parmi O, N, S, F, Si et P ; lesdits radicaux benzyle, phényle ou alkyle pouvant comprendre en outre une fonction choisie parmi les fonctions suivantes: Succinimido ; Glutarate-succinimido ; Glutarate ; maléimido ; Mésityle ; Benzoate ; Tosyle ; Triéthoxysilane ; Phtalimide ; Thioester ; Benzotriazole carbonate ; Butyraldéhyde ; Acétaldéhyde diéthylacétal ; Biotine ; Phospholipide ; Succinate ; N-hydroxysuccinimide ; - SO₃H, -COOH, -PO₄, -NR5R6 ou -N⁺ R5R6R7, avec R5, R6 et R7, indépendamment l'un de l'autre, choisis parmi H ou alkyles en C1-C18, linéaire, ramifié ou cyclique, notamment méthyle, comprenant éventuellement 1 ou plusieurs hétéroatomes ou encore des groupements protecteurs tels que le t-butyloxycarbonyle ou le 9-fluorenylmethoxycarbonyle.

7. Composition selon l'une des revendications précédentes, **caractérisée en ce que** le monomère de formule (I) est choisi parmi, seul ou en mélange :
- le (méth)acrylate de poly(éthylène glycol) ;
- le (méth)acrylate de méthyl-poly(éthylène glycol) ;
- les (méth)acrylate d'alkyl-poly(éthylène glycol) ;
- les (méth)acrylates de phényl-poly(éthylène glycol);
- le monomère suivant : 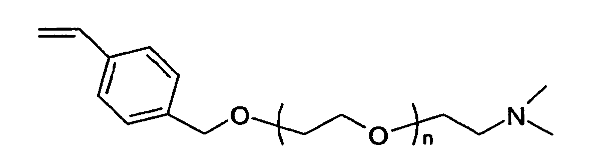
**caractérisée en ce que** n est de préférence compris entre 3 et 100 inclus, notamment 5 à 50 inclus, voire 7 à 30 inclus.

8. Composition selon l'une des revendications précédentes, **caractérisée en ce que** le monomère de formule (I), seul ou en mélange, est présent à raison de 20 à 55% en poids, par rapport au poids du polymère final, de préférence de 30 à 50% en poids.

9. Composition selon l'une des revendications précédentes, **caractérisée en ce que** dans les formules (IIa), (IIb), (IIc) et/ou (IId), le radical R'2 est choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 5 hétéroatomes choisis parmi N, O, S, F, Si et/ou P; ou bien un radical benzylène -C₆H₄-CH₂- éventuellement substitué, par un radical alkyle en C1-C12 comprenant éventuellement 1 à 5 hétéroatomes choisis parmi O, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -CH₂-CH₂-CHR'-O- avec R' et R" représentant un alkyle linéaire
ou ramifié en C1-C22 comprenant éventuellement 1 à 12 hétéroatomes choisis parmi O, N, S, F, Si et P;
- ou un mélange de ces radicaux;

10. Composition selon l'une des revendications précédentes, **caractérisée en ce que** dans la formule (IIa), les radicaux R6 et R7 présents dans X sont choisis parmi l'hydrogène, un groupement méthyle, éthyle, propyle, isopropyle, n-butyle, t-butyle, isobutyle, octyle, lauryle, stéaryle.

11. Composition selon l'une des revendications précédentes, **caractérisée en ce que** dans la formule (IIa), X est un radical choisi parmi les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, phosphonium, et leurs mélanges.

12. Composition selon l'une des revendications précédentes, **caractérisée en ce que** les monomères de formule (IIa) sont neutralisés par des agents neutralisants choisi parmi les agents neutralisants ayant un log P inférieur ou égal à 2, par exemple compris entre -8 et 2, de préférence compris entre -6 et 1, notamment compris entre -6 et 0 ; et/ou par des agents ayant un log P supérieur à 2, de préférence supérieur ou égal à 2,5, notamment supérieur à 3, et en particulier compris entre 3 et 15, voire entre 3,5 et 10.

13. Composition selon l'une des revendications précédentes, **caractérisée en ce que** le monomère de formule (IIa) est choisi parmi, seul ou en mélange :
le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle, et les monomères ci-après :

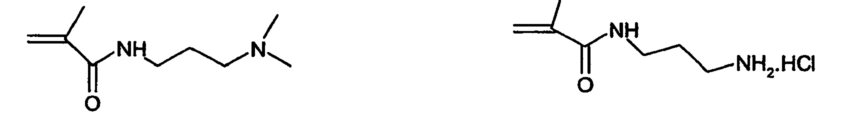

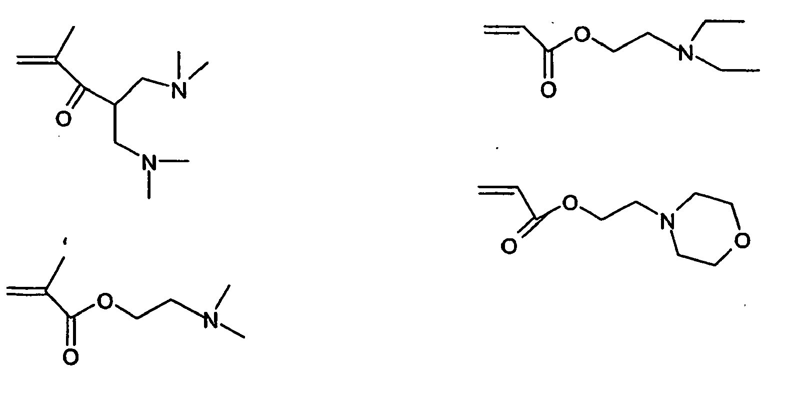

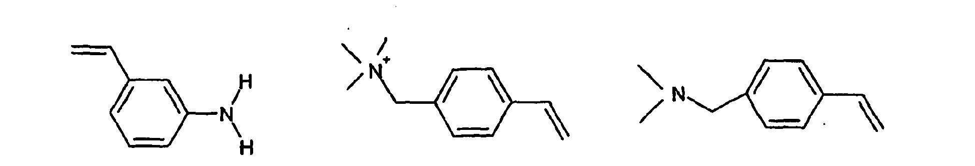

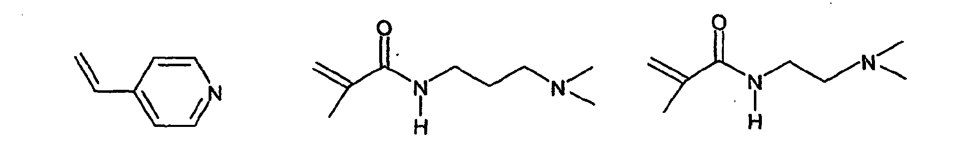

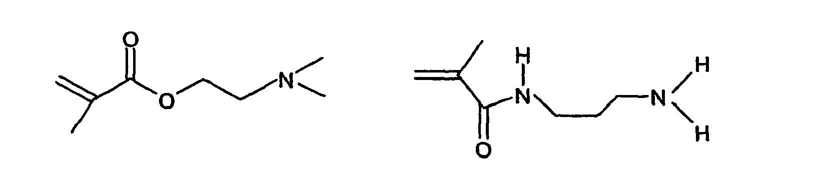

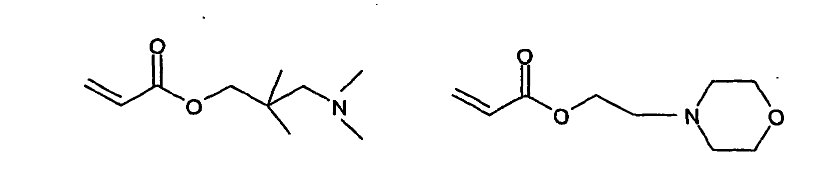

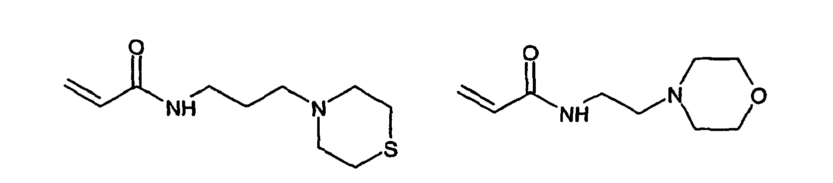

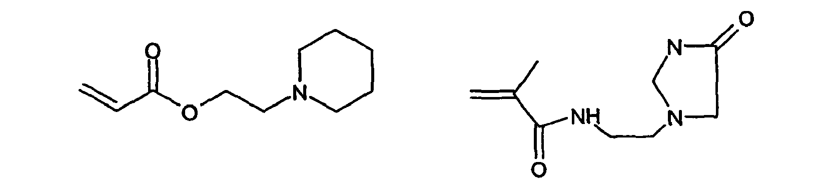

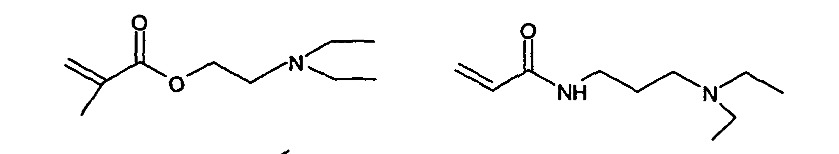

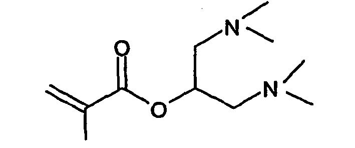

14. Composition selon l'une des revendications précédentes, **caractérisée en ce que** les monomères anioniques sont choisis parmi l'anhydride maléique, l'acide acrylique, l'acide méthacrylique, l'acide crotonique, l'acide itaconique, l'acide fumarique, l'acide maléique, acrylate de 2-carboxyéthyle (CH₂=CH-C(O)-O-(CH₂)₂-COOH); l'acide styrènesulfonique, l'acide 2-acrylamido 2-methylpropanesulfonique, l'acide vinylbenzoïque, l'acide vinylphosphorique, le (méth)acrylate de sulfopropyle, et les sels de ceux-ci.

15. Composition selon l'une des revendications précédentes, **caractérisée en ce que** dans la formule (IIc), le radical X'⁺ est choisi parmi les radicaux de type pyridine, indolyle, isoindolinyle, imidazolyle, imidazolinyle, piperidinyl, pyrazolynyl, pyrazolyl, quinoline, pyrazolinyl, pyridinyl, piperazinyl, pyrrolidinyl, quinidinyl, thiazolinyl, morpholine, guanidino, amidino, et leurs mélanges.

16. Composition selon l'une des revendications précédentes, **caractérisée en ce que** dans les formules (IIc) et/ou (IId), le radical R'3 est choisi parmi :
- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène -C₆H₄-(ortho, méta ou para) éventuellement substitué, par un radical alkyle en C1-C12 comprenant éventuellement 1 à 5 hétéroatomes choisis parmi 0, N, S, F, Si et P; ou bien un radical benzylène -C₆H₄-CH₂- éventuellement substitué, par un radical alkyle en C1-C12 comprenant éventuellement 1 à 5 hétéroatomes choisis parmi 0, N, S, F, Si et P;
- un radical de formule -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, - CH₂-CH₂-O-CO-NH-; -CH₂-NH-CO-NH- ou -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, - CH₂-CH(NHR')-, -CH₂-CH₂-CH(NR'R")-, -CH₂-CH(NR'R")-, -CH₂-CH₂-CH₂-NR'-, - CH₂-CH₂-CH₂-O-; -[CH₂-CH₂-O]ₙ- et -[CH₂-CH(CH₃)-O]ₙ-, -CH₂-CH₂-CHR'-O- avec R' et R" représentant un alkyle linéaire ou ramifié en C1-C22 comprenant éventuellement 1 à 12 hétéroatomes choisis parmi O, N, S, F, Si et P;
- ou un mélange de ces radicaux;

17. Composition selon l'une des revendications précédentes, **caractérisée en ce que** dans la formule (IId), X"⁺ est choisi parmi les radicaux triméthylammonium; triéthylammonium; N,N-diméthyl,N-octylammonium; N,N-diméthyl,N-laurylammonium.

18. Composition selon l'une des revendications précédentes, **caractérisée en ce que** le monomère de formules (IIc) ou (IId) sont choisis parmi la N,N-dimethyl-N-(2-methacryloyloxyethyl)-N-(3-sulfopropyl) ammonium bétaïne, la N,N-dimethyl-N-(3-methacrylamidopropyl)-N-(3-sulfopropyl) ammonium bétaïne, la 1-(3-sulfopropyl)-2- vinylpyridinlum bétaïne et la 2-méthacryloyloxyéthyl phosphorylcholine.

19. Composition selon l'une des revendications précédentes, **caractérisée en ce que**, lorsque le monomère 'essentiellement cationique' est choisi parmi les mélanges de monomères cationiques et/ou amphotères avec des monomères anioniques, lesdits monomères anioniques sont de préférence présents à raison de 5 à 40% en poids, par rapport au poids du mélange "cationiques et/ou amphotères + anioniques", notamment de 10 à 30% en poids, de préférence de 15 à 25% en poids.

20. Composition selon l'une des revendications précédentes, **caractérisée en ce que** le monomère 'essentiellement cationique' est présent à raison de 45 à 80% en poids par rapport au poids du polymère final, de préférence de 50 à 70% en poids.

21. Composition selon l'une des revendications précédentes, **caractérisée en ce que** le monomère hydrophile non ionique additionnel présente un log P compris entre -8 et 2, de préférence inférieur ou égal à 1,5, notamment inférieur ou égal à 1, et en particulier compris entre -7 et 1, voire entre -6 et 0.

22. Composition selon l'une des revendications précédentes, **caractérisée en ce que** le monomère hydrophile non ionique additionnel est choisi parmi ceux de formule (III), seuls ou en mélange, : 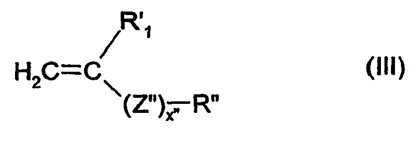 dans laquelle :
- R'₁ est l'hydrogène ou -CH₃;
- Z" est un groupement divalent choisi parmi -COO-, -CONH-, -CONCH₃-, -OCO-, - SO₂, -CO-O-CO-, -CO-CH₂-CO- ou -O- ; de préférence COO et CONH;
- x" est 0 ou 1;
- R" est un radical carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P; notamment méthyle, éthyle, propyle, isopropyle, n-butyle, isobutyle, tertiobutyle, phényle, benzyle, ou un radical de formule -CH₂-CH₂-CH₂OH, -CH₂-CH₂-OH, -CH₂-CH₂-CH₂OH ou furfuryle.

23. Composition selon l'une des revendications précédentes, **caractérisée en ce que** le monomère hydrophile non ionique additionnel est choisi parmi : le méthacrylate de méthyle, l'acrylate de méthyle, le méthacrylate d'éthyle, l'acrylate d'éthyle, l'acrylate de propyle, acrylate d'isopropyle, le méthacrylate de tetrahydrofurfuryle, acrylate de tetrahydrofurfuryle, le méthacrylate de 2-hydroxyéthyle, l'acrylate de 2-hydroxyéthyle, le méthacrylate d'éthoxyéthyle, acrylate d'éthoxyéthyle, le N-isopropyl acrylamide, le N-isopropyl méthacrylamide, le N,N-diméthyl acrylamide, le N,N-diméthyl méthacrylamide, l'acétate de vinyle, le méthyl vinyl éther, l'éthyl vinyl éther, la vinylpyrrolidone, la vinylcaprolactame, la N-vinyl acétamide et l'acrylate d'hydroxypropyle ; la N-vinyllactame, l'acrylamide, la N-méthyl acrylamide, la N,N-diméthyl acrylamide, la N-méthyl-N-vinylacétamide, le N-vinylformamide, le N-méthyl-N-vinylformamide, l'alcool vinylique (copolymérisé sous forme d'acétate de vinyle puis hydrolysé).

24. Composition selon l'une des revendications précédentes, **caractérisée en ce que** le monomère hydrophile non ionique additionnel, seul ou en mélange, est présent en une quantité de 0,1 à 35% en poids, de préférence de 1 à 25% en poids, par exemple de 3 à 15% en poids, voire de 5 à 9,5% en poids, par rapport au poids total du polymère.

25. Composition selon l'une des revendications précédentes, **caractérisée en ce que** le copolymère est neutralisé.

26. Composition selon l'une des revendications précédentes, **caractérisée en ce que** le copolymère est neutralisé par un agent neutralisant choisi parmi les acides aliphatiques linéaires, ramifiés ou cycliques et/ou les acides insaturés ou aromatiques, et peuvent notamment comprendre 1 à 1000 atomes de carbone, notamment 2 à 500 atomes de carbone; possédant au moins une fonction acide au sens de Bronsted, et notamment un ou plusieurs groupes acide carboxylique, sulfonique et/ou phosphonique; et pouvant comporter, en outre, un ou plusieurs hétéroatomes choisis parmi O, N, Si, F et P, par exemple sous la forme de groupes hydroxyle.

27. Composition selon l'une des revendications précédentes, **caractérisée en ce que** le copolymère est neutralisé par un agent neutralisant choisi parmi, seul ou en mélange, :
- les acides gras ayant 6 à 32 atomes de carbone, notamment 8 à 28, linéaires, ramifiés ou cycliques, saturés ou insaturés, éventuellement aromatiques, et comportant au moins une fonction COOH ou acide sulfonique (-SO₃H);
- les hydroxyacides, notamment des alphahydroxy-acides, ayant 6 à 32 atomes de carbone, notamment 8 à 28, linéaires, ramifiés ou cycliques, saturés ou insaturés, éventuellement aromatiques, et comportant au moins une fonction COOH ou acide sulfonique (-SO₃H);.
- les acides alkylbenzène-sulfoniques, dans lesquels le groupement alkyle peut comprendre de 4 à 30, notamment 6 à 24, atomes de carbone;
- les agents neutralisants amphotères, notamment du type alkyl-bétaïnes ou alkylamidopropylbétaïnes, dans lesquels le groupement alkyle peut comprendre 4 à 30, notamment 6 à 24, atomes de carbone; en particulier la cocoamidopropylbétaïne.

28. Composition selon l'une des revendications précédentes, **caractérisée en ce que** le copolymère est neutralisé par un agent neutralisant choisi choisi parmi l'acide alpha-hydroxyéthanoïque, l'acide alpha-hydroxyoctanoïque, l'acide alpha-hydroxycaprylique, l'acide ascorbique, l'acide acétique, l'acide benzoïque, l'acide béhénique, l'acide caprique, l'acide citrique, l'acide caproïque, l'acide caprylique, l'acide dodécylbenzène sulfonique, l'acide 2-éthylcaproïque, l'acide folique, l'acide fumarique, l'acide galactarique, l'acide gluconique, l'acide glycolique, l'acide 2-hexadécyl eicosanoïque, l'acide hydroxycaproique, l'acide 12-hydroxystéarique, l'acide isolaurique (ou 2-butyl octanoïque), l'acide isomyristique (ou 2-hexyl octanoïque), l'acide isoarachidique (ou 2-octyl dodécanoïque), l'acide isolignocérique (ou 2-décyl tétradécanoïque), l'acide lactique, l'acide laurique, l'acide malique, l'acide myristique, l'acide oléique, l'acide palmitique, l'acide propionique, l'acide sébacique, l'acide stéarique, l'acide tartrique, l'acide téréphtalique, l'acide trimésique, l'acide undécylénique, la propyle-bétaine, la cocoamidopropylbétaïne, et le chlorhydrate de bétaïne de formule [(CH₃)₃N+CH₂CO₂H.Cl-], ainsi que leurs mélanges.

29. Composition selon l'une des revendications précédentes, **caractérisée en ce que** le copolymère est neutralisé par un agent neutralisant choisi parmi l'acide caproïque, l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement le chlorhydrate de bétaïne et/ou l'acide béhénique.

30. Composition selon l'une des revendications 25 à 29, **caractérisée en ce que** l'agent neutralisant est ajouté en une quantité de 1 à 300%, notamment 5 à 250%, voire 10 à 200%, par rapport aux fonctions amines totales du polymère ou des monomères.

31. Composition selon la revendication 30, dans lequel l'agent neutralisant, seul ou en mélange, est ajouté en une quantité de 1 à 99%, notamment 5 à 90%, voire 10 à 80%, par rapport aux fonctions amines totales du polymère ou des monomères.

32. Composition selon la revendication 30, dans lequel l'agent neutralisant, seul ou en mélange, est ajouté en une quantité de 0,01 à 3 équivalent molaire, notamment 0,05 à 2,5, voire 0,1 à 2 équivalent molaire, par rapport aux fonctions amines totales du polymère ou des monomères.

33. Composition selon l'une des revendications précédentes, **caractérisée en ce que** le copolymère présente une masse moléculaire moyenne en poids (Mw) comprise entre 500 et 5 000 000, notamment comprise entre 1 000 et 3 000 000 et plus préférentiellement comprise entre 2 000 et 2 000 000, voire 4 000 et 500 000, entre autre 7 000 et 250 000, encore mieux 8 000 et 100 000.

34. Composition selon l'une des revendications précédentes, **caractérisée en ce que** le copolymère comprend:
- un monomère de formule (I), seul ou en mélange, présent à raison de 20 à 80% en poids, par rapport au poids du polymère final, notamment de 20 à 60% en poids, de préférence de 30 à 50% en poids, et choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- un monomère 'essentiellement cationique' est présent à raison de 40 à 90% en poids par rapport au poids du polymère final, notamment de 40 à 80% en poids, de préférence de 50 à 70% en poids et est choisi parmi, seul ou en mélange, le (méth)acrylamide de diméthylaminopropyle, le (méth)acrylamide de diméthylaminoéthyle, le (méth)acrylate de diéthylaminoéthyle, le (méth)acrylate de diméthylaminoéthyle, la vinylimidazole, la vinylpyridine, le (méth)acrylate de morpholinoéthyle; et
- le copolymère étant neutralisé par un agent neutralisant choisi parmi l'acide 2-éthylcaproïque, l'acide oléïque, l'acide béhénique, l'acide stéarique, l'acide acétique, l'acide citrique, l'acide tartrique, le chlorhydrate de bétaïne et/ou l'acide gluconique, et préférentiellement l'acide béhénique et/ou le chlorhydrate de bétaïne.

35. Composition selon l'une des revendications précédentes, **caractérisée en ce que** le copolymère comprend:
- un monomère de formule (I), seul ou en mélange, présent à raison de 20 à 80% en poids, par rapport au poids du polymère final, notamment de 20 à 60% en poids, de préférence de 30 à 50% en poids, et choisi parmi, seul ou en mélange, les (méth)acrylates de poly(éthylène glycol) de préférence ceux ayant un poids moléculaire compris entre 350 et 13 000 g/mol., notamment entre 500 et 8000 g/mol.; et
- un monomère 'essentiellement cationique' est présent à raison de 40 à 90% en poids par rapport au poids du polymère final, notamment de 40 à 80% en poids, de préférence de 50 à 70% en poids et choisi parmi, seul ou en mélange, le (méth)acrylamide de diméthylaminopropyle, et
- le polymère étant neutralisé par un agent neutralisant choisi parmi l'acide béhénique et/ou le chlorhydrate de bétaïne.

36. Composition selon l'une des revendications précédentes, dans laquelle le copolymère est présent à raison de 0,01 à 30% en poids de matière sèche, notamment de 0,1 à 20% en poids, voire de 0,1 à 10% en poids, encore mieux de 0,5 à 3% en poids, par rapport au poids total de la composition.

37. Composition selon l'une quelconque des revendications précédentes, **caractérisée par le fait que** l'agent tensioactif anionique est choisi parmi les sels des alkylsufates, les alkyléthersulfates, les alkyléthercarboxylates, et leur mélanges.

38. Composition selon l'une quelconque des revendications précédentes, **caractérisée par le fait que** le ou les agents tensioactifs anioniques sont présents à une concentration allant de 3% à 40% en poids, de préférence de 5% à 25% en poids par rapport au poids total de la composition.

39. Composition selon l'une quelconque des revendications précédentes, **caractérisée par le fait que** l'agent tensioactif non ionique est choisi parmi les alcools, les alpha-diols, les alkylphénols ou les acides gras polyéthoxylés, polypropoxylés ou polyglycérolés, ayant une chaîne grasse comportant par exemple 8 à 22 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C10-C14) amines ou les oxydes de N-acylaminopropylmorpholine.

40. Composition selon l'une quelconque des revendications précédentes, **caractérisée par le fait que** le ou les agents tensioactifs non ioniques sont présents à une concentration allant de 0.5% à 25% en poids, en particulier de 1% à 20% en poids, rapportée au poids total de la composition cosmétique et plus particulièrement de 2 à 10% en poids.

41. Composition selon l'une quelconque des revendications précédentes, **caractérisée par le fait qu'**elle comprend en outre un tensioactif amphotère.

42. Composition selon l'une quelconque des revendications précédentes, **caractérisée par le fait que** la quantité totale de tensioactif va de 3,5% à 50% en poids, de préférence de 5% à 30% en poids, et encore plus préférentiellement de 8% à 25% en poids, par rapport au poids total de la composition.

43. Composition selon l'une des revendications précédentes, dans laquelle le milieu cosmétiquement acceptable comprend au moins un constituant choisi parmi l'eau ; les solvants organiques hydrophiles comme les alcools, notamment les monoalcools, linéaires ou ramifiés en C1-C6 et les polyols et les éthers de glycols

44. Composition selon l'une quelconque des revendications précédentes, **caractérisée par le fait que** la composition comprend en outre au moins un polymère cationique ou amphotère.

45. Composition selon la revendication précédente, **caractérisée en ce que** le ou les polymères cationiques ou amphotères représentent de 0,001 % à 20 % en poids, de préférence de 0,01 % à 10% en poids par rapport au poids total de la composition finale.

46. Composition selon l'une quelconque des revendications précédentes, **caractérisée par le fait que** la composition comprend en outre au moins une silicone.

47. Composition selon la revendication précédente, **caractérisée en ce que** le ou les silicones représentent de 0,001 % à 20 % en poids, de préférence de 0,1 % à 5% en poids par rapport au poids total de la composition finale.

48. Composition selon l'une quelconque des revendications précédentes, **caractérisée par le fait que** la composition comprend en outre au moins un additif choisi parmi les cires, les corps gras pâteux, les gommes et leurs mélanges, les huiles d'origine animale, végétale, minérale ou synthétique, les esters et les éthers de synthèse ; des alcools gras ayant de 12 à 26 atomes de carbone ; les huiles siliconées volatiles ou non, linéaires ou cycliques, liquides ou pâteux à température ambiante;les polymères différents des polymères à groupement PEG; les vitamines, les parfums, les agents nacrants, les épaississants, les gélifiants, les oligo-éléments, les adoucissants, les séquestrants, les parfums, les agents alcalinisants ou acidifiants, les conservateurs, les filtres solaires, les anti-oxydants, les agents anti-chutes des cheveux, les agents anti-pelliculaires, les agents propulseurs, les céramides ; leurs mélanges.

49. Composition selon l'une des revendications précédentes, se présentant sous la forme d'une composition capillaire, notamment pour le maintien de la coiffure ou la mise en forme des cheveux, par exemple sous forme de shampooings.

50. Procédé cosmétique de traitement des matières kératiniques telles que la peau du corps ou du visage, des ongles, des poils, des cheveux et/ou des cils, **caractérisé en ce qu'**il consiste à appliquer sur les matières kératiniques une composition cosmétique telle que définie dans l'une des revendications 1 à 34 et à faire suivre ou non l'application d'un rinçage après un éventuel temps de pause.

## Claims

1. Cosmetic composition, **characterized in that** it comprises, in a cosmetically acceptable aqueous medium:
I) at least one anionic surfactant and at least one nonionic surfactant, and
II) at least one ethylenic copolymer comprising, as a weight percentage relative to the total weight of the polymer:
- a) 10-60% by weight of one or more monomers of formula (I): 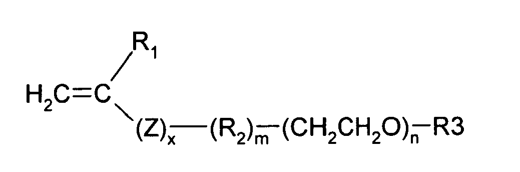 in which:
- R1 is a hydrogen atom or a linear or branched hydrocarbon-based radical, of the type CₚH₂ₚ₊₁, with p being an integer between 1 and 12 inclusive;
- Z is a divalent group chosen from -COO-, -CONH-,
- CONCH₃-, -OCO-, -O-, -SO₂-, -CO-O-CO- and -CO-CH₂-CO-;
- x is 0 or 1;
- R2 is a saturated or unsaturated, optionally aromatic, linear, branched or cyclic carbon-based divalent radical of 1 to 30 carbon atoms, possibly comprising 1 to 18 heteroatoms chosen from O, N, S, F, Si and P;
- m is 0 or 1;
- n is an integer between 3 and 300 inclusive;
- R3 is a hydrogen atom or a saturated or unsaturated, optionally aromatic, linear, branched or cyclic carbon-based radical of 1 to 30 carbon atoms, possibly comprising 1 to 20 heteroatoms chosen from 0, N, S, F, Si and P;
and salts thereof;
- b) 40-90% by weight of at least one "essentially cationic" monomer or a salt thereof, chosen from:
- (i) one or more cationic monomers of formula (IIa),
- (ii) one or more amphoteric monomers of formulae (IIc) and (IId), and
- (iii) a mixture of one or more cationic monomers of formula (IIa) with one or more anionic monomers chosen from maleic anhydride and/or those of formula (IIb); and/or with one or more amphoteric monomers chosen from those of formulae (IIc) and (IId); 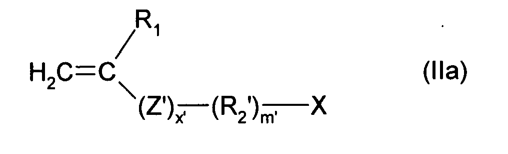 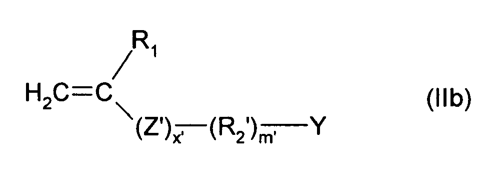 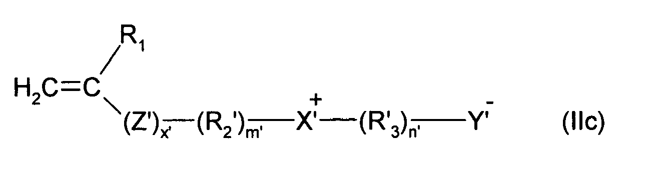 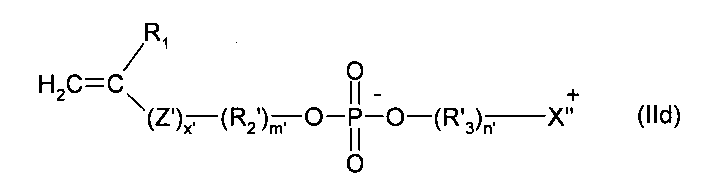
in which:
- R1 is a hydrogen atom or a linear or branched hydrocarbon-based radical of the type CₚH₂ₚ₊₁, with p being an integer between 1 and 12 inclusive; preferably hydrogen or a methyl, ethyl, propyl or butyl radical.
- Z' is a divalent group chosen from -COO-, -CONH-,
- CONCH₃-, -OCO- or -0-, -SO₂- -CO-O-CO- or -CO-CH₂-CO-; preferably, COO and CONH.
- x' is 0 or 1, preferably 1.
- R'2 is a saturated or unsaturated, optionally aromatic, linear, branched or cyclic divalent carbon-based radical of 1 to 30 carbon atoms, possibly comprising 1 to 18 heteroatoms chosen from O, N, S, F, Si and P;
- m' is 0 or 1;
- X (in formula IIa) is (a) a group of formula - N(R₆)(R₇) or -P(R₆)(R₇) or -P⁺R₆R₇R₈, with R6, R7 and R8 representing, independently of each other, either (i) a hydrogen atom, or (ii) a linear, branched or cyclic, saturated or unsaturated, optionally aromatic alkyl group containing from 1 to 18 carbon atoms, possibly comprising 1 to 10 heteroatoms chosen from O, N, S, F, Si and P; or (iii) R6 and R7 may form with the nitrogen or phosphorus atom a first saturated or unsaturated, optionally aromatic ring comprising in total 5, 6, 7 or 8 atoms, and especially 4, 5 or 6 carbon atoms and/or 2 to 4 heteroatoms chosen from O, S and N; said first ring possibly being fused with one or more other saturated or unsaturated, optionally aromatic rings each comprising 5, 6 or 7 atoms, and especially 4, 5, 6 or 7 carbon atoms and/or 2 to 4 heteroatoms chosen from O, S and N;
or (b) X represents a group -R'6-N-R'7- in which R'6 and R'7 form with the nitrogen atom a saturated or unsaturated, optionally aromatic ring comprising in total 5, 6, 7 or 8 atoms, and especially 4, 5 or 6 carbon atoms and/or 2 to 4 heteroatoms chosen from 0, S and N; said ring possibly being fused with one or more other saturated or unsaturated, optionally aromatic rings, each comprising 5, 6 or 7 atoms, and especially 4, 5, 6, 7 or 8 carbon atoms and/or 2 to 4 heteroatoms chosen from 0, S and N;
- Y is a group chosen from -COOH, -SO₃H, -OSO₃H, -PO₃H₂ and -OPO₃H₂;
- X'⁺ is a divalent group of formula -N⁺(R₆)(R₇)- with R6 and R7 representing, independently of each other, either (i) a hydrogen atom, or (ii) a linear, branched or cyclic, optionally aromatic alkyl group containing from 1 to 25 carbon atoms, possibly comprising 1 to 20 heteroatoms chosen from 0, N, S and P; or (iii) R6 and R7 may form with the nitrogen atom a first saturated or unsaturated, optionally aromatic ring comprising in total 5, 6, 7 or 8 atoms, and especially 4, 5, 6 or 7 carbon atoms and/or 2 to 3 heteroatoms chosen from O, S and N; said first cycle possibly being fused with one or more other saturated or unsaturated, optionally aromatic rings, each comprising 5, 6, 7 or 8 atoms, and especially 4, 5, 6 or 7 carbon atoms and/or 2 to 3 heteroatoms chosen from 0, S and N;
- Y'⁻ is a group chosen from -COO⁻, -SO₃⁻, -OSO₃⁻, -PO₃²⁻ and
- OPO₃²⁻;
- R'3 is a saturated or unsaturated, optionally aromatic, linear, branched or cyclic divalent carbon-based radical of 1 to 30 carbon atoms, possibly comprising 1 to 18 heteroatoms chosen from 0, N, S, F, Si and P;
- n' is between 1 and 100 and preferably between 1 and 5 inclusive;
- X"⁺ is a group of formula -N⁺R₆R₇R₈ with R6, R7 and R8 representing, independently of each other, either (i) a hydrogen atom, or (ii) a linear, branched or cyclic, optionally aromatic alkyl group containing from 1 to 18 carbon atoms, possibly comprising 1 to 5 heteroatoms chosen from O, N, S and P; or (iii) R6 and R7 may form with the nitrogen atom a first saturated or unsaturated, optionally aromatic ring comprising in total 5, 6 or 7 atoms, and especially 4, 5 or 6 carbon atoms and/or 2 to 3 heteroatoms chosen from O, S and N; said first ring possibly being fused with one or more other saturated or unsaturated, optionally aromatic rings, each comprising 5, 6 or 7 atoms, and especially 4, 5, 6 or 7 carbon atoms and/or 2 to 3 heteroatoms chosen from O, S and N;
- c) and optionally 0-50% by weight of nonionic hydrophilic monomers, excluding methyl acrylate, methyl methacrylate and isopropyl acrylate if they are present in an amount of greater than or equal to 10% by weight.

2. Composition according to Claim 1, **characterized in that**, in formula (I), R1 represents hydrogen or a methyl, ethyl, propyl or butyl radical.

3. Composition according to either of the preceding claims, **characterized in that**, in formula (I), Z represents COO or CONH.

4. Composition according to one of the preceding claims, **characterized in that**, in formula (I), the radical R2 is chosen from:
- an alkylene radical such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para), optionally substituted with a C1-C12 alkyl radical optionally comprising 1 to 25 heteroatoms chosen from O, N, S, F, Si and P; or alternatively a benzylene radical -C₆H₄-CH₂-, optionally substituted with a C1-C12 alkyl radical optionally comprising 1 to 8 heteroatoms chosen from O, N, S, F, Si and P;
- a pyridinium radical of formula: 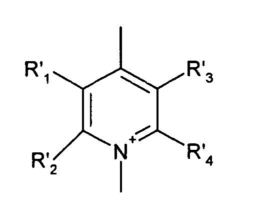 with R'1 to R'4, which may be identical or different, chosen from H and a C1-C12 alkyl radical optionally comprising 1 to 8 heteroatoms chosen from 0, N, S, F, Si and P; R'1 to R'4 may especially be methyl and/or ethyl;
- a radical of formula -CH₂-CHOH-, -CH₂-CH₂-CHOH-, CH₂-CH₂-CH(NH₂)-, -CH₂-CH(NH₂)-, -CH₂-CH₂-CH(NHR')-, -CH₂-CH(NHR')-, -CH₂-CH₂-CH (NR'R") -, -CH₂-CH (NR'R") -, -CH₂-CH₂-CH₂-NR'-, -CH₂-O-CO-O-, CH₂-CH₂-O-CO-O-, -CH₂-CO-O-,
- CH₂-CH₂-CO-O-, -CH₂-O-CO-NH-, -CH₂-CH₂-O-CO-NH-; -CH₂-NH-CO-NH- or -CH₂-CH₂-NH-CO-NH-; -CH₂-CH₂-CH₂-O-; -CH₂-CH₂-CHR'-O- with R' and R" representing a linear or branched C1-C22 alkyl optionally comprising 1 to 12 heteroatoms chosen from O, N, S, F, Si and P;
- or a mixture of these radicals.

5. Composition according to one of the preceding claims, **characterized in that**, in formula (I), n is between 5 and 200 inclusive and better still between 7 and 100 inclusive, or even between 9 and 50 inclusive.

6. Composition according to one of the preceding claims, **characterized in that**, in formula (I), R3 is a hydrogen atom; a succinimido, maleimido, mesityl, tosyl, triethoxysilane, phthalimide or -CH₂-CH₂CN radical; or alternatively a benzyl or phenyl radical optionally substituted with a C1-C12 alkyl radical optionally comprising 1 to 8 heteroatoms chosen from 0, N, S, F, Si and P; a C1-C30 and especially C1-C22 or even C2-C16 alkyl radical, optionally comprising 1 to 18 heteroatoms chosen from 0, N, S, F, Si and P; said benzyl, phenyl or alkyl radicals also possibly comprising a function chosen from the following functions: succinimido; glutarate-succinimido; glutarate; maleimido; mesityl, benzoate; tosyl; triethoxysilane; phthalimide; thioester; benzotriazole carbonate; butyraldehyde; acetaldehyde diethyl acetal; biotin; phospholipid ; succinate; N-hydroxysuccinimide;
- SO₃H, -COOH, -PO₄, -NR5R6 or -N⁺ R5R6R7, with R5, R6 and R7, independently of each other, chosen from H and linear, branched or cyclic C1-C18 alkyls, especially methyl, optionally comprising one or more heteroatoms or alternatively protecting groups such as t-butyloxycarbonyl or 9-fluorenylmethoxycarbonyl.

7. Composition according to one of the preceding claims, **characterized in that** the monomer of formula
(I) is chosen, alone or as a mixture, from:
- poly(ethylene glycol) (meth)acrylate;
- methylpoly(ethylene glycol) (meth)acrylate;
- alkylpoly(ethylene glycol) (meth)acrylates;
- phenylpoly(ethylene glycol) (meth)acrylates;
- the following monomer: 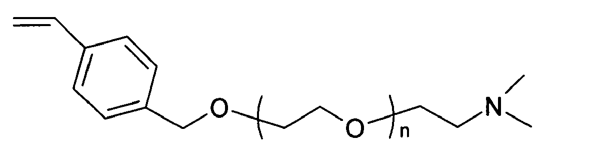
**characterized in that** n is preferably between 3 and 100 inclusive and especially 5 to 50 inclusive, or even 7 to 30 inclusive.

8. Composition according to one of the preceding claims, **characterized in that** the monomer of formula (I), alone or as a mixture, is present in a proportion of from 20% to 55% by weight and preferably from 30% to 50% by weight relative to the weight of the final polymer.

9. Composition according to one of the preceding claims, **characterized in that**, in formulae (IIa), (IIb), (IIc) and/or (IId), the radical R'2 is chosen from:
- an alkylene radical such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para), optionally substituted with a C1-C12 alkyl radical optionally comprising 1 to 5 heteroatoms chosen from N, O, S, F, Si and/or P; or alternatively a benzylene radical -C₆H₄-CH₂-, optionally substituted with a C1-C12 alkyl radical optionally comprising 1 to 5 heteroatoms chosen from O, N, S, F, Si and P;
- a radical of formula -CH₂-O-CO-O-, CH₂-CH₂-O-CO-O-, -CH₂-CO-O-, -CH₂-CH₂-CO-O-, - [(CH₂)₅-CO-O]ₙ-, -CH₂-CH(CH₃)-O-, -(CH₂)₂-O-, -CH₂-O-CO-NH-, -CH₂-CH₂-O-CO-NH-; -CH₂-NH-CO-NH- or -CH₂-CH₂-NH-CO-NH-, -CH₂-CHOH-, -CH₂-CH₂-CHOH-, -CH₂-CH₂-CH (NH₂)-, -CH₂-CH(NH₂)-, -CH₂-CH₂-CH(NHR')-, -CH₂-CH (NHR')-, -CH₂-CH₂-CH(NR'R")-, -CH₂-CH(NR'R")-, -CH₂-CH₂-CH₂-NR'-, -CH₂-CH₂-CH₂-O-; -CH₂-CH₂-CHR'-O- with R' and R" representing a linear or branched C1-C22 alkyl optionally comprising 1 to 12 heteroatoms chosen from 0, N, S, F, Si and P;
- or a mixture of these radicals.

10. Composition according to one of the preceding claims, **characterized in that**, in formula (IIa), the radicals R6 and R7 present in X are chosen from hydrogen and a methyl, ethyl, propyl, isopropyl, n-butyl, t-butyl, isobutyl, octyl, lauryl or stearyl group.

11. Composition according to one of the preceding claims, **characterized in that**, in formula (IIa), X is a radical chosen from radicals of pyridine, indolyl, isoindolinyl, imidazolyl, imidazolinyl, piperidyl, pyrazolinyl, pyrazolyl, quinoline, pyrazolinyl, pyridyl, piperazinyl, pyrrolidinyl, quinidinyl, thiazolinyl, morpholine, guanidino, amidino or phosphonium type, and mixtures thereof.

12. Composition according to one of the preceding claims, **characterized in that** the monomers of formula (IIa) are neutralized with neutralizers chosen from neutralizers with a log P of less than or equal to 2, for example between -8 and 2, preferably between -6 and 1 and especially between -6 and 0; and/or with agents with a log P of greater than 2, preferably greater than or equal to 2.5, especially greater than 3, and in particular between 3 and 15, or even between 3.5 and 10.

13. Composition according to one of the preceding claims, **characterized in that** the monomer of formula (IIa) is chosen, alone or as a mixture, from: dimethylaminopropyl(meth)acrylamide, dimethylaminoethyl(meth)acrylamide, diethylaminoethyl (meth)acrylate, dimethylaminoethyl (meth)acrylate, vinylimidazole, vinylpyridine and morpholinoethyl (meth)acrylate, and the monomers below:

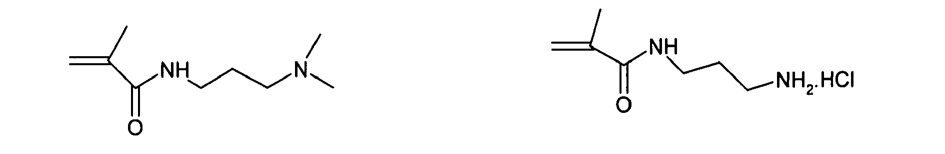

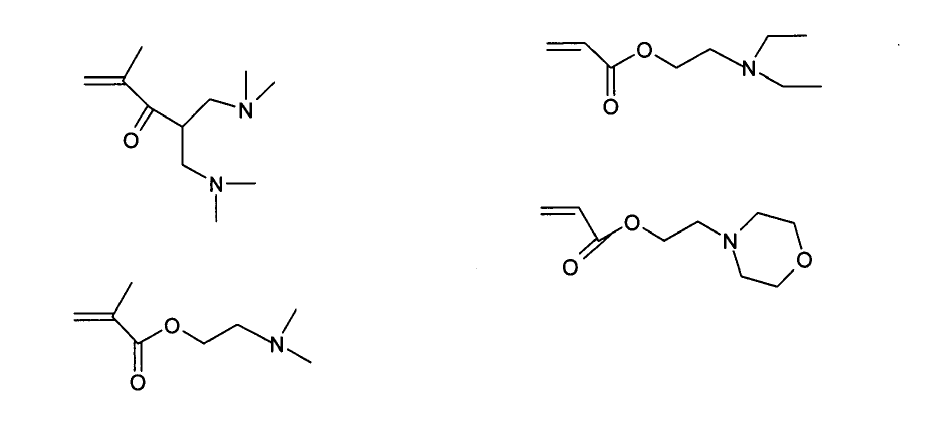

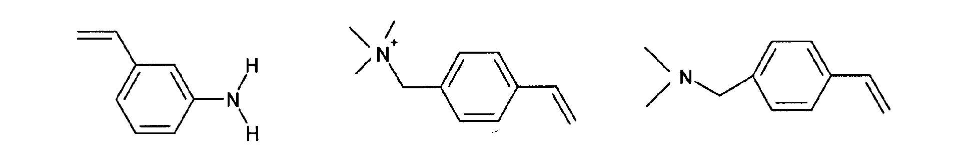

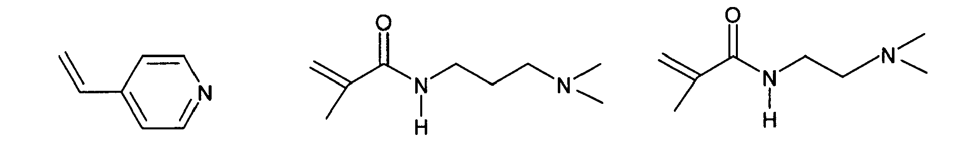

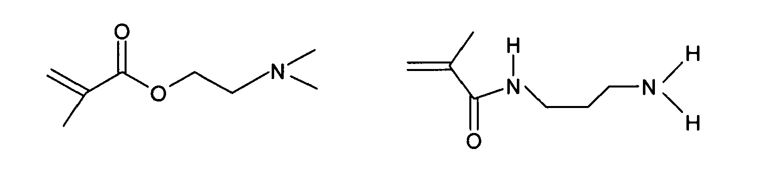

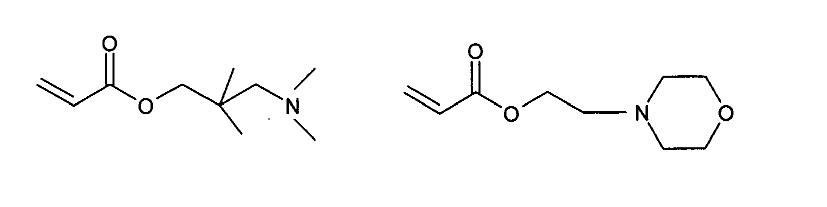

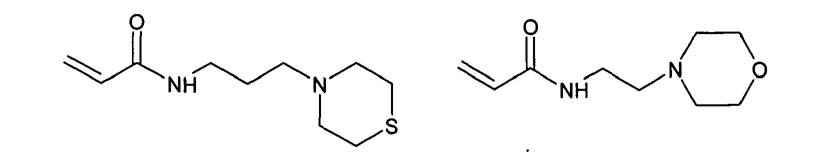

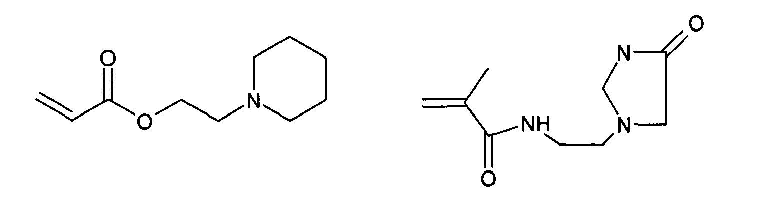

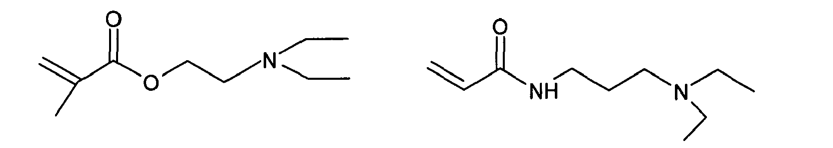

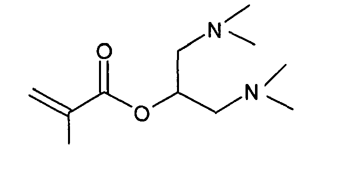

14. Composition according to one of the preceding claims, **characterized in that** the anionic monomers are chosen from maleic anhydride, acrylic acid, methacrylic acid, crotonic acid, itaconic acid, fumaric acid, maleic acid, 2-carboxyethyl acrylate (CH2=CH-C(O)-O-(CH₂)₂-COOH); styrenesulfonic acid, 2-acrylamido-2-methylpropanesulfonic acid, vinylbenzoic acid, vinylphosphoric acid and sulfopropyl (meth)acrylate, and the salts thereof.

15. Composition according to one of the preceding claims, **characterized in that**, in formula (IIc), the radical X'⁺ is chosen from the radicals of pyridine, indolyl, isoindolinyl, imidazolyl, imidazolinyl, piperidyl, pyrazolinyl, pyrazolyl, quinoline, pyrazolinyl, pyridyl, piperazinyl, pyrrolidinyl, quinidinyl, thiazolinyl, morpholine, guanidino and amidino type, and mixtures thereof.

16. Composition according to one of the preceding claims, **characterized in that**, in formulae (IIc) and/or (IId), the radical R'3 is chosen from:
- an alkylene radical such as methylene, ethylene, propylene, n-butylene, isobutylene, tert-butylene, n-hexylene, n-octylene, n-dodecylene, n-octadecylene, n-tetradecylene or n-docosanylene;
- a phenylene radical -C₆H₄- (ortho, meta or para), optionally substituted with a C1-C12 alkyl radical optionally comprising 1 to 5 heteroatoms chosen from O, N, S, F, Si and P; or alternatively a benzylene radical
- C₆H₄-CH₂-, optionally substituted with a C1-C12 alkyl radical optionally comprising 1 to 5 heteroatoms chosen from 0, N, S, F, Si and P;
- a radical of formula -CH₂-O-CO-O-, CH₂-CH₂-O-CO-O-,
- CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, -CH₂-CH₂-O-CO-NH-;
- CH₂-NH-CO-NH- or -CH₂-CH₂-NH-CO-NH-, -CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-CH₂-CH₂-O-; -[CH₂-CH₂-O]ₙ- and -[CH₂-CH(CH₃)-O]ₙ-, -CH₂-CH₂-CHR'-O- with R' and R" representing a linear or branched C1-C22 alkyl optionally comprising 1 to 12 heteroatoms chosen from O, N, S, F, Si and P;
- or a mixture of these radicals.

17. Composition according to one of the preceding claims, **characterized in that**, in formula (IId), X"⁺ is chosen from trimethylammonium; triethylammonium; N,N-dimethyl-N-octylammonium; N,N-dimethyl-N-laurylammonium radicals.

18. Composition according to one of the preceding claims, **characterized in that** the monomer of formula (IIc) or (IId) is chosen from N,N-dimethyl-N-(2-methacryloyloxyethyl)-N-(3-sulfopropyl)ammonium betaine, N,N-dimethyl-N-(3-methacrylamidopropyl)-N-(3-sulfopropyl)ammonium betaine, 1-(3-sulfopropyl)-2-vinylpyridinium betaine and 2-methacryloyloxyethylphosphorylcholine.

19. Composition according to one of the preceding claims, **characterized in that**, when the "essentially cationic" monomer is chosen from mixtures of cationic and/or amphoteric monomers with anionic monomers, said anionic monomers are preferably present in a proportion of from 5% to 40% by weight, especially from 10% to 30% by weight and preferably from 15% to 25% by weight relative to the weight of the "cationic and/or amphoteric + anionic" mixture.

20. Composition according to one of the preceding claims, **characterized in that** the "essentially cationic" monomer is present in a proportion of from 45% to 80% by weight and preferably from 50% to 70% by weight relative to the weight of the final polymer.

21. Composition according to one of the preceding claims, **characterized in that** the additional nonionic hydrophilic monomer has a log P of between -8 and 2, preferably less than or equal to 1.5, especially less than or equal to 1 and in particular between -7 and 1, or even between -6 and 0.

22. Composition according to one of the preceding claims, **characterized in that** the additional nonionic hydrophilic monomer is chosen from those of formula (III), alone or as a mixture: 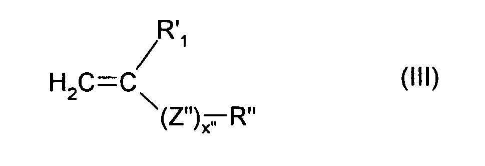 in which:
- R'₁ is hydrogen or -CH₃;
- Z" is a divalent group chosen from -COO-, -CONH-,
- CONCH₃-, -OCO-, -SO₂ , -CO-O-CO-, -CO-CH₂-CO- and -O-; preferably COO and CONH;
- x" is 0 or 1;
- R" is a saturated or unsaturated, optionally aromatic, linear, branched or cyclic carbon-based radical of 1 to 30 carbon atoms, possibly comprising 1 to 18 heteroatoms chosen from O, N, S, F, Si and P; especially methyl, ethyl, propyl, isopropyl, n-butyl, isobutyl, tert-butyl, phenyl, benzyl or a radical of formula -CH₂-CH₂-CH₂OH, -CH₂-CH₂-OH, -CH₂-CH₂-CH₂OH or furfuryl.

23. Composition according to one of the preceding claims, **characterized in that** the additional nonionic hydrophilic monomer is chosen from: methyl methacrylate, methyl acrylate, ethyl methacrylate, ethyl acrylate, propyl acrylate, isopropyl acrylate, tetrahydrofurfuryl methacrylate, tetrahydrofurfuryl acrylate, 2-hydroxyethyl methacrylate, 2-hydroxyethyl acrylate, ethoxyethyl methacrylate, ethoxyethyl acrylate, N-isopropylacrylamide, N-isopropylmethacrylamide, N,N-dimethylacrylamide, N,N-dimethylmethacrylamide, vinyl acetate, methyl vinyl ether, ethyl vinyl ether, vinylpyrrolidone, vinylcaprolactam, N-vinylacetamide and hydroxylpropyl acrylate; N-vinyllactam, acrylamide, N-methylacrylamide, N,N-dimethylacrylamide, N-methyl-N-vinylacetamide, N-vinylformamide, N-methyl-N-vinylformamide, vinyl alcohol (copolymerized in the form of vinyl acetate and then hydrolyzed).

24. Composition according to one of the preceding claims, **characterized in that** the additional nonionic hydrophilic monomer, alone or as a mixture, is present in an amount of from 0.1% to 35% by weight, preferably from 1% to 25% by weight, for example from 3% to 15% by weight, or even from 5% to 9.5% by weight, relative to the total weight of the polymer.

25. Composition according to one of the preceding claims, **characterized in that** the copolymer is neutralized.

26. Composition according to one of the preceding claims, **characterized in that** the copolymer is neutralized with a neutralizer chosen from linear, branched or cyclic aliphatic acids and/or unsaturated or aromatic acids, and may especially contain 1 to 1000 carbon atoms and especially 2 to 500 carbon atoms; containing at least one acid function in the Brönsted sense, and especially one or more carboxylic, sulfonic and/or phosphonic acid groups; and also possibly comprising one or more heteroatoms chosen from 0, N, Si, F and P, for example in the form of hydroxyl groups.

27. Composition according to one of the preceding claims, **characterized in that** the copolymer is neutralized with a neutralizer chosen, alone or as a mixture, from:
- linear, branched or cyclic, saturated or unsaturated, optionally aromatic fatty acids containing 6 to 32 and especially 8 to 28 carbon atoms, and comprising at least one COOH or sulfonic acid (-SO₃H) function;
- linear, branched or cyclic, saturated or unsaturated, optionally aromatic hydroxy acids, especially α-hydroxy acids, containing 6 to 32 and especially 8 to 28 carbon atoms, and comprising at least one COOH or sulfonic acid (-SO₃H) function;
- alkylbenzenesulfonic acids, in which the alkyl group may contain from 4 to 30 and especially from 6 to 24 carbon atoms;
- amphoteric neutralizers, especially of the alkylbetaine or alkylamidopropylbetaine type, in which the alkyl group may contain 4 to 30 and especially 6 to 24 carbon atoms; in particular cocoamidopropylbetaine.

28. Composition according to one of the preceding claims, **characterized in that** the copolymer is neutralized with a neutralizer chosen from α-hydroxyethanoic acid, α-hydroxyoctanoic acid, α-hydroxycaprylic acid, ascorbic acid, acetic acid, benzoic acid, behenic acid, capric acid, citric acid, caproic acid, caprylic acid, dodecylbenzenesulfonic acid, 2-ethylcaproic acid, folic acid, fumaric acid, galactaric acid, gluconic acid, glycolic acid, 2-hexadecyleicosanoic acid, hydroxycaproic acid, 12-hydroxystearic acid, isolauric acid (or 2-butyloctanoic acid), isomyristic acid (or 2-hexyloctanoic acid), isoarachidic acid (or 2-octyldodecanoic acid), isolignoceric acid (or 2-decyltetradecanoic acid), lactic acid, lauric acid, malic acid, myristic acid, oleic acid, palmitic acid, propionic acid, sebacic acid, stearic acid, tartaric acid, terephthalic acid, trimesic acid, undecylenic acid, propylbetaine, cocoamidopropylbetaine, and betaine hydrochloride of formula [(CH₃)₃N+CH₂CO₂H.Cl-], and mixtures thereof.

29. Composition according to one of the preceding claims, **characterized in that** the copolymer is neutralized with a neutralizer chosen from caproic acid, 2-ethylcaproic acid, oleic acid, behenic acid, stearic acid, acetic acid, citric acid, tartaric acid, betaine hydrochloride and/or gluconic acid, and preferentially betaine hydrochloride and/or behenic acid.

30. Composition according to one of Claims 25 to 29, **characterized in that** the neutralizer is added in an amount of from 1% to 300% and especially from 5% to 250% or even 10% to 200% relative to the total amine functions of the polymer or of the monomers.

31. Composition according to Claim 30, in which the neutralizer, alone or as a mixture, is added in an amount of from 1% to 99%, especially 5% to 90% or even 10% to 80% relative to the total amine functions of the polymer or of the monomers.

32. Composition according to Claim 30, in which the neutralizer, alone or as a mixture, is added in an amount of from 0.01 to 3 molar equivalents, especially 0.05 to 2.5 or even 0.1 to 2 molar equivalents relative to the total amine functions of the polymer or of the monomers.

33. Composition according to one of the preceding claims, **characterized in that** the copolymer has a weight-average molecular mass (Mw) of between 500 and 5 000 000, especially between 1000 and 3 000 000 and more preferentially between 2000 and 2 000 000, or even between 4000 and 500 000, better still between 7000 and 250 000 and even better between 8000 and 100 000.

34. Composition according to one of the preceding claims, **characterized in that** the copolymer comprises:
- a monomer of formula (I), alone or as a mixture, present in a proportion of from 20% to 80% by weight, especially from 20% to 60% by weight and preferably from 30% to 50% by weight, relative to the weight of the final polymer, and chosen, alone or as a mixture, from poly(ethylene glycol) (meth)acrylates, preferably those with a molecular weight of between 350 and 13 000 g/mol and especially between 500 and 8000 g/mol; and
- an "essentially cationic" monomer present in a proportion of from 40% to 90% by weight, especially from 40% to 80% by weight and preferably from 50% to 70% by weight relative to the weight of the final polymer, and chosen, alone or as a mixture, from dimethylaminopropyl (meth)acrylamide, dimethylaminoethyl (meth)acrylamide, diethylaminoethyl (meth)acrylate, dimethylaminoethyl (meth)acrylate, vinylimidazole, vinylpyridine and morpholinoethyl (meth)acrylate; and
- the copolymer being neutralized with a neutralizer chosen from 2-ethylcaproic acid, oleic acid, behenic acid, stearic acid, acetic acid, citric acid, tartaric acid, betaine hydrochloride and/or gluconic acid, and preferentially behenic acid and/or betaine hydrochloride.

35. Composition according to one of the preceding claims, **characterized in that** the copolymer comprises:
- a monomer of formula (I), alone or as a mixture, present in a proportion of from 20% to 80% by weight, especially from 20% to 60% by weight and preferably from 30% to 50% by weight, relative to the weight of the final polymer, and chosen, alone or as a mixture, from poly(ethylene glycol) (meth)acrylates, preferably those with a molecular weight of between 350 and 13 000 g/mol and especially between 500 and 8000 g/mol; and
- an "essentially cationic" monomer present in a proportion of from 40% to 90% by weight, especially from 40% to 80% by weight and preferably from 50% to 70% by weight, relative to the weight of the final polymer, and chosen, alone or as a mixture, from dimethylaminopropyl (meth)acrylamide, and
- the polymer being neutralized with a neutralizer chosen from behenic acid and/or betaine hydrochloride.

36. Composition according to one of the preceding claims, in which the copolymer is present in a proportion of from 0.01% to 30% by weight of solids, especially from 0.1% to 20% by weight or even from 0.1% to 10% by weight and better still from 0.5% to 3% by weight relative to the total weight of the composition.

37. Composition according to any one of the preceding claims, **characterized in that** the anionic surfactant is chosen from the salts of alkyl sulfates, alkyl ether sulfates and alkyl ether carboxylates, and mixtures thereof.

38. Composition according to any one of the preceding claims, **characterized in that** the anionic surfactant(s) is (are) present in a concentration ranging from 3% to 40% by weight and preferably from 5% to 25% by weight relative to the total weight of the composition.

39. Composition according to any one of the preceding claims, **characterized in that** the nonionic surfactant is chosen from polyethoxylated, polypropoxylated or polyglycerolated fatty acids, alkylphenols, α-diols or alcohols having a fatty chain containing, for example, 8 to 22 carbon atoms, it being possible for the number of ethylene oxide or propylene oxide groups to range in particular from 2 to 50 and for the number of glycerol groups to range in particular from 2 to 30. Mention may also be made of copolymers of ethylene oxide and of propylene oxide, condensates of ethylene oxide and of propylene oxide with fatty alcohols; polyethoxylated fatty amides preferably having from 2 to 30 mol of ethylene oxide, polyglycerolated fatty amides containing on average 1 to 5, and in particular 1.5 to 4, glycerol groups; oxyethylenated fatty acid esters of sorbitan having from 2 to 30 mol of ethylene oxide; fatty acid esters of sucrose, fatty acid esters of polyethylene glycol, alkylpolyglycosides, N-alkylglucamine derivatives, amine oxides such as (C₁₀-C₁₄) alkylamine oxides or N-acylaminopropylmorpholine oxides.

40. Composition according to any one of the preceding claims, **characterized in that** the nonionic surfactant(s) is (are) present in a concentration ranging from 0.5% to 25% by weight, in particular from 1% to 20% by weight and more particularly from 2% to 10% by weight relative to the total weight of the cosmetic composition.

41. Composition according to any one of the preceding claims, **characterized in that** it also comprises an amphoteric surfactant.

42. Composition according to any one of the preceding claims, **characterized in that** the total amount of surfactant ranges from 3.5% to 50% by weight, preferably from 5% to 30% by weight and even more preferentially from 8% to 25% by weight relative to the total weight of the composition.

43. Composition according to one of the preceding claims, in which the cosmetically acceptable medium comprises at least one constituent chosen from water; hydrophilic organic solvents, for instance alcohols, especially linear or branched C₁-C₆ monoalcohols and polyols and glycol ethers.

44. Composition according to any one of the preceding claims, **characterized in that** the composition also comprises at least one cationic or amphoteric polymer.

45. Composition according to the preceding claim, **characterized in that** the cationic or amphoteric polymer(s) represent(s) from 0.001% to 20% by weight and preferably from 0.01% to 10% by weight relative to the total weight of the final composition.

46. Composition according to any one of the preceding claims, **characterized in that** the composition also comprises at least one silicone.

47. Composition according to the preceding claim, **characterized in that** the silicones represent(s) from 0.001% to 20% by weight and preferably from 0.1% to 5% by weight relative to the total weight of the final composition.

48. Composition according to any one of the preceding claims, **characterized in that** the composition also comprises at least one additive chosen from waxes, pasty fatty substances, gums and mixtures thereof, oils of animal, plant, mineral or synthetic origin, synthetic esters and synthetic ethers; fatty alcohols containing from 12 to 26 carbon atoms; volatile or nonvolatile, linear or cyclic silicone-based oils, which are liquid or pasty at room temperature; polymers other than polymers with a PEG group; vitamins, fragrances, nacres, thickeners, gelling agents, trace elements, softeners, sequestrants, fragrances, acidifying or basifying agents, preserving agents, sunscreens, antioxidants, hair-loss counteractants, antidandruff agents, propellants and ceramides, and mixtures thereof.

49. Composition according to one of the preceding claims, which is in the form of a haircare composition, especially for holding the hair style or shaping the hair, for example in the form of shampoos.

50. Cosmetic process for treating keratin materials such as bodily or facial skin, the nails, bodily hair, head hair and/or the eyelashes, **characterized in that** it consists in applying to the keratin materials a cosmetic composition as defined in one of Claims 1 to 34 and in optionally following the application by rinsing, after an optional action time.

## Patentansprüche

1. Kosmetische Zusammensetzung, **dadurch gekennzeichnet, dass** sie in einem kosmetisch akzeptablen Medium enthält:
I) mindestens einen anionischen grenzflächenaktiven Stoff und mindestens einen nichtionischen grenzflächenaktiven Stoff;
II) mindestens ein ethylenisches Copolymer, das in Gew.-%, bezogen auf das Gesamtgewicht des Polymers, enthält:
a) 10 bis 60 Gew.-% eines oder mehrerer Monomere der Formel (I): 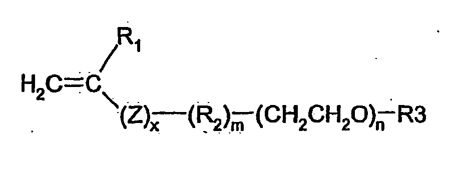 in der Formel:
- R1 ist ein Wasserstoffatom oder eine geradkettige oder verzweigte Kohlenwasserstoffgruppe vom Typ CₚH₂ₚ₊₁, wobei p eine ganze Zahl im Bereich von 1 bis 12 bedeutet;
- Z ist eine zweiwertige Gruppe, die unter -COO-, -CONH-, -CONCH₃-, -OCO- , -O-, -SO₂-, -CO-O-CO- oder -CO-CH₂-CO- ausgewählt ist;
- x ist 0 oder 1;
- R2 bedeutet eine zweiwertige, gesättigte oder ungesättigte, gegebenenfalls aromatische, lineare, verzweigte oder cyclische, kohlenstoffhaltige Gruppe mit 1 bis 30 Kohlenstoffatomen, die 1 bis 18 Heteroatome enthalten kann, die unter O, N, S, F, Si und P ausgewählt sind;
- m ist 0 oder 1;
- n bedeutet eine ganze Zahl von 3 bis einschließlich 300;
- R3 bedeutet ein Wasserstoffatom oder eine gesättigte oder ungesättigte, gegebenenfalls aromatische, lineare, verzweigte oder cyclische, kohlenstoffhaltige Gruppe mit 1 bis 30 Kohlenstoffatomen, die 1 bis 20 Heteroatome enthalten kann, die unter O, N, S, F, Si und P ausgewählt sind;
und deren Salze;
b) 40 bis 90 Gew.-% mindestens eines "im Wesentlichen kationischen" Monomers oder eines seiner Salze, das ausgewählt ist unter:
- (i) einem oder mehreren kationischen Monomeren der Formel (IIa),
- (ii) einem oder mehreren amphoteren Monomeren der Formeln (IIc) und (IId),
- (iii) einem Gemisch aus einem oder mehreren kationischen Monomeren der Formel (IIa) mit einem oder mehreren anionischen Monomeren, die unter Maleinsäureanhydrid und/oder Monomeren der Formel (IIb) ausgewählt sind, und/oder mit einem oder mehreren amphoteren Monomeren, die unter den Monomeren der Formeln (IIc) und (IId) ausgewählt sind; 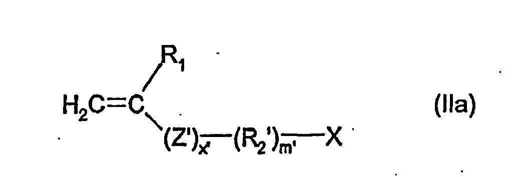 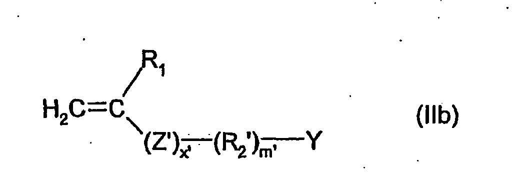 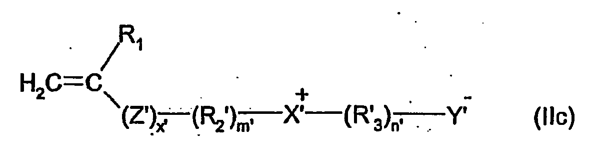 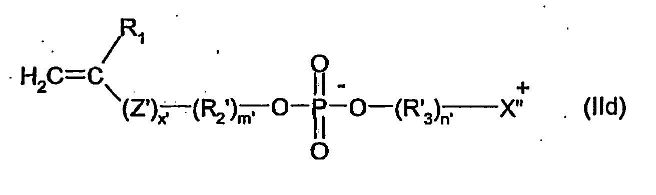
worin bedeuten:
- R1 ist ein Wasserstoffatom oder eine geradkettige oder verzweigte Kohlenwasserstoffgruppe vom Typ CₚH₂ₚ₊₁, wobei p eine ganze Zahl im Bereich von 1 bis 12 bedeutet; vorzugsweise Wasserstoff oder Methyl, Ethyl, Propyl, Butyl;
- Z' ist eine zweiwertige Gruppe, die unter -COO-, -CONH-, -CONCH₃-, -OCO- , -O-, -SO₂-, -CO-O-CO- oder -CO-CH₂-CO- ausgewählt ist; vorzugsweise COO und CONH;
- x' ist 0 oder 1, vorzugsweise 1;
- R'2 bedeutet eine zweiwertige, gesättigte oder ungesättigte, gegebenenfalls aromatische, lineare, verzweigte oder cyclische, kohlenstoffhaltige Gruppe mit 1 bis 30 Kohlenstoffatomen, die 1 bis 18 Heteroatome enthalten kann, die unter O, N, S, F, Si und P ausgewählt sind;
- m' ist 0 oder 1;
- X (in der Formel IIa) ist (a) eine Gruppe der Formel -N(R₆)(R₇) oder -P(R₆)(R₇) oder -P⁺R₆R₇R₈, wobei R6, R7 und R8 unabhängig voneinander entweder (i) ein Wasserstoffatom oder (ii) eine lineare, verzweigte oder cyclische, gesättigte oder ungesättigte, gegebenenfalls aromatische Alkylgruppe mit 1 bis 18 Kohlenstoffatomen bedeuten, die 1 bis 10 Heteroatome enthalten kann, die unter O, N, S, F, Si und P ausgewählt sind; oder (iii) R6 und R7 mit dem Stickstoffatom oder dem Phosphoratom einen gesättigten oder ungesättigten, gegebenenfalls aromatischen ersten Ring bilden können, der insgesamt 5, 6, 7 oder 8 Atome und insbesondere 4, 5 oder 6 Kohlenstoffatome und/oder 2 bis 4 Heteroatome enthält, die unter O, S und N ausgewählt sind; wobei der erste Ring mit einem oder mehreren anderen gesättigten oder ungesättigten, gegebenenfalls aromatischen Ringen verbunden sein kann, die jeweils 5, 6 oder 7 Atome und insbesondere 4, 5, 6 oder 7 Kohlenstoffatome und/oder 2 bis 4 Heteroatome enthalten, die unter O, S und N ausgewählt sind;
oder (b) X bedeutet eine Gruppe -R'6-N-R'7-, wobei R'6 und R'7 mit dem Stickstoffatom einen gesättigten oder ungesättigten, gegebenenfalls aromatischen Ring bilden, der insgesamt 5, 6, 7 oder 8 Atome und insbesondere 4, 5 oder 6 Kohlenstoffatome und/oder 2 bis 4 Heteroatome enthält, die unter O, S und N ausgewählt sind, wobei dieser Ring mit einem oder mehreren anderen gesättigten oder ungesättigten, gegebenenfalls aromatischen Ringen verbunden sein kann, die jeweils 5, 6 oder 7 Atome und insbesondere 4, 5, 6, 7 oder 8 Kohlenstoffatome und/ oder 2 bis 4 Heteroatome enthalten, die unter O, S und N ausgewählt sind;
- Y ist eine Gruppe, die unter -COOH, -SO₃H, -OSO₃H, -PO₃H₂ et -OPO₃H₂ ausgewählt ist;
- X'⁺ ist eine zweiwertige Gruppe der Formel -N⁺(R₆)(R₇)-, wobei R6 und R7 unabhängig voneinander entweder (i) ein Wasserstoffatom oder (ii) eine lineare, verzweigte oder cyclische, gesättigte oder ungesättigte, gegebenenfalls aromatische Alkylgruppe mit 1 bis 25 Kohlenstoffatomen bedeuten, die 1 bis 20 Heteroatome enthalten kann, die unter O, N, S und P ausgewählt sind; oder (iii) R6 und R7 mit dem Stickstoffatom einen gesättigten oder ungesättigten, gegebenenfalls aromatischen ersten Ring bilden können, der insgesamt 5, 6, 7 oder 8 Atome und insbesondere 4, 5, 6 oder 7 Kohlenstoffatome und/oder 2 bis 3 Heteroatome enthält, die unter O, S und N ausgewählt sind, wobei der erste Ring mit einem oder mehreren anderen gesättigten oder ungesättigten, gegebenenfalls aromatischen Ringen verbunden sein kann, die jeweils 5, 6, 7 oder 8 Atome und insbesondere 4, 5, 6 oder 7 Kohlenstoffatome und/oder 2 bis 3 Heteroatome enthalten, die unter O, S und N ausgewählt sind;
- Y'- ist eine Gruppe, die unter -COO-, -SO₃⁻, -OSO₃⁻, -PO₃²⁻ und -OPO₃²⁻ ausgewählt ist;
- R'3 bedeutet eine zweiwertige, gesättigte oder ungesättigte, gegebenenfalls aromatische, lineare, verzweigte oder cyclische, kohlenstoffhaltige Gruppe mit 1 bis 30 Kohlenstoffatomen, die 1 bis 18 Heteroatome enthalten kann, die unter O, N, S, F, Si und P ausgewählt sind;
- n' liegt im Bereich von 1 bis 100 und vorzugsweise 1 bis 5;
- X"⁺ ist eine Gruppe der Formel -N+R₆R₇R₈, wobei R₆, R₇ und R₈ unabhängig voneinander entweder (i) ein Wasserstoffatom oder (ii) eine lineare, verzweigte oder cyclische, gegebenenfalls aromatische Alkylgruppe mit 1 bis 18 Kohlenstoffatomen bedeuten, die 1 bis 5 Heteroatome enthalten kann, die unter O, N, S, und P ausgewählt sind; oder (iii) R₆ und R₇ mit dem Stickstoffatom einen gesättigten oder ungesättigten, gegebenenfalls aromatischen ersten Ring bilden können, der insgesamt 5, 6 oder 7 Atome und insbesondere 4, 5 oder 6 Kohlenstoffatome und/oder 2 bis 3 Heteroatome enthält, die unter O, S und N ausgewählt sind, wobei der erste Ring mit einem oder mehreren anderen gesättigten oder ungesättigten, gegebenenfalls aromatischen Ringen verbunden sein kann, die jeweils 5, 6 oder 7 Atome und insbesondere 4, 5, 6 oder 7 Kohlenstoffatome und/oder 2 bis 3 Heteroatome enthalten, die unter O, S und N ausgewählt sind;
c) und gegebenenfalls 0 bis 50 Gew.-% nichtionischer hydrophiler Monomere, wobei Methylacrylat, Methylmethacrylat und Isopropylacrylat ausgenommen sind, wenn sie in einem Mengenanteil von größer oder gleich 10 Gew.-% vorliegen.

2. Zusammensetzung nach Anspruch 1, **dadurch gekennzeichnet, dass** in der Formel (I) R1 Wasserstoff oder Methyl, Ethyl, Propyl, Butyl bedeutet.

3. Zusammensetzung nach einem der vorhergehenden Ansprüche, **dadurch gekennzeichnet, dass** in der Formel (I) Z COO oder CONH bedeutet.

4. Zusammensetzung nach einem der vorhergehenden Ansprüche, **dadurch gekennzeichnet, dass** in der Formel (I) R2 ausgewählt ist unter:
- einer Alkylengruppe, wie Methylen, Ethylen, Propylen, n-Butylen, Isobutylen, tert-Butylen, n-Hexylen, n-Octylen, n-Dodecylen, n-Octadecylen, n-Tetradecylen, n-Docosanylen;
- einer Phenylengruppe -C₆H₄- (ortho, meta oder para), die gegebenenfalls mit einer C₁₋₁₂-Alkylgruppe substituiert ist, die gegebenenfalls 1 bis 25 Heteroatome enthält, die unter O, N, S, F, Si und P ausgewählt sind; oder eine Benzylengruppe
- C₆H₄-CH₂-, die gegebenenfalls mit einer C₁₋₁₂-Alkylgruppe substituiert ist, die gegebenenfalls 1 bis 8 Heteroatome enthält, die unter O, N, S, F, Si und P ausgewählt sind;
- einer Pyridiniumgruppe der Formel 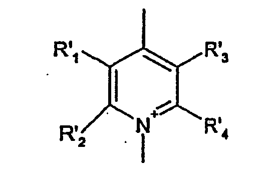
wobei R'1 bis R'4, die gleich oder verschieden sind, unter H und einer C₁₋₁₂-Alkylgruppe ausgewählt sind, die gegebenenfalls 1 bis 8 Heteroatome enthält, die unter O, N, S, F, Si und P ausgewählt sind; wobei R'1 bis R'4 Methyl und/oder Ethyl bedeuten können;
- einer Gruppe der Formel -CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-O-CO-O-, CH₂-CH₂-O-CO-O-, -CH₂-CO-O-, -CH₂-CH₂-CO-O-, -CH₂-O-CO-NH-, -CH₂-CH₂-O-CO-NH-; -CH₂-NH-CO-NH-; oder -CH₂-CH₂-NH-CO-NH- ; -CH₂-CH₂-CH₂-O-; -CH₂-CH₂-CHR'-O-wobei R' et R" eine C₁₋₂₂-Alkylgruppe bedeuten, die gegebenenfalls 1 bis 12 Heteroatome enthält, die unter O, N, S, F, Si und P ausgewählt sind;
- oder einem Gemisch dieser Gruppen.

5. Zusammensetzung nach einem der vorhergehenden Ansprüche, **dadurch gekennzeichnet, dass** in der Formel (I) n im Bereich von 5 bis 200 und noch besser im Bereich von 7 bis 100 und insbesondere 9 bis 50 liegt.

6. Zusammensetzung nach einem der vorhergehenden Ansprüche, **dadurch gekennzeichnet, dass** in der Formel (I) R3 ein Wasserstoffatom; eine Gruppe Succinimido, Maleimido, Mesityl, Tosyl, Triethoxysilan, Phthalimid oder -CH₂-CH₂CN; oder eine Benzyl- oder Phenylgruppe, die gegebenenfalls mit einer C₁₋₁₂-Alkylgruppe substituiert ist, die gegebenenfalls 1 bis 8 Heteroatome enthält, die unter O, N, S, F, Si und P ausgewählt sind; eine C₁₋₃₀-Alkylgruppe, insbesondere C₁₋₂₂-Alkylgruppe, besonders C₁₋₁₆-Alkylgruppe, die gegebenenfalls 1 bis 18 Heteroatome enthält, die unter O, N, S, F, Si und P ausgewählt sind, bedeutet; wobei die Benzyl-, Phenyl- oder Alkylgruppen ferner eine Funktion umfassen können, die unter den folgenden Funktionen ausgewählt ist: Succinimido, Glutaratsuccinimido ; Glutarat ; Maleimido ; Mesityl; Benzoat ; Tosyl ; Triethoxysilan; Phthalimid; Thioester; Benzotriazolcarbonat; Butyraldehyd; Acetaldehyddiethylacetal; Biotin; Phospholipid; Succinat; N-Hydroxysuccinimid; - SO₃H, -COOH, -PO₄, -NR₅R₆ oder -N⁺R₅ R₆R₇, wobei R₅, R₆ und R₇ unabhängig voneinander unter H oder linearen, verzweigten oder cyclischen Alkylgruppen mit 1 bis 18 Kohlenstoffatomen und insbesondere Methyl ausgewählt sind, die gegebenenfalls 1 oder mehrere Heteroatome oder Schutzgruppen enthalten können, wie t-Butyloxycarbonyl oder 9-Fluorenylmethoxycarbonyl.

7. Zusammensetzung nach einem der vorhergehenden Ansprüche, **dadurch gekennzeichnet, dass** das Monomer der Formel (I) einzeln oder als Gemisch ausgewählt ist unter:
Poly(ethylenglycol)(meth)acrylat;
Methyl-poly(ethylenglycol)(meth)acrylat;
Alkyl-poly(ethylenglycol(meth)acrylaten;
Phenyl-poly(ethylenglycol)(meth)acrylaten;
dem folgenden Monomer : 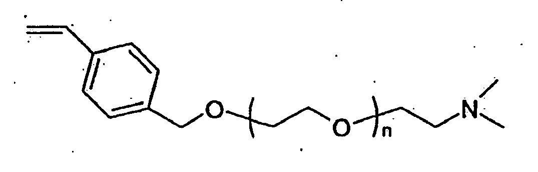 **dadurch gekennzeichnet, dass** n vorzugsweise im Bereich von 3 et 100, insbesondere 5 bis 50 und besser 7 bis 30 liegt.

8. Zusammensetzung nach einem der vorhergehenden Ansprüche, **dadurch gekennzeichnet, dass** das Monomer der Formel (I), einzeln oder als Gemisch, in einer Menge von 20 bis 55 Gew.-%, bezogen auf das Gewicht des fertigen Polymers, und vorzugsweise 30 bis 50 Gew.-% enthalten ist.

9. Zusammensetzung nach einem der vorhergehenden Ansprüche, **dadurch gekennzeichnet, dass** in den Formeln (IIa), (IIb), (IIc) und/oder (IId) die Gruppe R'2 ausgewählt ist unter:
- einer Alkylengruppe, wie Methylen, Ethylen, Propylen, n-Butylen, Isobutylen, tert-Butylen, n-Hexylen, n-Octylen, n-Dodecylen, n-Octadecylen, n-Tetradecylen, n-Docosanylen;
- einer Phenylengruppe -C₆H₄- (ortho, meta oder para), die gegebenenfalls mit einer C₁₋₁₂-Alkylgruppe substituiert ist, die gegebenenfalls 1 bis 5 Heteroatome enthält, die unter N, O, S, F, Si und P ausgewählt sind; oder eine Benzylengruppe -C₆H₄-CH₂-, die gegebenenfalls mit einer C₁₋₁₂-Alkylgruppe substituiert ist, die gegebenenfalls 1 bis 5 Heteroatome enthält, die unter O, N, S, F, Si und P ausgewählt sind;
- einer Gruppe der Formel -CH₂-O-CO-O-, CH₂-CH₂-O-CO-O-, -CH₂-CO-O-, -CH₂-CH₂-CO-O-,-[(CH₂)₅-CO-O]ₙ-, -CH₂-CH(CH₃)-O-, -(CH₂)₂-O-, -CH₂-O-CO-NH-, -CH₂-CH₂-O-CO-NH-; -CH₂-NH-CO-NH- oder -CH₂-CH₂-NH-CO-NH-, -CH₂-CHOH-, -CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-CH₂-CH₂-O-; -CH₂-CH₂-CHR'-O-, wobei R' et R" eine lineare oder verzweigte C₁₋₂₂-Alkylgruppe bedeuten, die gegebenenfalls 1 bis 12 Heteroatome enthält, die unter O, N, S, F, Si und P ausgewählt sind;
- oder einem Gemisch dieser Gruppen.

10. Zusammensetzung nach einem der vorhergehenden Ansprüche, **dadurch gekennzeichnet, dass** in der Formel (IIa) die in X vorhandenen Gruppen R6 und R7 unter Wasserstoff, Methyl, Ethyl, Propyl, Isopropyl, n-Butyl, t-Butyl, Isobutyl, Octyl, Lauryl, Stearyl ausgewählt sind.

11. Zusammensetzung nach einem der vorhergehenden Ansprüche, **dadurch gekennzeichnet, dass** in der Formel (IIa) X eine Gruppe bedeutet, die unter den Gruppen vom Typ Pyridin, Indolyl, Isoindolinyl, Imidazolyl, Imidazolinyl, Piperidinyl, Pyrazolinyl, Pyrazolyl, Chinolin, Pyrazolinyl, Pyridinyl, Piperazinyl, Pyrrolidinyl, Chinidinyl, Thiazolinyl, Morpholin, Guanidino, Amidino, Phosphonium und deren Gemischen ausgewählt ist.

12. Zusammensetzung nach einem der vorhergehenden Ansprüche, **dadurch gekennzeichnet, dass** die Monomere der Formel (IIa) mit Neutralisationsmitteln neutralisiert wurden, die unter den Neutralisationsmitteln mit log P kleiner oder gleich 2, beispielsweise im Bereich von -8 bis 2 und vorzugsweise im Bereich von -6 bis 1, besonders im Bereich von -6 bis 0; und/oder Stoffen mit log P größer 2, vorzugsweise größer oder gleich 2,5, besonders größer 3 und insbesondere im Bereich von 3 bis 15 oder besser 3,5 bis 10 ausgewählt sind.

13. Zusammensetzung nach einem der vorhergehenden Ansprüche, **dadurch gekennzeichnet, dass** das Monomer der Formel (IIa) einzeln oder in Form eines Gemisches ausgewählt ist unter: Dimethylaminopropyl(meth)acrylamid, Dimethylaminoethyl(meth)acrylamid, Diethylaminoethyl(meth)acrylat, Dimethylaminoethyl(meth)acrylat, Vinylimidazol, Vinylpyridin, Morpholinoethyl(meth)acrylat und den nachfolgenden Monomeren:

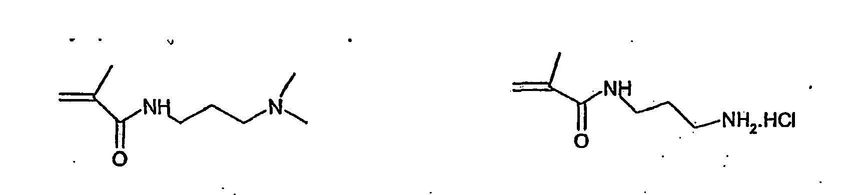

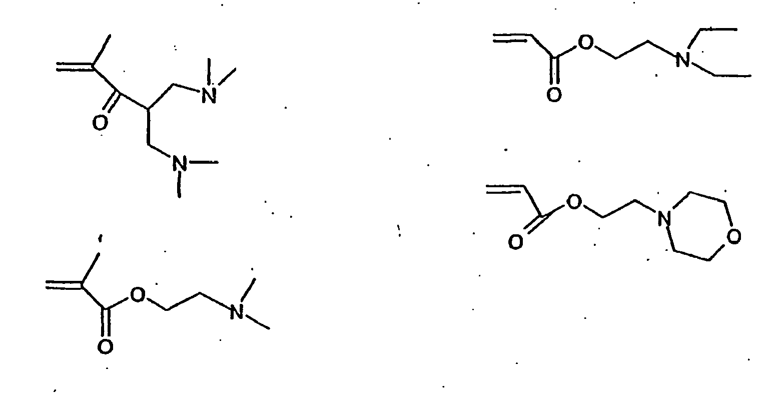

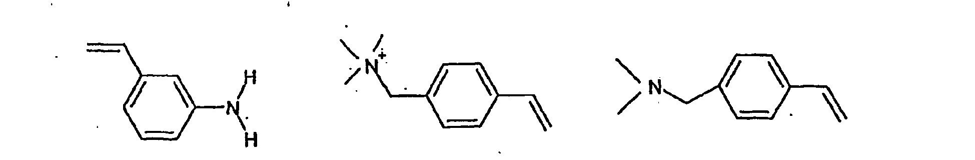

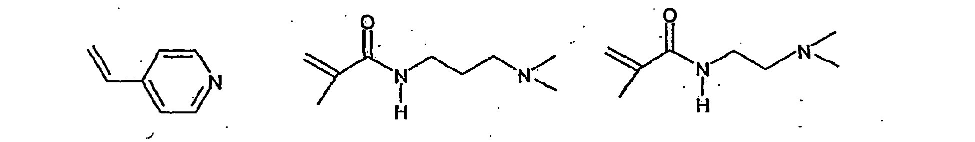

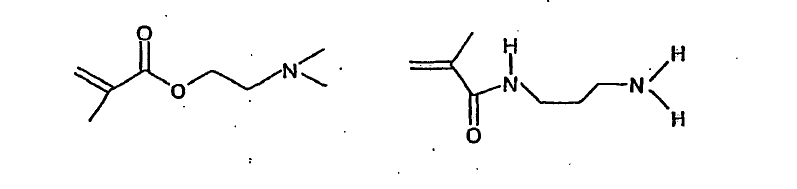

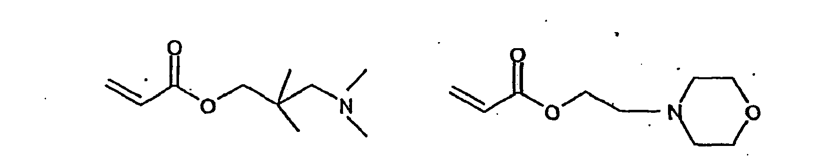

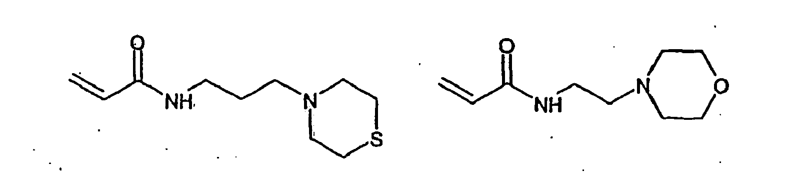

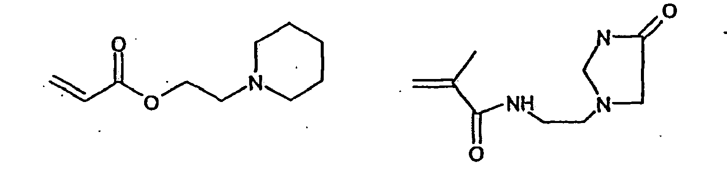

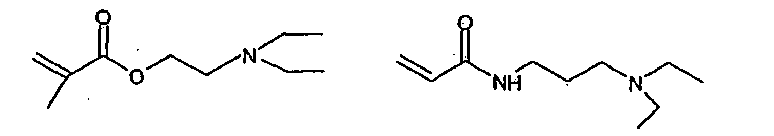

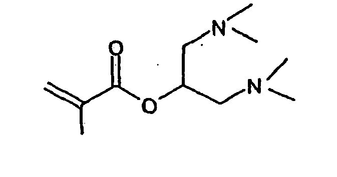

14. Zusammensetzung nach einem der vorhergehenden Ansprüche, **dadurch gekennzeichnet, dass** die anionischen Monomere ausgewählt sind unter: Maleinsäureanhydrid, Acrylsäure, Methacrylsäure, Crotonsäure, Itaconsäure, Fumarsäure, Maleinsäure, 2-Carboxyethylacrylat (CH₂=CH-C(O)-O-(CH₂)₂-COOH), Styrolsulfonsäure, 2-Acrylamido-2-methylpropansulfonsäure, Vinylbenzoesäure, Vinylphosphorsäure, Sulfopropyl(meth)acrylat und deren Salzen.

15. Zusammensetzung nach einem der vorhergehenden Ansprüche, **dadurch gekennzeichnet, dass** in der Formel (IIc) die Gruppe X'⁺ unter den Gruppen vom Typ Pyridin, Indolyl, Isoindolinyl, Imidazolyl, Imidazolinyl, Piperidinyl, Pyrazolinyl, Pyrazolyl, Chinolin, Pyrazolinyl, Pyridinyl, Piperazinyl, Pyrrolidinyl, Chinidinyl, Thiazolinyl, Morpholin, Guanidino, Amidino, und deren Gemischen ausgewählt ist.

16. Zusammensetzung nach einem der vorhergehenden Ansprüche, **dadurch gekennzeichnet, dass** in den Formeln (IIc) und/oder (IId) die Gruppe R'3 ausgewählt ist unter:
- einer Alkylengruppe, wie Methylen, Ethylen, Propylen, n-Butylen, Isobutylen, tert-Butylen, n-Hexylen, n-Octylen, n-Dodecylen, n-Octadecylen, n-Tetradecylen, n-Docosanylen;
- einer Phenylengruppe -C₆H₄- (ortho, meta oder para), die gegebenenfalls mit einer C₁₋₁₂-Alkylgruppe substituiert ist, die gegebenenfalls 1 bis 5 Heteroatome enthält, die unter O, N, S, F, Si und P ausgewählt sind; oder eine Benzylengruppe -C₆H₄-CH₂-, die gegebenenfalls mit einer C₁₋₁₂-Alkylgruppe substituiert ist, die gegebenenfalls 1 bis 5 Heteroatome enthält, die unter O, N, S, F, Si und P ausgewählt sind;
- einer Gruppe der Formel -CH₂-O-CO-O-, CH₂-CH₂-O-CO-O-, -CH₂-CO-O-, -CH₂-CH₂-CO-O-, -[(CH₂)₅-CO-O]ₙ-, -CH₂-CH(CH₃)-O-, -(CH₂)₂-O-, -CH₂-O-CO-NH-, -CH₂-CH₂-O-CO-NH-; -CH₂-NH-CO-NH- oder -CH₂-CH₂-NH-CO-NH-, -CH₂-CHOH-, - CH₂-CH₂-CHOH-, -CH₂-CH₂-CH(NH₂)-, -CH₂-CH(NH₂)-, -CH₂-CH₂-CH(NHR')-, -CH₂-CH(NHR')-, -CH₂-CH₂-CH(NR'R")-, -CH₂-CH(NR'R")-, -CH₂-CH₂-CH₂-NR'-, -CH₂-CH₂-CH₂-O-; -[CH₂-CH₂-O]ₙ- und -[CH₂-CH(CH₃)-O]ₙ- -CH₂-CH₂-CHR'O-, wobei R' et R" eine geradkettige oder verzweigte C₁₋₂₂-Alkylgruppe bedeuten, die gegebenenfalls 1 bis 12 Heteroatome enthält, die unter O, N, S, F, Si und P ausgewählt sind;
- oder einem Gemisch dieser Gruppen.

17. Zusammensetzung nach einem der vorhergehenden Ansprüche, **dadurch gekennzeichnet, dass** in der Formel (IId) X"⁺ unter Trimethylammonium; Triethylammonium; N,N-Dimethyl-N-octylammonium, N,N-Dimethyl-N-laurylammonium ausgewählt ist.

18. Zusammensetzung nach einem der vorhergehenden Ansprüche, **dadurch gekennzeichnet, dass** das Monomer der Formel (IIc) oder (IId) unter N,N-Dimethyl-N-(2-methacryloyloxyethyl)-N-(3-sulfopropyl)-ammoniumbetain, N,N-Dimethyl-N-(3-methacrylamidopropyl)-N-(3-sulfopropyl)-ammoniumbetain, 1-(3-Sulfopropyl)-2-vinylpyridiniumbetain und 2-Methacryloyloxyethylphosphorylcholin ausgewählt ist.

19. Zusammensetzung nach einem der vorhergehenden Ansprüche, **dadurch gekennzeichnet, dass,** wenn das "im Wesentlichen kationische" Monomer unter den Gemischen aus kationischen und/oder amphoteren Monomeren und anionischen Monomeren ausgewählt ist, die anionischen Monomere vorzugsweise in einem Mengenanteil von 5 bis 40 Gew.-%, bezogen auf das Gewicht des Gemisches der "kationischen und/oder amphoteren + anionischen Monomere", insbesondere 10 bis 30 Gew.-% und vorzugsweise 15 bis 25 Gew.-% enthalten ist.

20. Zusammensetzung nach einem der vorhergehenden Ansprüche, **dadurch gekennzeichnet, dass** das "im Wesentlichen kationische" Monomer in einem Mengenanteil von 45 bis 80 Gew.-%, bezogen auf das Gewicht des fertigen Polymers, und vorzugsweise 50 bis 70 Gew.-% enthalten ist.

21. Zusammensetzung nach einem der vorhergehenden Ansprüche, **dadurch gekennzeichnet, dass** das ergänzende nichtionische hydrophile Monomer einen log P im Bereich von -8 bis 2, vorzugsweise kleiner oder gleich 1,5 und besonders kleiner oder gleich 1 und insbesondere -7 bis 1, besser -6 bis 0 aufweist.

22. Zusammensetzung nach einem der vorhergehenden Ansprüche, **dadurch gekennzeichnet, dass** das ergänzende nichtionische hydrophile Monomer unter den Monomeren der Formel (III) oder deren Gemischen ausgewählt ist: 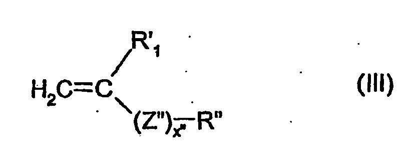 in der Formel:
- R'₁ bedeutet Wasserstoff oder -CH₃;
- Z" ist eine zweiwertige Gruppe, die unter -COO-, -CONH-,
- CONCH₃, -OCO-, - SO₂, -CO-O-CO-, -CO-CH₂-CO- oder -O- ; vorzugsweise COO et CONH ausgewählt ist;
- x" ist 0 oder 1;
- R" ist eine gesättigte oder ungesättigte, gegebenenfalls aromatische, lineare, verzweigte oder cyclische, kohlenstoffhaltige Gruppe mit 1 bis 30 Kohlenstoffatomen, die 1 bis 18 Heteroatome enthalten kann, die unter O, N, S, F, Si und P ausgewählt sind; insbesondere Methyl, Ethyl, Propyl, Isopropyl, n-Butyl, Isobutyl, tert-Butyl, Phenyl, Benzyl oder eine Gruppe der Formel -CH₂-CH₂-CH₂OH, -CH₂-CH₂-OH, -CH₂-CH₂-CH₂OH oder Furfuryl.

23. Zusammensetzung nach einem der vorhergehenden Ansprüche, **dadurch gekennzeichnet, dass** das ergänzende nichtionische hydrophile Monomer ausgewählt ist unter: Methylmethacrylat, Methylacrylat, Ethylmethacrylat, Ethylacrylat, Propylacrylat, Isopropylacrylat, Tetrahydrofurfurylmethacrylat, Tetrahydrofurfurylacrylat, 2-Hydroxyethylmethacrylat, 2-Hydroxyethylacrylat, E-thoxyethylmethacrylat, Ethoxyethylacrylat, N-Isopropylacrylamid, N-Isopropylmethacrylamid, N,N-Dimethylacrylamid, N,N-Dimethylmethacrylamid, Vinylacetat, Methylvinylether, Ethylvinylether, Vinylpyrrolidon, Vinylcaprolactam, N-Vinylacetamid und Hydroxypropylacrylat; N-Vinyllactam, Acrylamid, N-Methylacrylamid, N,N-Dimethylacrylamid, N-Methyl-N-vinylacetamid, N-Vinylformamid, N-Methyl-N-vinylformamid, Vinylalkohol (copolymerisiert in Form von Vinylacetat und anschließend hydrolysiert).

24. Zusammensetzung nach einem der vorhergehenden Ansprüche, **dadurch gekennzeichnet, dass** das ergänzende nichtionische hydrophile Monomer, einzeln oder in Form eines Gemisches, in einem Mengenanteil von 0,1 bis 35 Gew.-%, vorzugsweise 1 bis 25 Gew.-%, beispielsweise 3 bis 15 Gew.-% und besser 5 bis 9,5 Gew.-%, bezogen auf das Gewicht des Polymers, enthalten ist.

25. Zusammensetzung nach einem der vorhergehenden Ansprüche, **dadurch gekennzeichnet, dass** das Copolymer neutralisiert ist.

26. Zusammensetzung nach einem der vorhergehenden Ansprüche, **dadurch gekennzeichnet, dass** das Copolymer mit einem Neutralisationsmittel neutralisiert wurde, das unter den linearen, verzweigten oder cyclischen aliphatischen Säuren und/oder ungesättigten oder aromatischen Säuren ausgewählt ist und insbesondere 1 bis 1000 Kohlenstoffatome, insbesondere 2 bis 500 Kohlenstoffatome enthalten kann; wobei es mindestens eine Säurefunktion im Sinne von Bronsted enthält, insbesondere eine oder mehrere Carbonsäuregruppen, Sulfonsäuregruppen und/oder Phosphonsäuregruppen; und ferner ein oder mehrere Heteroatome enthalten kann, die unter O, N, Si, F und P ausgewählt sind, beispielsweise in Form von Hydroxygruppen.

27. Zusammensetzung nach einem der vorhergehenden Ansprüche, **dadurch gekennzeichnet, dass** das Copolymer mit einem Neutralisationsmittel neutralisiert wurde, das, einzeln oder in Form eines Gemisches ausgewählt ist unter:
- linearen, verzweigten oder cyclischen, gesättigten oder ungesättigten, gegebenenfalls aromatischen Fettsäuren mit 6 bis 32 Kohlenstoffatomen, insbesondere 8 bis 28 Kohlenstoffatomen, die mindestens eine Funktion COOH oder eine Sulfonsäurefunktion (-SO₃H) enthalten;
- linearen, verzweigten oder cyclischen, gesättigten oder ungesättigten, gegebenenfalls aromatischen Hydroxysäuren und insbesondere α-Hydroxysäuren mit 6 bis 32 Kohlenstoffatomen, insbesondere 8 bis 28 Kohlenstoffatomen, die mindestens eine Funktion COOH oder eine Sulfonsäurefunktion (-SO₃H) enthalten;
- Alkylbenzolsulfonsäuren, bei denen die Alkylgruppe 4 bis 30 und insbesondere 6 bis 24 Kohlenstoffatome enthalten kann;
- amphoteren Neutralisationsmitteln, insbesondere vom Typ der Alkylbetaine oder Alkylamidopropylbetaine, bei denen die Alkylgruppe 4 bis 30 und insbesondere 6 bis 24 Kohlenstoffatome enthalten kann; besonders Cocamidopropylbetain.

28. Zusammensetzung nach einem der vorhergehenden Ansprüche, **dadurch gekennzeichnet, dass** das Copolymer mit einem Neutralisationsmittel neutralisiert wurde, das ausgewählt ist unter: α-Hydroxyethansäure, α-Hydroxyoctansäure, α-Hydroxycaprylsäure, Ascorbinsäure, Essigsäure, Benzoesäure, Behensäure, Caprinsäure, Citronensäure, Capronsäure, Caprylsäure, Dodecylbenzolsulfonsäure, 2-Ethylcapronsäure, Folsäure, Fumarsäure, Galactarsäure, Gluconsäure, Glycolsäure, 2-Hexadecyleicosansäure, Hydroxycapronsäure, 12-Hydroxystearinsäure, I-solaurinsäure (oder 2-Butyloctansäure), Isomyristinsäure (oder 2-Hexyloctansäure), Isoarachidinsäure (oder 2-Octyldodecansäure), Isolignocerinsäure (oder 2-Decyltetradecansäure), Milchsäure, Laurinsäure, Äpfelsäure, Myristinsäure, Ölsäure, Palmitinsäure, Propionsäure, Sebacinsäure, Stearinsäure, Weinsäure, Terephthalsäure, Trimesinsäure, Undecylensäure, Propylbetain, Cocoamidopropylbetain und dem Betain-Hydrochlorid der Formel [(CH₃)₃N⁺CH₂CO₂H.Cl-] sowie deren Gemischen.

29. Zusammensetzung nach einem der vorhergehenden Ansprüche, **dadurch gekennzeichnet, dass** das Copolymer mit einem Neutralisationsmitteln neutralisiert wurde, das ausgewählt ist unter Capronsäure, 2-Ethylcapronsäure, Ölsäure, Behensäure, Stearinsäure, Essigsäure, Citronensäure, Weinsäure, dem Betain-Hydrochlorid und/ oder Gluconsäure und vorzugsweise dem Betain-Hydrochlorid und/oder Behensäure.

30. Zusammensetzung nach einem der Ansprüche 25 bis 29, **dadurch gekennzeichnet, dass** das Neutralisationsmittel in einem Mengenanteil von 1 bis 300 %, insbesondere 5 bis 250 % und besser 10 bis 200 %, bezogen auf die gesamten Aminofunktionen des Polymers oder der Monomere, zugegeben wird.

31. Zusammensetzung nach Anspruch 30, wobei das Neutralisationsmittel einzeln oder als Gemisch in einer Menge von 1 bis 99 %, insbesondere 5 bis 90 Gew.-% und besser 10 bis 80 %, bezogen auf die gesamten Aminofunktionen des Polymers oder der Monomere, zugegeben wird.

32. Zusammensetzung nach Anspruch 30, wobei das Neutralisationsmittel einzeln oder als Gemisch in einer Menge von 0,01 bis 3 Moläquivalenten, insbesondere 0,05 bis 2,5 und sogar 0,1 bis 2 Moläquivalenten, bezogen auf die gesamten Aminofunktionen des Polymers oder der Monomere, zugegeben wird.

33. Zusammensetzung nach einem der vorhergehenden Ansprüche, **dadurch gekennzeichnet, dass** das Copolymer eine gewichtsmittlere Molmasse (Mw) im Bereich von 500 bis 5000000, besonders 1000 bis 3000000 und besonders bevorzugt 2000 bis 2000000, besonders 4000 bis 500000, u.a. 7000 bis 250000 und besser 8000 bis 100000 aufweist.

34. Zusammensetzung nach einem der vorhergehenden Ansprüche, **dadurch gekennzeichnet, dass** das Copolymer umfasst:
- ein Monomer der Formel (I), einzeln oder als Gemisch, enthalten in einer Menge von 20 bis 80 Gew.-%, bezogen auf das Gewicht des fertigen Polymers, insbesondere 20 bis 60 Gew.-% und vorzugsweise 30 bis 50 Gew.-% und, einzeln oder als Gemisch, ausgewählt unter den Poly(ethylenglycol)(meth)-acrylaten und vorzugsweise solchen mit einer Molmasse im Bereich von 350 bis 13000 g/mol und insbesondere 500 bis 8000 g/mol; und
- ein "im Wesentlichen kationisches" Monomer, enthalten in einer Menge von 40 bis 90 Gew.-%, bezogen auf das Gewicht des fertigen Polymers, insbesondere 40 bis 80 Gew.-% und vorzugsweise 50 bis 70 Gew.-% und, einzeln oder als Gemisch, ausgewählt unter Dimethylaminopropyl(meth)-acrylamid, Dimethylaminoethyl(meth)acrylamid, Dimethylaminoethyl(meth)acrylat, Vinylimidazol, Vinylpyridin, Morpholinoethyl(meth)acrylat; und
- wobei das Copolymer mit einem Neutralisationsmittel neutralisiert wurde, das ausgewählt ist unter 2-Ethylcapronsäure, Ölsäure, Behensäure, Stearinsäure, Essigsäure, Citronensäure, Weinsäure, dem Betain-Hydrochlorid und/oder Gluconsäure und vorzugsweise Behensäure und/oder dem Betain-Hydrochlorid.

35. Zusammensetzung nach einem der vorhergehenden Ansprüche, **dadurch gekennzeichnet, dass** das Copolymer umfasst:
- ein Monomer der Formel (I), einzeln oder als Gemisch, enthalten in einer Menge von 20 bis 80 Gew.-%, bezogen auf das Gewicht des fertigen Polymers, insbesondere 20 bis 60 Gew.-% und vorzugsweise 30 bis 50 Gew.-% und, einzeln oder als Gemisch, ausgewählt unter den Poly(ethylenglycol)(meth)-acrylaten und vorzugsweise solchen mit einer Molmasse im Bereich von 350 bis 13000 g/mol und insbesondere 500 bis 8000 g/mol; und
- ein "im Wesentlichen kationisches" Monomer, enthalten in einer Menge von 40 bis 90 Gew.-%, bezogen auf das Gewicht des fertigen Polymers, insbesondere 40 bis 80 Gew.-% und vorzugsweise 50 bis 70 Gew.-% und, einzeln oder als Gemisch, ausgewählt unter Dimethylaminopropyl(meth)-acrylamid; und
- wobei das Polymer mit einem Neutralisationsmittel neutralisiert wurde, das ausgewählt ist unter Behensäure und/oder dem Betain-Hydrochlorid.

36. Zusammensetzung nach einem der vorhergehenden Ansprüche, **dadurch gekennzeichnet, dass** das Copolymer in einer Menge von 0,01 bis 30 Gew.-% Trockensubstanz, insbesondere 0,1 bis 20 Gew.-%, besser 0,1 bis 10 Gew.-% und noch besser 0,5 bis 3 Gew.-%, bezogen auf das Gesamtgewicht der Zusammensetzung, enthalten ist.

37. Zusammensetzung nach einem der vorhergehenden Ansprüche, **dadurch gekennzeichnet, dass** der anionische grenzflächenaktive Stoff unter den Salzen von Alkylsulfaten, Alkylethersulfaten, Alkylethercarboxylaten und deren Gemischen ausgewählt ist.

38. Zusammensetzung nach einem der vorhergehenden Ansprüche, **dadurch gekennzeichnet, dass** der oder die anionischen grenzflächenaktiven Stoffe in einer Konzentration von 3 bis 40 Gew.-%, vorzugsweise 5 bis 25 Gew.-%, bezogen auf das Gesamtgewicht der Zusammensetzung, enthalten sind.

39. Zusammensetzung nach einem der vorhergehenden Ansprüche, **dadurch gekennzeichnet, dass** der nichtionische grenzflächenaktive Stoff ausgewählt ist unter den Alkoholen, α-Diolen, Alkylphenolen oder Fettsäuren, die polyethoxyliert, polypropoxyliert oder mehrfach mit Glycerin verethert sind und eine Fettkette mit beispielsweise 8 bis 22 Kohlenstoffatomen aufweisen, wobei die Anzahl der Ethylenoxidgruppen oder Propylenoxidgruppen insbesondere im Bereich von 2 bis 50 und die Anzahl der Glyceringruppen insbesondere im Bereich von 2 bis 30 liegen kann. Es können auch die Copolymere von Ethylenoxid und Propylenoxid, die Kondensate von Ethylenoxid und Propylenoxid mit Fettalkoholen; polyethoxylierte Fettamide mit vorzugsweise 2 bis 30 mol Ethylenoxid, mehrfach mit Glycerin veretherte Fettamide, die im Mittel 1 bis 5 Glyceringruppen und insbesondere 1,5 bis 4 Glyceringruppen enthalten; ethoxylierte Sorbitanfettsäureester mit 2 bis 30 mol Ethylenoxid; Saccharosefettsäureester, Polyethylenglycolfettsäureester, Alkylpolyglycoside, N-Alkylglucaminderivate und Aminoxide, wie Alkyl(C₁₀₋₁₄)aminoxide oder N-Acylaminopropylmorpholinoxide, genannt werden.

40. Zusammensetzung nach einem der vorhergehenden Ansprüche, **dadurch gekennzeichnet, dass** der oder die nichtionischen grenzflächenaktiven Stoffe in einer Konzentration von 0,5 bis 25 Gew.-%, insbesondere 1 bis 20 Gew.-%, bezogen auf das Gesamtgewicht der kosmetischen Zusammensetzung, und besonders 2 bis 10 Gew.-% enthalten sind.

41. Zusammensetzung nach einem der vorhergehenden Ansprüche, **dadurch gekennzeichnet, dass** sie ferner eine amphoteren grenzflächenaktiven Stoff enthält.

42. Zusammensetzung nach einem der vorhergehenden Ansprüche, **dadurch gekennzeichnet, dass** das Gesamtgewicht des grenzflächenaktiven Stoffes im Bereich von 3,5 bis 50 Gew.-%, vorzugsweise 5 bis 30 Gew.-% und noch bevorzugter 8 bis 25 Gew.-%, bezogen auf das Gesamtgewicht der Zusammensetzung, liegt.

43. Zusammensetzung nach einem der vorhergehenden Ansprüche, **dadurch gekennzeichnet, dass** das kosmetisch akzeptable Medium mindestens einen Bestandteil enthält, der unter Wasser; hydrophilen organischen Lösemitteln, wie Alkoholen, insbesondere linearen oder verzweigten Monoalkoholen mit 1 bis 6 Kohlenstoffatomen und Polyolen und Glycolethern ausgewählt ist.

44. Zusammensetzung nach einem der vorhergehenden Ansprüche, **dadurch gekennzeichnet, dass** die Zusammensetzung ferner mindestens ein kationisches oder amphoteres Polymer enthält.

45. Zusammensetzung nach dem vorhergehenden Anspruch, **dadurch gekennzeichnet, dass** das oder die kationischen oder amphoteren Polymere 0,001 bis 20 Gew.-%, vorzugsweise 0,01 bis 10 Gew.-%, bezogen auf das Gesamtgewicht der fertigen Zusammensetzung, ausmachen.

46. Zusammensetzung nach einem der vorhergehenden Ansprüche, **dadurch gekennzeichnet, dass** die Zusammensetzung ferner ein Silicon enthält.

47. Zusammensetzung nach dem vorhergehenden Anspruch, **dadurch gekennzeichnet, dass** das oder die Siliconen 0,001 bis 20 Gew.-%, vorzugsweise 0,1 bis 5 Gew.-%, bezogen auf das Gesamtgewicht der fertigen Zusammensetzung, ausmachen.

48. Zusammensetzung nach einem der vorhergehenden Ansprüche, **dadurch gekennzeichnet, dass** die Zusammensetzung ferner mindestens einen Zusatzstoff enthält, der unter den Wachsen, pastösen Fettsubstanzen, Gummis und deren Gemischen, Ölen tierischer, pflanzlicher, mineralischer oder synthetischer Herkunft, synthetischen Estern oder Ethern; Fettalkoholen mit 12 bis 26 Kohlenstoffatomen; flüchtigen oder nichtflüchtigen, linearen oder cyclischen, bei Raumtemperatur flüssigen oder pastösen Siliconölen; Polymeren, die von Polymeren mit PEG-Gruppen verschieden sind; Vitaminen, Parfums, Perlglanzstoffen, verdickungsmittel; Gelbildnern, Spurenelementen, beruhigenden Stoffen, Maskierungsmitteln, Parfums, Alkalisierungsmitteln oder Ansäuerungsmitteln, Konservierungsmitteln, Sonnenschutzfiltem, Antioxidantien, Wirkstoffen gegen Haarausfall, Antischuppenmitteln, Treibmitteln, Ceramiden; und deren Gemischen ausgewählt ist.

49. Zusammensetzung nach einem der vorhergehenden Ansprüche, die in der Form einer Zusammensetzung für die Haarbehandlung, insbesondere für den Halt der Frisur oder die Formgebung der Haare, beispielsweise als Haarwaschmittel vorliegt.

50. Kosmetisches Verfahren für die Behandlung von Keratinsubstanzen, wie der Haut des Körpers oder des Gesichts, der Nägel, der Körperhaare, der Haare und/ oder der Wimpern, **dadurch gekennzeichnet, dass** es darin besteht, auf die Keratinfasern eine kosmetische Zusammensetzung, wie sie in einem der Ansprüche 1 bis 34 definiert ist, auf die Keratinsubstanzen aufzubringen und nach einer gegebenenfalls abgewarteten Einwirkzeit gegebenenfalls zu spülen.
